# EUROPEAN PATENT APPLICATION

(11) **EP 4 119 948 A1**
(43) Date of publication of application: **18.01.2023**
(21) Application number: 21194734.6
(22) Date of filing: 03.09.2021
(51) Int. Cl.: G01N 33/68, G01N 33/50, A61K 38/17, A61K 38/44, A61P 37/06

(54) **METHOD FOR STRATIFICATION AND TREATMENT OF MULTIPLE SCLEROSIS**

(30) Priority: 16.07.2021 EP 21186270
(71) Applicant: Universität Zürich, 8006 Zürich (CH)
(72) Inventor: SOSPEDRA RAMOS, Mireia, 8952 Schlieren (CH); MARTIN, Roland, 8952 Schlieren (CH); LUTTEROTTI, Andreas, 8952 Schlieren (CH)
(74) Representative: König Szynka Tilmann von Renesse Patentanwälte Partnerschaft mbB Düsseldorf

(57) **Abstract**

The disclosure relates to the field of multiple sclerosis (MS) stratification by analyzing the body fluid of an MS patient for CD27- Th1 CD4+ cells in a body fluid, such as blood or CSF. The invention also relates to the field of antigen specific immunotherapies for MS, such as the induction of tolerance involving e.g. GDP-L-fucose synthase for responders.

## Description

### FIELD OF INVENTION

The disclosure relates to the field of multiple sclerosis (MS) stratification by analyzing the body fluid of an MS patient. The invention also relates to the field of antigen specific immunotherapies, such as the induction of tolerance.

### BACKGROUND OF THE INVENTION

Multiple sclerosis (MS) is a devastating autoimmune inflammatory disease mainly affecting young adults. MS is a prototypic example of an organ-specific autoimmune disease (AID), as the autoimmune response only targets the central nervous system (CNS) consisting of brain and spinal cord. Organ-specific AID means that the immune system of the patient damages a specific tissue or cell type by autoreactive T cells and/or antibodies.

MS preferentially affects young adults between 20 and 40 years, but children and older individuals can also develop MS. The disease is about 2-3 times more frequent in women than in men. MS usually becomes clinically manifest by temporary problems with vision (acute optic neuritis), sensation, or motor and autonomous function, but can lead to a broad range of neurological symptoms.

At the time of first manifestation, when differential diagnoses have been excluded, the disease is referred to as clinically isolated syndrome (CIS) provided that the cerebrospinal fluid (CSF) and magnetic resonance imaging (MRI) findings are consistent with the diagnosis. MRI discloses lesions in locations typical for MS, i.e. juxtacortical, periventricular, in the brain stem or spinal cord. If certain criteria are fulfilled that can be summarized as dissemination in space (more than one lesion or clinical symptom/sign) and time (more than one event) then the diagnosis of relapsing-remitting multiple sclerosis (RRMS) can be made. A special scenario is the accidental discovery of MRI lesions compatible with MS without clinical symptoms. This is referred to as radiologically isolated syndrome (RIS) and can be considered a pre-stage of CIS and RRMS. More than 80% of patients suffer from one of these, and the majority of patients develops later what is called secondary progressive MS (SPMS). At this time, relapses/exacerbations become less frequent or stop altogether and neurological disability increases steadily either between relapses or without these.

A special form of MS is primary progressive MS (PPMS), which never shows relapses, but rather begins with steady worsening of neurological symptoms, e.g. of the ability to walk. PPMS affects approximately 10% of MS patients and males and females with equal frequency. Its onset is usually later than CIS or RRMS. With respect to causes and disease mechanisms PPMS is considered similar to the above RIS-CIS-RRMS-SPMS.

Typically, MS is diagnosed according to the revised McDonald or recently Lublin criteria. These criteria also allow distinguishing between the different forms and disease activity of MS (Thompson et al., 2018, Lancet Neurol, 17(2):162-173).

MS is a disease with a complex genetic background. More than 200 MS risk alleles or quantitative traits (common variants of genes detected as single nucleotide polymorphisms, SNPs) have been identified in the last decade, however, by far the most important is the human leukocyte antigen (HLA)-DR15 haplotye. In addition, several environmental/lifestyle risk factors have been found. These include infection with Epstein Barr virus (EBV), smoking, low vitamin D3 levels and obesity as the most important ones.

All the genetic and environmental risk factors are common and shared by many individuals in the healthy population. The exact reasons, why the disease starts in individuals with certain genetic and environmental risk factors, are not clear, but one assumes that viral and bacterial infections, for instance by changes in the gut microbiota, can be triggers. The concordance rate of monozygotic twins of 10-30% and the risk of first-degree relatives of an MS patient of approximately 2-4%, compared to a risk of 1/1000 in the general population, provide an estimate of the genetic versus the environmental risk, although the interplay between the two is also complex.

In order to identify the components of the CNS, against which the autoimmune response in MS is directed, researchers oriented their efforts towards the cells and structures that are affected in MS, particularly myelin and axons/neurons and the proteins that are specific for these cells/structures. During the last thirty years, several myelin proteins such as myelin basic protein (MBP), proteolipid protein (PLP) and myelin oligodendroglia glycoprotein (MOG) have been identified as encephalitogenic in animal models (experimental autoimmune encephalomyelitis; EAE), i.e. their injection into susceptible rodent strains leads to a disease with similarities with MS, but also by examining immune cells from MS patients (Sospedra and Martin, 2005, Annu Rev Immunol, 23:683-747). The above autoantigens are CNS-specific and exclusively (PLP and MOG) or almost exclusively (MBP) expressed in the brain. In MS, a few autoantigens that are not CNS-specific such as alpha-B crystallin and transaldolase-H have also been described as potential targets.

Recently, a further MS-relevant antigen has been identified (GDP-L-fucose synthase (GDP-L-FS); WO 2020/002674). This protein has been found to be immunodominant in MS and is an autoantigen.

Further evidence suggests CD4+ autoreactive T cells as a central factor for the autoimmune pathogenesis of MS probably relevant not only for the induction and maintenance of the autoimmune response, but also during tissue damage (Sospedra and Martin, 2005). The frequency of high avidity CD4+ T cells reactive to main constituents of the myelin sheath, such as MBP, PLP and MOG is increased in MS patients (Bielekova et al., 2004, J Immunol, 172:3893-3904). Due to their involvement in disease pathogenesis CD4+ T cells are a target for therapeutic interventions.

Detailed investigation of the immune response against the CNS-specific proteins showed that certain peptides thereof are recognized by a large fraction of patients and in the context of the disease-associated HLA-DR molecules. Such peptides are referred to as immunomodominant (Bielekova et al., 2004).

The following characteristics indicate that a certain peptide of a protein is immunodominant in the context of MS:
a) frequent recognition of this peptide by T cells, i. e. by approximately 10% or more of MS patients, often in the context of a disease-associated HLA allele or haplotype (Sospedra and Martin, 2005), and
b) recognition of this peptide by disease-relevant T cells such as those that respond to peptides at low concentrations (high avidity T cells) (Bielekova et al., 2004) and are therefore considered particularly dangerous, and/or have a proinflammatory phenotype, and/or are isolated from the target organ or compartment (CNS), in the case of MS, brain-, spinal cord- or CSF-infiltrating T cells.

However, high avidity recognition is not a prerequisite, since low-avidity myelin-specific T cells have also been shown to be pathogenic in humanized transgenic mouse models (Quandt et al. 2012, J Immunol, 189(6):2897-2908).

It has recently been demonstrated that T cells of MS patients show increased *in vitro* proliferation in the absence of an exogenous antigen (Mohme et al., 2013, Brain, 136:1783-1798). These "autoproliferating" T cells are enriched for cells that home to the CNS compartment of MS patients and can thus be considered as a peripheral blood source of brain-/CSF-infiltrating T cells (Jelcic et al., 2018, Cell, 175(1):85-100.e23).

In the case that data from testing T cells *in vitro* is not available or in addition to such testings, immune recognition of peptides can also be predicted/inferred from those peptides that will bind well to the HLA-class I or -class II alleles of the individual and for CD8+ and CD4+ T cells respectively. Peptide binding predictions are well known to the skilled person. They can be performed by well-established prediction algorithms (NetMHCII - www.cbs.dtu.dk/services/NetMHCII/; IEDB - www.iedb.org/) and analysis of the HLA-binding motifs (SYFPEITHI - www.syfpeithi.de/).

Immunodominant peptides can be used in antigen-specific immunotherapies such as tolerance induction. One example is EP 2 205 273 B1, which discloses immunodominant peptides of MBP, PLP and MOG and their application for MS treatment. In the approach disclosed therein, the peptides are coupled to white or red blood cells.

Tolerance induction is antigen-specific and renders autoreactive T cells non-functional or anergic or induces regulatory T (Treg) cells that specifically suppress untoward autoimmunity to said target antigens. The induction of tolerance to target autoantigens is a highly important therapeutic goal in autoimmune diseases. It offers the opportunity to attenuate specifically the pathogenic autoimmune response in an effective way with few side effects. Tolerance induction can also be achieved by applying a whole protein instead of or in addition to an immunodominant peptide being a fragment of the protein (Kennedy et al., 1990, J Immunol, 144(3):909-915).

Some pathological characteristics of MS are reflected in the EAE model, a paradigmatic animal model of Th1/Th17 cell-driven autoimmune disease. Studies in relapsing EAE (R-EAE) in the SJL mouse have clearly shown that chronic demyelination involves the activation of T cell responses to an immunodominant myelin peptide, i.e. PLP 139-154, to which the first disease exacerbation is directed. Subsequently, the immune response broadens to other myelin peptides of PLP, MBP and MOG, a process, which is referred to as epitope spreading. Unresponsiveness of T cells, i.e. tolerance, can for example be induced when antigen presenting cells (APC) pulsed with antigenic peptide are for example treated with the cross linker 1-ethyl-3-(3-dimethylaminopropyl)-carbodiimide (ECDI; also abbreviated EDC).

Preclinical experiments have proven that a single i.v. injection of naïve murine splenocytes pulsed with a mixture of encephalitogenic myelin peptides and fixed with the cross-linker EDC is highly efficient in inducing peptide-specific tolerance in vivo. In EAE, this protocol not only prevented animals from disease, but even effectively reduced the onset and severity of all subsequent relapses when given after disease induction, indicating that specific tolerance can downregulate an ongoing autoimmune response (Miller et al., 1991, Acad Sci, 636:79-94). More relevant to the treatment of MS, studies in EAE have shown that tolerance can be simultaneously induced to multiple epitopes using a cocktail of encephalitogenic myelin peptides, thus providing the capacity to target autoreactive T cells with multiple specificities.

Tolerization of human T cells by autologous antigen-coupled cells, e. g. APCs (Vandenbark et al., 2000, Int Immunol, 12:57-66) or non-nucleated cells, i.e. red blood cells (RBCs), treated with EDC is effective *in vitro* as shown by failure of tolerized T cells to proliferate or to produce Th1 cytokines and a decreased expression of costimulatory molecules on these cells.

There is evidence that at least two distinct mechanisms are involved in the induction of antigen-specific tolerance by this regime:
1) Direct tolerance where Th1 clones encountering nominal antigen/MHC complexes on antigen-coupled APCs were anergized as a result of failure to receive adequate CD28-mediated costimulation or deleted, and
2) indirect mechanisms such as cross tolerance, where tolerance is induced by reprocessing and re-presentation of antigens by tolerogenic host APCs and/or expansion of Treg cells.

The latter cross tolerance likely involves the induction and/or expansion of antigen-specific Treg cells which assumption is also supported by data obtained in a phase Ib trial as disclosed herein. Further, treatment of cells with EDC induces apoptosis in a substantial percentage of treated cells. Thus, an indirect mechanism that involves APC that take up fixed cells undergoing apoptosis, which are then processed and represented by host APC, is likely. This is further supported by effective induction of tolerance in MHC-deficient and allogeneic mice. In-vitro bone marrow-derived dendritic cells effectively phagocytose and process antigen-pulsed, fixed APC.

Currently approved therapies for MS involve various antigen-nonspecific immunomodulating or immunosuppressive strategies, which are only partially effective. All current therapeutics need to be taken orally daily or injected/infused at various time intervals and for long periods of time. Further, they are associated with numerous and sometimes severe side effects.

A therapy that addresses the pathogenesis of MS at its roots should aim to specifically delete or functionally inhibit pathogenic autoreactive cells without altering the "normal" immune system. This is of importance because global immunomodulation and/or immunosuppression come at the cost of inhibiting beneficial regulatory cells and immune cells that serve protective functions against pathogens.

Ideally, a therapy should be personalized taking into account a patient's specific characteristics, such as the genetic background or the ability of the patient's immune system to react to certain antigens.

A personalized therapy can thereby help improving the patient's outcome. For a personalized treatment regime, MS stratification is important, i. e. the identification of subtypes that are particularly responsive to a certain treatment.

### SUMMARY OF THE INVENTION

It is an object of the present invention to improve MS patient stratification in order to develop personalized treatment regimens. It is also an object of the present invention to develop antigen-specific tolerization strategies, in particular to treat certain MS patient subgroups.

In a first aspect of the invention, a method for stratification of a multiple sclerosis (MS) patient is provided comprising the following steps:
- obtaining body fluid, in particular blood, preferably peripheral blood, or cerebrospinal fluid (CSF), from an MS patient, and
- detecting CD27- Th1 CD4+ cells in the body fluid.

In a particular embodiment, the method further comprises:
- detecting responsiveness of T cells and/or antibodies in the body fluid to the protein GDP-L-fucose synthase (GDP-L-FS) or a fragment, derivative and/or splice variant thereof.

In a further particular embodiment, the protein GDP-L-FS
a) has an amino acid sequence as set forth in SEQ ID NO: 1 or
b) has an amino acid sequence which is at least 85 %, preferably at least 90 %, more preferably at least 95 % identical to the amino acid sequence as set forth in SEQ ID NO: 1 or
c) has an amino acid sequence which is at least 70 %, preferably at least 80 %, more preferably at least 90 % homologous to the amino acid sequence as set forth in SEQ ID NO: 1 or
d) has an amino acid sequence which is at least 60 %, preferably at least 70 %, more preferably at least 80 %, even more preferably at least 90 % homologous to the amino acid sequence as set forth in SEQ ID NO: 1 and the protein or fragment or splice variant thereof binds to an autologous HLA allele, is recognized by a T cell and/or is recognized by an antibody which binds to or recognizes the amino acid sequence as set forth in SEQ ID NO: 1 or a fragment thereof or
e) is encoded by a TSTA3 gene, in particular by a gene sequence of nucleotides 143612618 to 143618048 of NC_000008.11, or is encoded by a gene which is at least 80 %, preferably at least 90 %, even more preferably at least 95 % identical to the gene sequence of nucleotides 143612618 to 143618048 of NC_000008.11.

In one embodiment, the HLA allele is the HLA allele DRB3*02:02 or the HLA allele DRB3*03:01.

Preferably, the fragment comprises 5 to 50, preferably 5 to 20, more preferably 10 to 15 amino acids, even more preferably 15 amino acids.

Preferably, the fragment is
a) at least 85 %, preferably at least 90 %, more preferably at least 95 % identical to a respective corresponding amino acid sequence or
b) at least 70 %, preferably at least 80 %, more preferably at least 90 % homologous to a respective corresponding amino acid sequence or
c) at least 60 %, preferably at least 70 %, more preferably at least 80 %, even more preferably at least 90 % homologous to a respective corresponding amino acid sequence and binds to an autologous HLA allele, is recognized by a T cell and/or is recognized by an antibody which binds to or recognizes the respective amino acid sequence.

In a further preferred embodiment, the fragment comprises a sequence selected from the group comprising SEQ ID NOs: 2 to 6 and SEQ ID NO: 37, preferably consists of a sequence selected from the group comprising SEQ ID NOs: 2 to 6 and SEQ ID NO: 37. In another preferred embodiment, the fragment comprises or consists of any sequence that lies within the sequence as defined by SEQ ID NO: 37.

In a second aspect of the invention, a GDP-L-FS protein or a fragment, derivative or splice variant thereof, or a nucleotide sequence encoding the GDP-L-FS protein or fragment, derivative or splice variant thereof as defined *supra* is provided, for use in the treatment of MS in an MS patient, wherein CD27- Th1 CD4+ cells are detected in body fluid, in particular blood, preferably peripheral blood, or CSF, previously obtained from the MS patient.

In a particular embodiment, T cells and/or antibodies previously obtained from the body fluid of the MS patient respond to the protein GDP-L-FS or a fragment, derivative and/or splice variant thereof.

In a third aspect of the invention, at least one GDP-L-FS protein, fragment, derivative, splice variant, nucleotide sequence and/or gene sequence as defined above is provided, and/or at least one carrier coupled to at least one GDP-L-FS protein, fragment, derivative, splice variant, nucleotide sequence and/or gene sequence as defined above is provided for use in a method for inducing antigen-specific tolerance to autoantigens in an MS patient, wherein CD27- Th1 CD4+ cells are detected in body fluid, in particular blood, preferably peripheral blood, or CSF, previously obtained from the MS patient.

In a particular embodiment, T cells and/or antibodies previously obtained from the body fluid of the MS patient respond to the protein GDP-L-FS or a fragment, derivative and/or splice variant thereof.

In a further particular embodiment, the at least one GDP-L-FS protein, fragment, derivative, splice variant, nucleotide sequence and/or gene sequence and/or the at least one carrier coupled to at least one GDP-L-FS protein, fragment, derivative, splice variant, nucleotide sequence and/or gene sequence is applied by nasal, inhaled, oral, subcutaneous (s.c.), intracoelomic (i.c), intramuscular (i.m.), intradermal (i.d.), transdermal (t.d.) or intravenous (i.v.) administration, preferably by i.v., s.c., i.d., t.d., oral, inhaled or nasal administration.

In a fourth aspect of the invention, CD27- Th1 CD4+ cells for use in a method of monitoring the response to the method for inducing antigen-specific tolerance as disclosed *supra* are provided, wherein the CD27- Th1 CD4+ cells are detected in body fluid, in particular blood, preferably peripheral blood, or CSF, previously obtained from an MS patient.

In a particular embodiment, additionally a responsiveness of T cells and/or antibodies previously obtained from the body fluid of the MS patient to the protein GDP-L-FS or a fragment, derivative and/or splice variant thereof as defined *supra* is detected.

Preferably, the CD27- Th1 CD4+ cells are further negative for the markers CCR7 and/or CD45RA.

In a particular embodiment, the MS patient has one or more of the following characteristics:
- inflammation and/or neurodegeneration in the central nervous system, in particular characterized by a Gd-contrast enhancing T1 lesion and/or a FLAIR T2 lesion,
- higher expression of genes associated with Th1 cells or cytotoxicity and/or genes encoding proinflammatory cytokines, such as IL-2 and/or IFN-γ, compared with healthy controls,
- HLA allotype HLA-DRB3*02:02 or DRB3*03:01.

In another aspect, a method for stratifying an MS patient is described herein, the method comprising: detecting in a sample obtained from the patient CD27- Th1 CD4+ cells, to thereby stratify the patient.

In an embodiment, the sample comprises body fluid.

In an embodiment, the body fluid comprises blood, e.g., peripheral blood, or cerebrospinal fluid (CSF).

In an embodiment, the method comprises detecting responsiveness of T cells and/or antibodies in the body fluid to the protein GDP-L-fucose synthase (GDP-L-FS) or a fragment, derivative and/or splice variant thereof.

In another aspect, a method for treating an MS patient is described herein, the method comprising: detecting in a sample obtained from the patient CD27- Th1 CD4+ cells, and administering to the patient an MS therapy, to thereby treat the patient.

In an embodiment, T cells and/or antibodies previously obtained from the body fluid of the patient respond to the protein GDP-L-FS or a fragment, derivative and/or splice variant thereof.

In an embodiment, the MS therapy comprises immunodominant peptides.

In an embodiment, the MS therapy comprises treating the patient with antigen-specific immunotherapies, such as tolerance induction.

In an embodiment, treating the patient comprises administering to the patient immunodominant peptides selected from MBP, PLP and MOG, e.g., disclosed in EP 2 205 273 B1.

In an embodiment, treating the patient comprises administering to the patient immunodominant proteins or peptides selected from GDP-L-FS or a fragment, derivative or splice variant thereof, and a protein from the RASGRP family or a fragment, derivative or splice variant thereof, e. g., disclosed in WO 2020/002674.

In an embodiment, the immunodominant peptides are coupled, e.g., chemically, to white or red blood cells.

In an embodiment, the sample comprises body fluid.

In an embodiment, the body fluid comprises blood, e.g., peripheral blood, or cerebrospinal fluid (CSF).

### BRIEF DESCRIPTION OF THE FIGURES

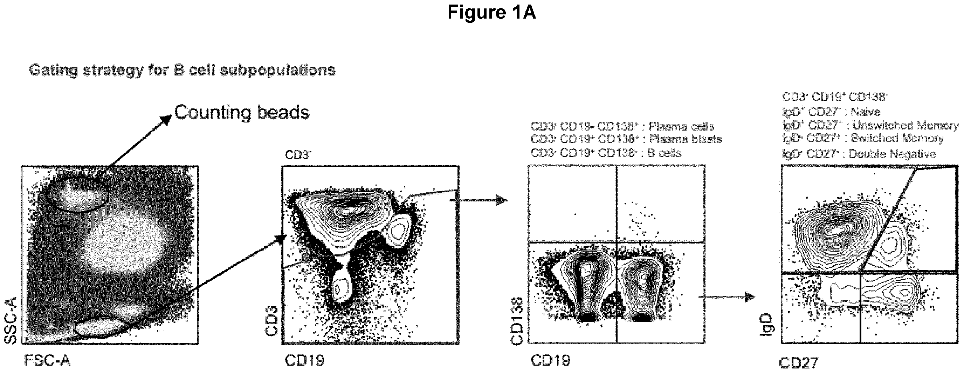

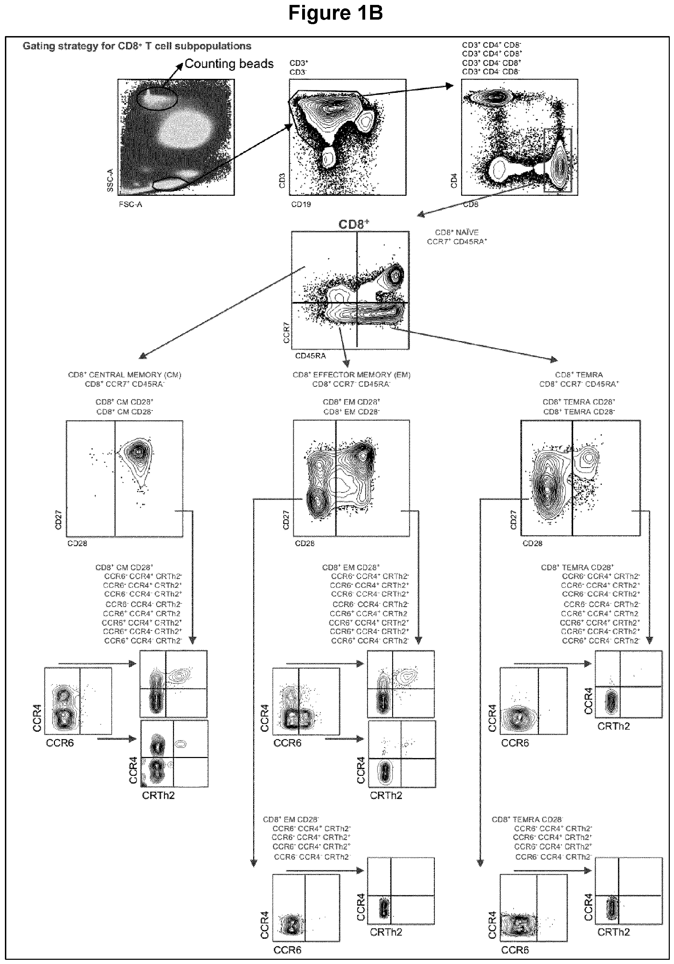

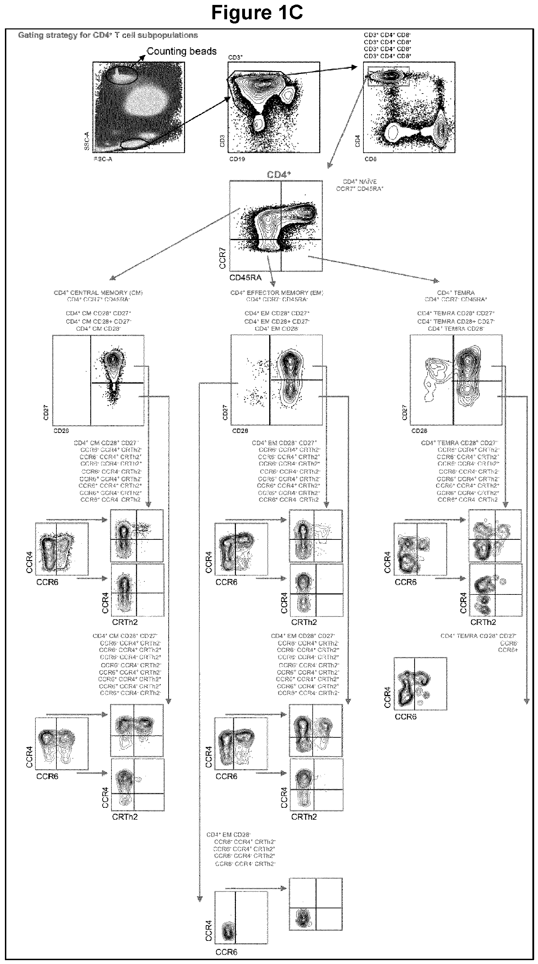

**Figure 1****. Flow cytometry gating strategy. (A-C)** First doublets are excluded, followed by identification of lymphocytes by size. **A.** CD3- are identified and among them, plasma cells (CD19- CD138+), plasma blasts (CD19+ CD138+), B cells (CD19+ CD138-) and CD19- CD138-cells. Among B cells, naïve (IgD+ CD27-), unswitched memory (IgD+CD27+), switched memory (IgD- CD27+) and double negative (IgD- CD27-) B cell subsets are also identified. **B.** In CD3+ T cells, CD3+ CD8+ cells are first identified and then separated in CM (CCR7+ CD45RA-), EM (CCR7- CD45RA-), TEMRA (CCR7- CD45RA+) and naive (CCR7+ CD45RA+). CM, EM and TEMRA CD8+ T cells are then separated in CD28+ and CD28-. Each one of these CD8+ T cells are separated first in CCR6- and CCR6+ and then in Th1 (CCR6- CCR4- CRTH2-), Th2-A (CCR6- CCR4+ CRTH2-), Th2-B (CCR6- CCR4+ CRTH2+), CCR6- CCR4- CRTH2+, Th1* (CCR6+ CCR4- CRTH2-), Th17 (CCR6- CCR4+ CRTH2-), CCR6+ CCR4+ CRTH2+ and CCR6+ CCR4- CRTH2+ cells. **C.** In CD3+ T cells, CD3+ CD4+ cells are first identified and then separated in CM (CCR7+ CD45RA-), EM (CCR7- CD45RA-), TEMRA (CCR7- CD45RA+) and naive (CCR7+ CD45RA+). CM, EM and TEMRA CD4+ T cells are then separated in CD28+ CD27+, CD28+ CD27- and CD28-. Each one of these CD4+ T cells are separated first in CCR6- and CCR6+ and then in Th1 (CCR6- CCR4- CRTH2-), Th2-A (CCR6- CCR4+ CRTH2-), Th2-B (CCR6- CCR4+ CRTH2+), CCR6- CCR4- CRTH2+, Th1* (CCR6+ CCR4- CRTH2-), Th17 (CCR6- CCR4+ CRTH2-), CCR6+ CCR4+ CRTH2+ and CCR6+ CCR4- CRTH2+ cells. SPHEROTM AccuCount Particles have been used to determine absolute counts. Antibodies: anti-CD3 AF700, anti-CD4 PE TR, anti-CD8 BV510, anti-CD45RA BV711, anti-CCR7 BV421, anti-CD27 APC Cy7, anti-CD28 PE Cy7, anti-CCR4 APC, anti-CRTh2 PE, anti-CCR6 BV785, anti-CD19 PerCPCy5.5, anti-lgD BV605 and anti-CD138 FITC.

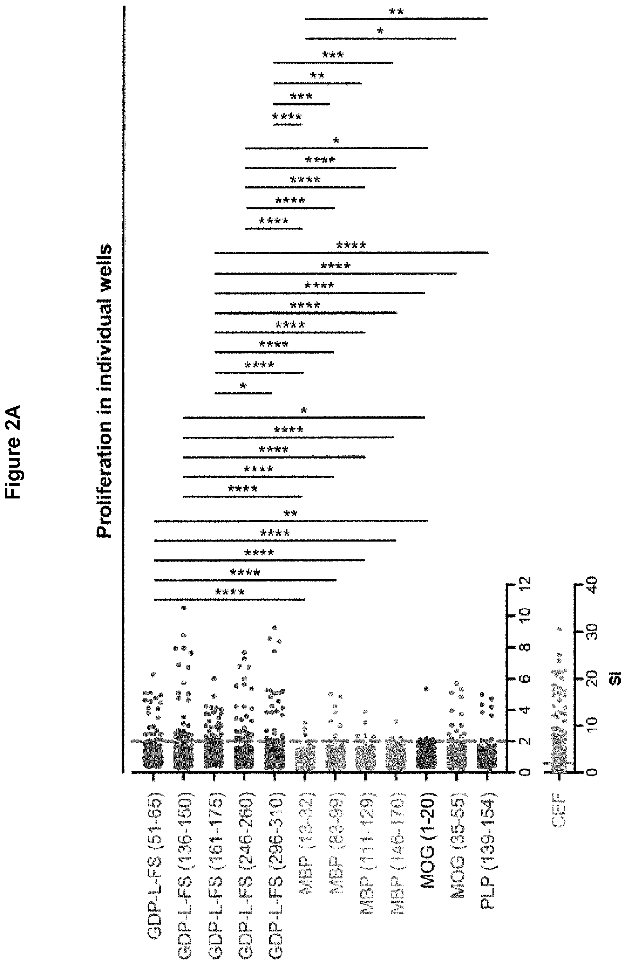

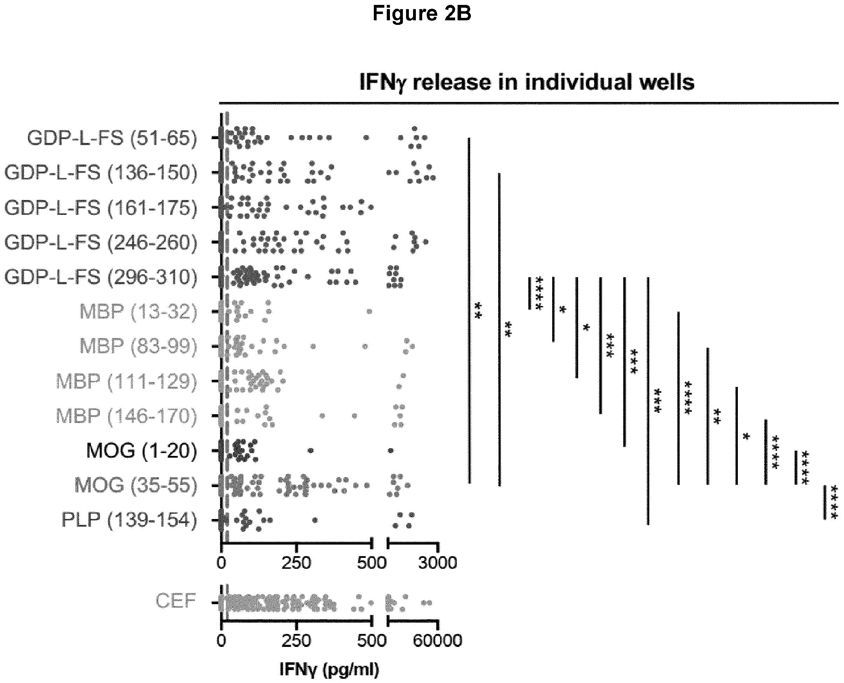

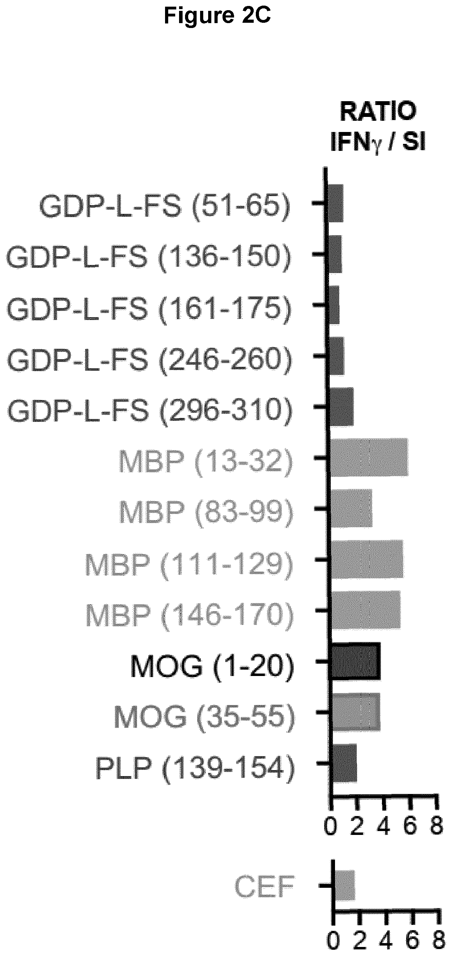

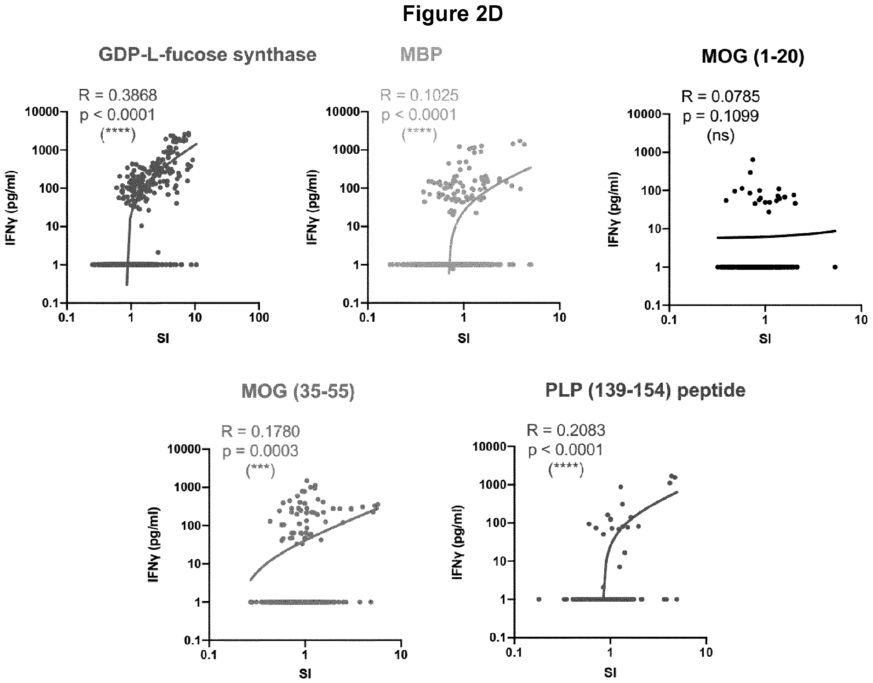

**Figure 2****. Recognition of GDP-L-FS and myelin derived peptides by CSF-infiltrating CD4+ T cells from patients with MS. A+B.** Proliferative responses expressed as stimulation indexes (SI) and IFN-γ release expressed as (pg/ml) of PHA-expanded CSF-infiltrating CD4+ T cells against GDP-L-FS, myelin (MBP, MOG(1-20), MOG(35-55), PLP) and CEF peptides presented by autologous PBMCs. Each dot represents one well. Each peptide has been tested in quadruplicates (4 wells) in 105 MS patients (420 wells in total per peptide). Dotted line shows the threshold for positivity (SI ≥ 2 for proliferation and pg/ml of IFN-γ ≥ 20 for IFN-γ release). Kruskal-Wallis test was used to compared peptide responses. Statistical significance of all comparisons (* p<0.05, ** p<0.01, *** p<0.001 and **** p<0.0001) is shown. **C.** Ratio of % of positive wells using IFN-γ release and proliferation for each peptide. **D.** Correlation between Sls and IFN-γ release (pg/ml) for GDP-L-FS, MBP, MOG(1-20), MOG(35-55) and PLP(139-154) peptides. Spearman r was used to test linear correlation between variables. R as well as p values are shown.

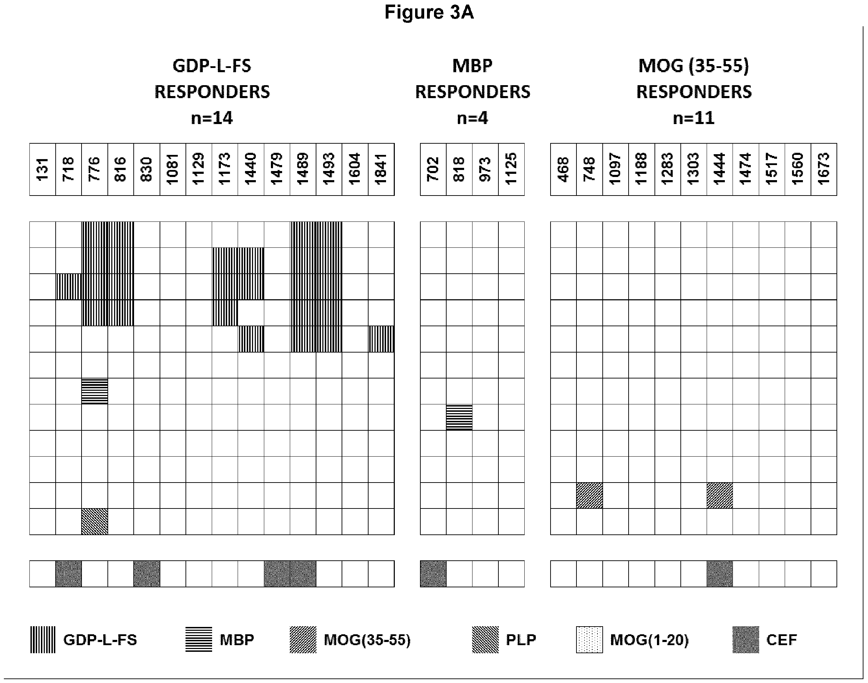

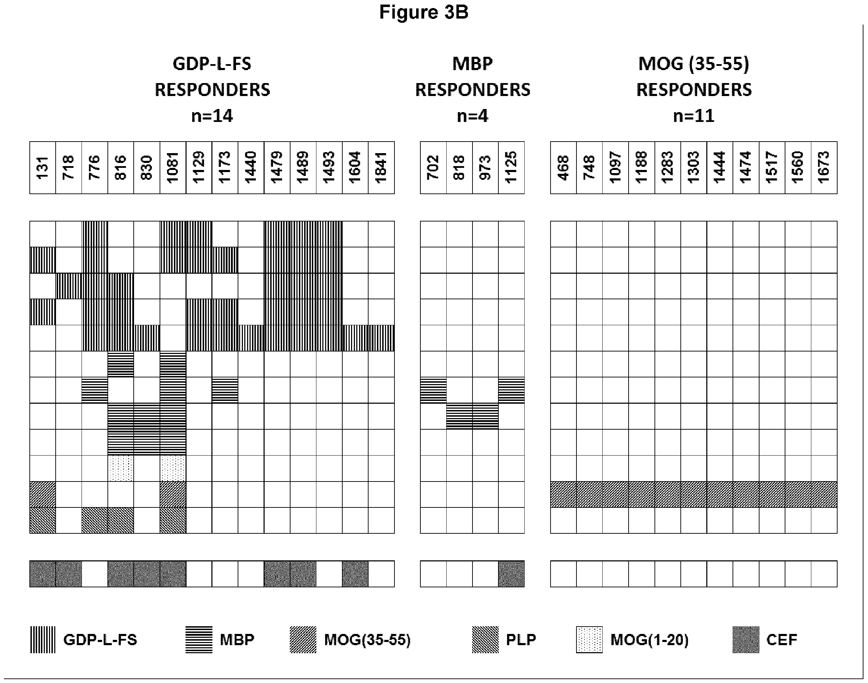

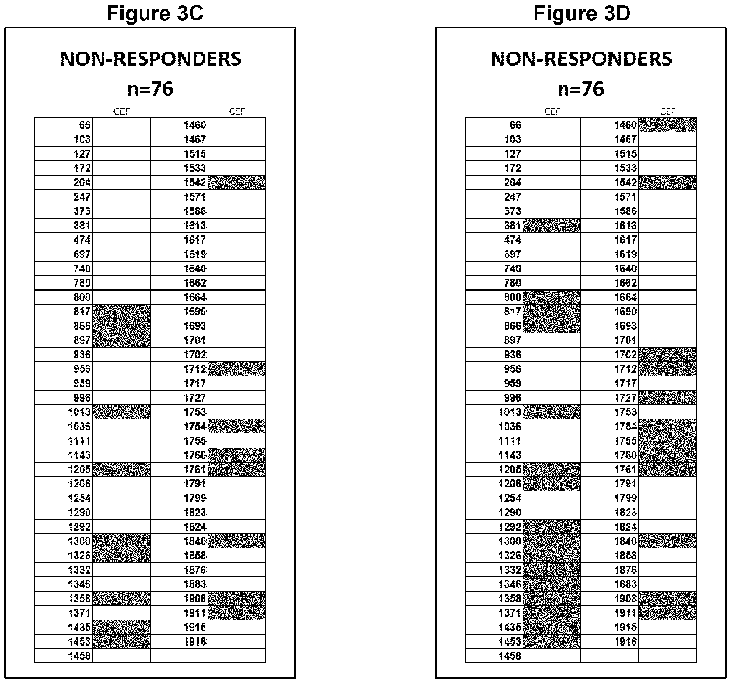

**Figure 3****. Identification of GDP-L-FS and myelin-responder patients.** Checkerboard graphs illustrating the response of each MS patient to the individual peptides. Filled and shaded cells are positive responses in proliferation (**3A**) and IFN-γ release (**3B**). Non-responders are shown as **3C** (proliferation) and **3D** (IFN-γ release). Number of GDP-L-FS-, MBP-, MOG(35-55)- and non-responders are shown.

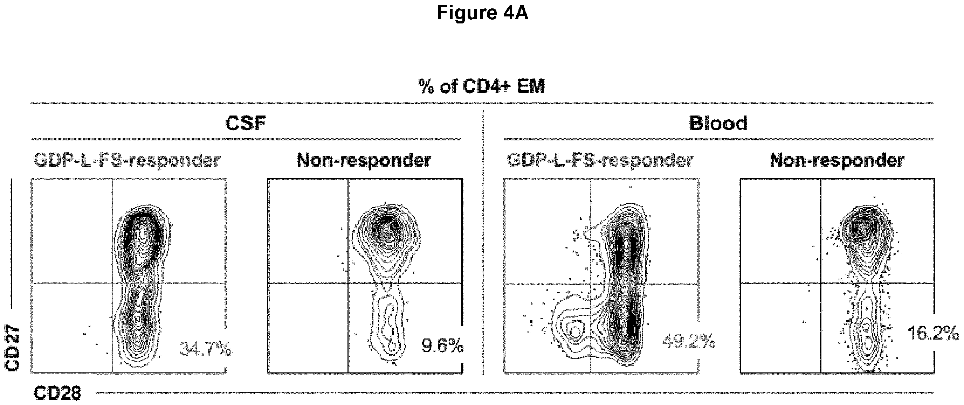

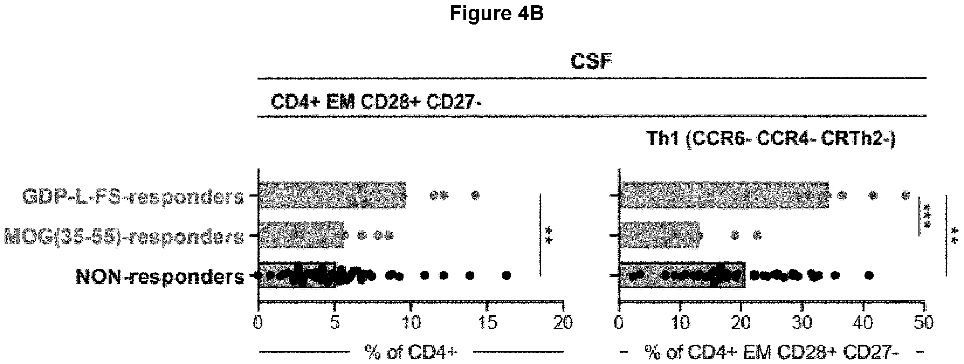

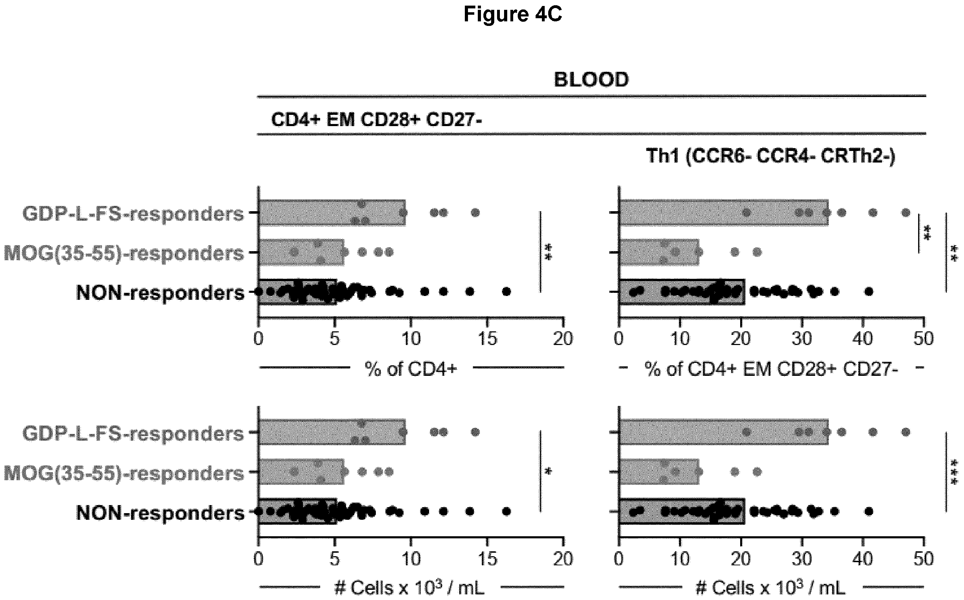

**Figure 4****. Distinct CSF-infiltrating and circulating lymphocytes in GDP-L-FS-, MOG(35-55)- and non-responder patients. A.** Dot plot showing CD28 and CD27 expression on CSF-infiltrating and peripheral circulating EM CD4⁺ cells from a GDP-L-FS- and a non-responders. Percentages of EM CD27- cells are shown. **B-C.** Frequencies of CSF-infiltrating **(B)** and frequencies and absolute numbers of peripheral circulating **(C)** EM CD27- and EM CD27- Th1 cells in GDP-L-FS, MOG 35-55 and non- responders. Cell counts were determined using SPHEROTM AccuCount Particles. Each dot corresponds to a single patient and bars show means. Kruskal-Wallis test was used to compare patients. Statistical significance (* p<0.05, ** p<0.01, *** p<0.001 and **** p<0.0001) is shown.

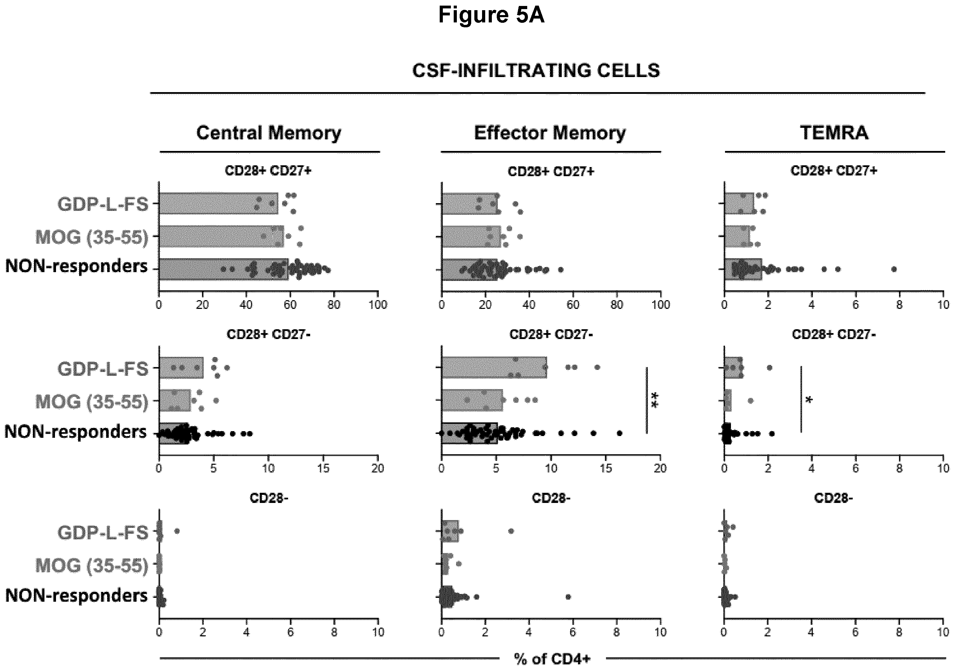

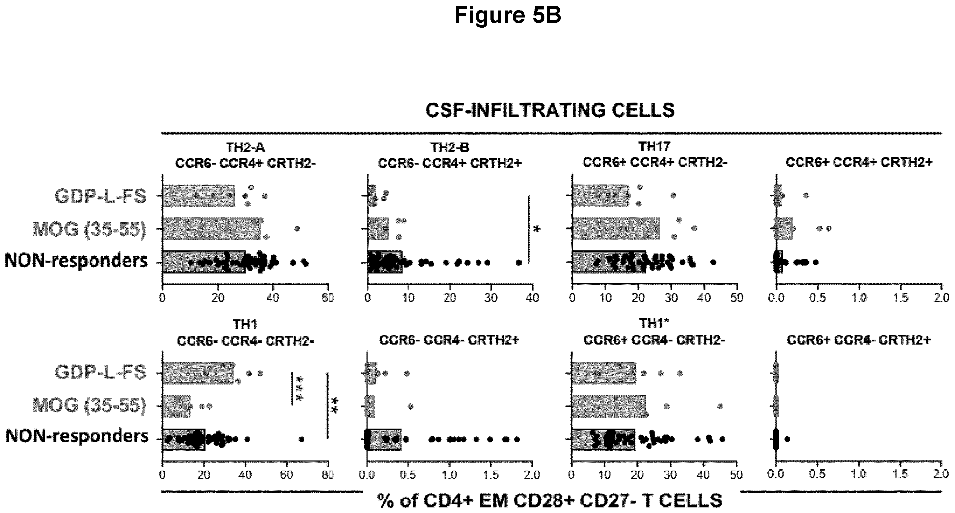

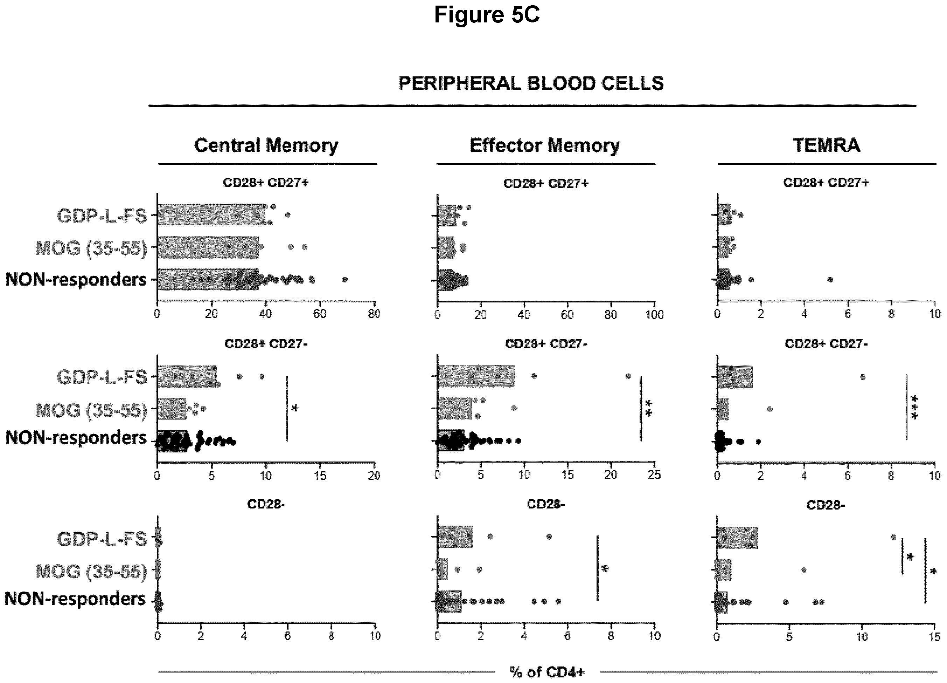

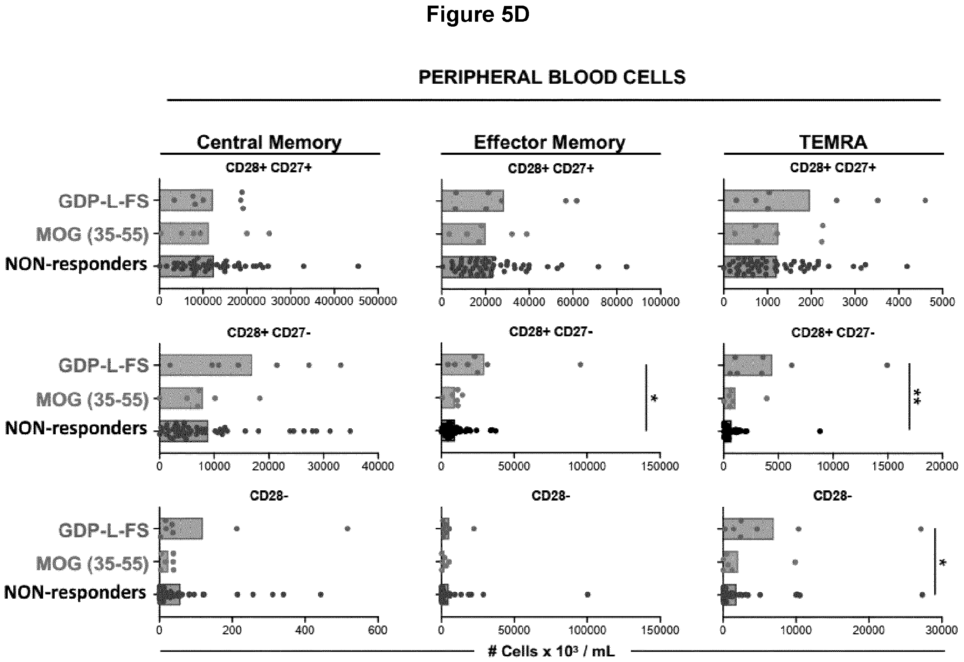

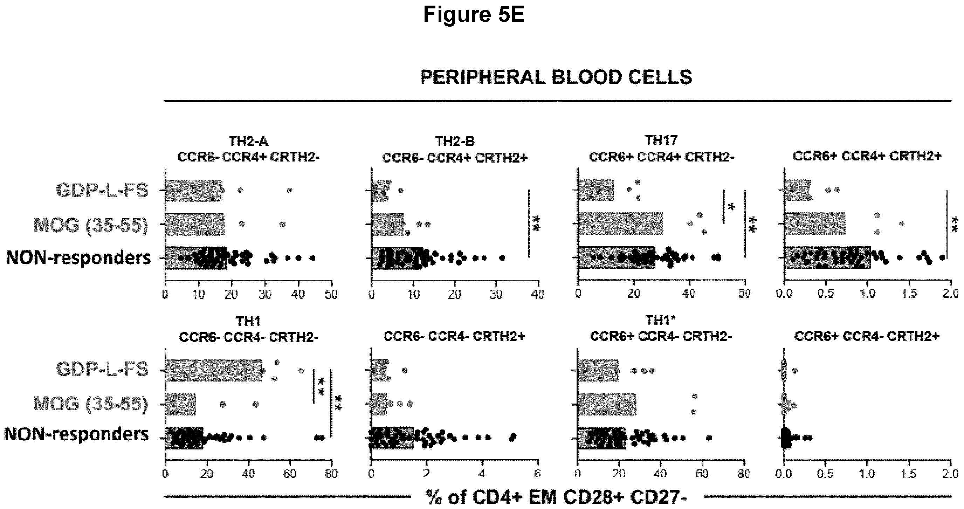

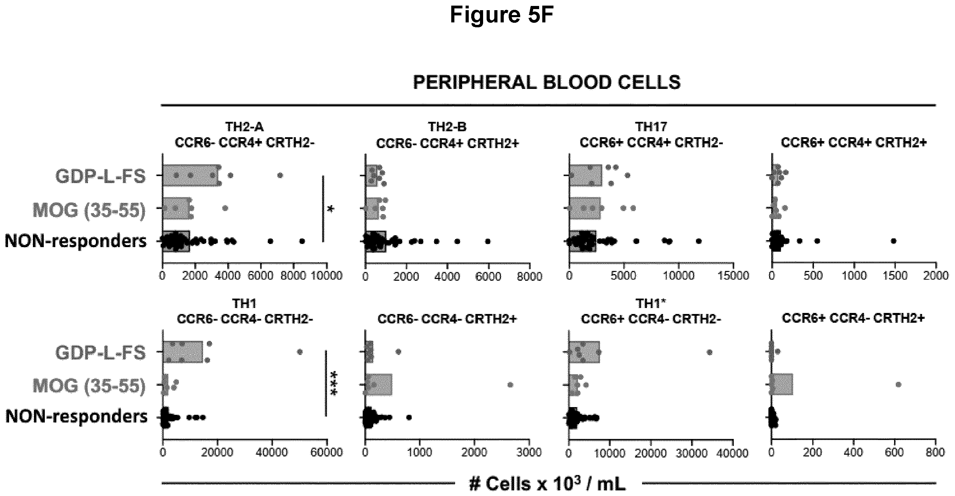

**Figure 5****. Ex-vivo flowcytometry immunophenotyping of CSF-infiltrating and circulating T lymphocytes.** CSF-infiltrating **(A-B)** and peripheral circulating **(C-F)** frequencies as well as real counts of central memory (CM, CCR7+ CD45RA-), effector memory (EM, CCR7- CD45RA-) and TEMRA (CCR7- CD45RA+) CD4+ T cell subsets expressing CD28+CD27+, CD28+CD27- and CD28-, as well as EM CD28+ CD27- CD4+ T cells with the following functional phenotypes based on the chemokine receptor expression: Th2A (CCR6- CCR4+ CRth2-), Th2B (CCR6-CCR4+ CRth2+), CCR6- CCR4- CRth2+, Th1 (CCR6- CCR4- CRTh2-), Th17 (CCR6+ CCR4+ CRth2-), CCR6+ CCR4+ CRth2+, CCR6+ CCR4- CRth2+ and Th1* (CCR6+ CCR4- CRth2-). Each dot in the graphs corresponds to a single patient and the bars show the mean. Cell counts have been determined using SPHEROTM AccuCount Particles. Kruskal-Wallis test was used to compare GDP-L-FS, MOG 35-55 and non-responder patients. Statistical significance (* p<0.05, ** p<0.01, *** p<0.001 and **** p<0.0001) is shown.

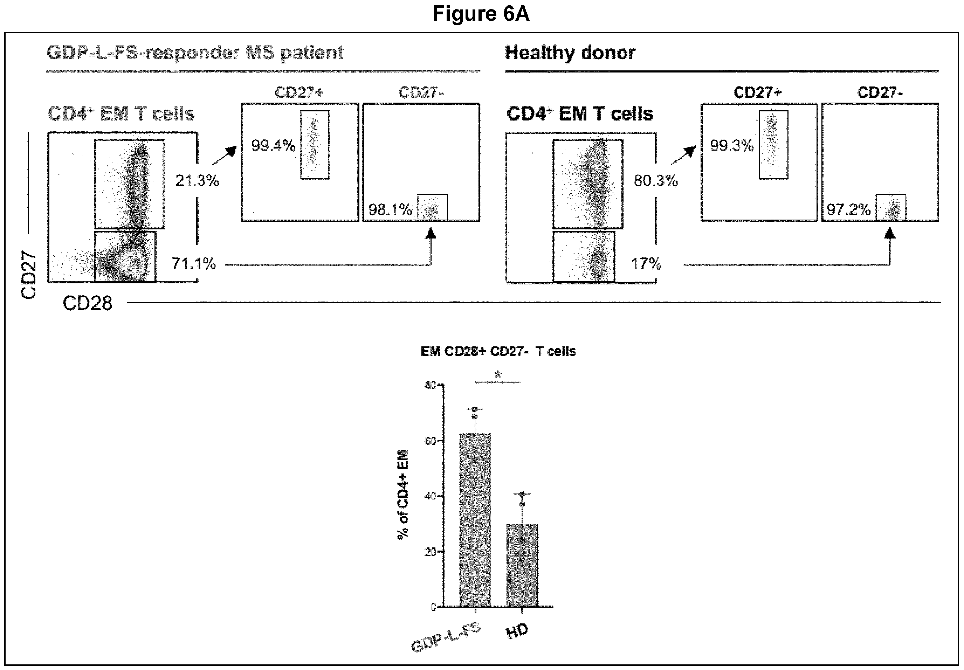

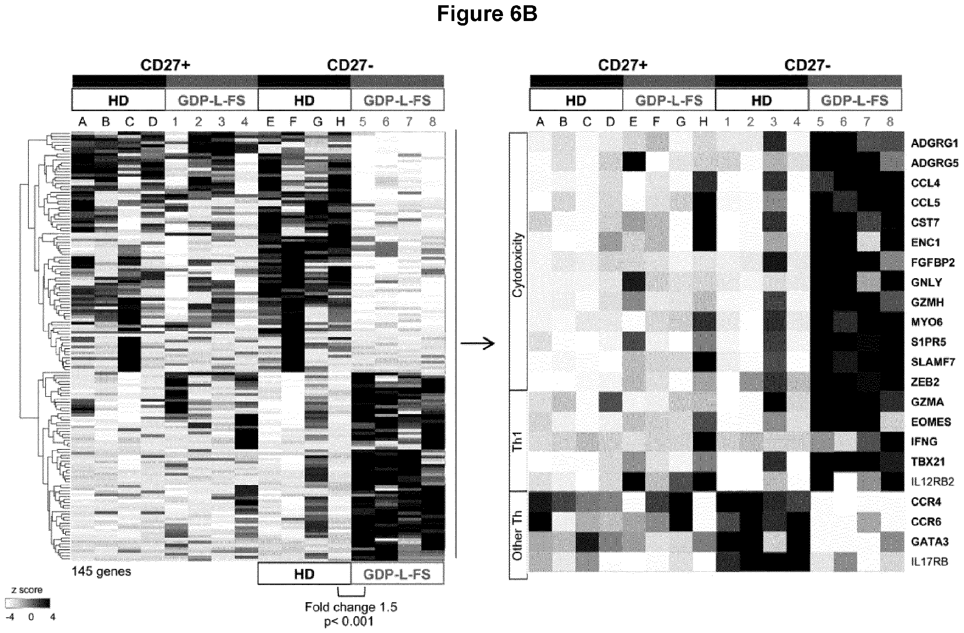

**Figure 6****. Purification and transcriptome analysis of EM CD27+/CD27- cells. A.** Gating strategy used to isolate EM CD28+ CD4+ T cells expressing or not CD27 from four GDP-L-FS-responder MS patients and four HD. The frequencies of EM CD28+ CD4+ T cells expressing or not CD27 before and after cell sorting from one representative GDP-L-FS-responder MS patient and one HD are shown. Mann-Whitney test has been used to compare the frequency of EM CD27 cells in GDP-L-FS-responder patients and HD and statistical significance (* p<0.05) is shown. **B**. Heat map showing row-wise z scores of 145 differentially expressed transcripts identified by RNA-seq analysis and pairwise comparison of EM CD27- from GDP-L-FS responders (columns 5-8) versus EM CD27- from HD (columns E-H) (Log2Ratio > 0.5, p< 0.001). Heat map also shows the row-wise z scores of these 145 transcripts in EM CD27+ cells from GDP-L-FS-responders (columns 1-4) and HD (columns A-D). Row-wise z scores of selected transcripts associated with cytotoxicity, Th1 and other Th subsets are shown in detail. In bold are genes that also were identified as differentially expressed by RNA-seq analysis and pairwise comparison of EM CD27- versus EM CD27+ in GDP-L-FS patients (Log2Ratio > 0.5, p< 0.001).

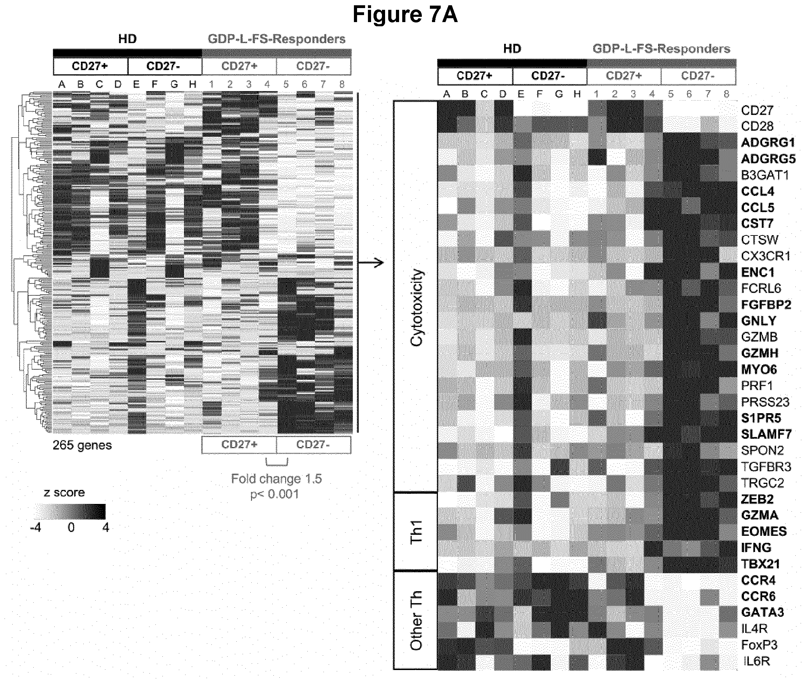

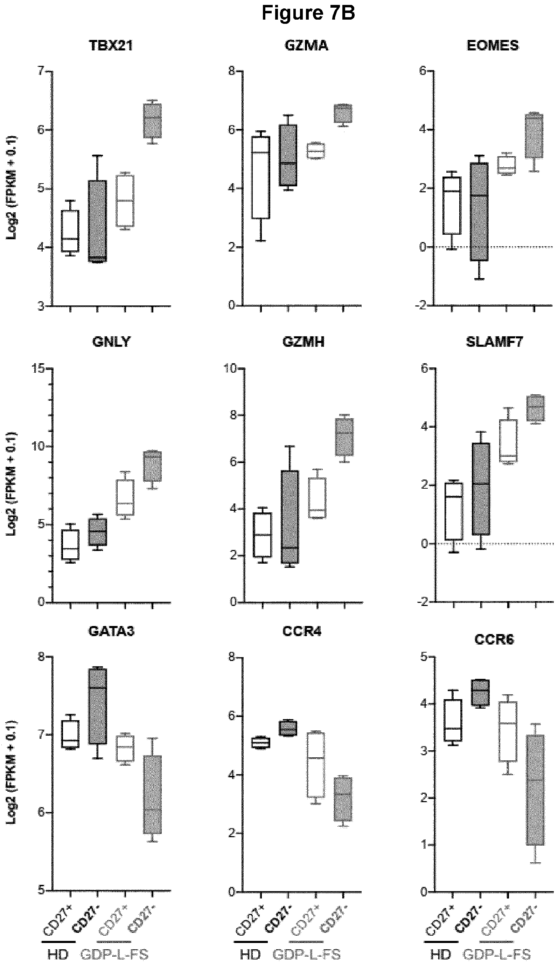

**Figure 7****. Transcriptome analysis of EM CD27- and EM CD27+ CD4+ T cells. A.** Heat map shows the row-wise z scores of 265 transcripts differentially expressed between EM CD27+ (columns 1-4) and EM CD27- (columns 5-8) cells (Log2Ratio > 0.5, p< 0.001) from four GDP-L-FS-responder patients. Z scores of these genes in EM CD27+ (columns A-D) and EM CD27-(columns E-H) cells from HD are also shown. Z scores of selected genes associated with cytotoxicity, Th1 and other Th subsets are shown in detail. In bold are genes that also were differentially expressed in EM CD27- from GDP-L-FS-responders versus EM CD27- from HD (Log2Ratio > 0.5, p< 0.001). **B.** Distribution of log2(Fragments Per Kilobase per Million (FPKM) + 0.1) gene expression of Th1/cytotoxic genes and genes associated with other Th subsets in EM CD27+ and CD27- cells from GDP-L-FS-responders and HD.

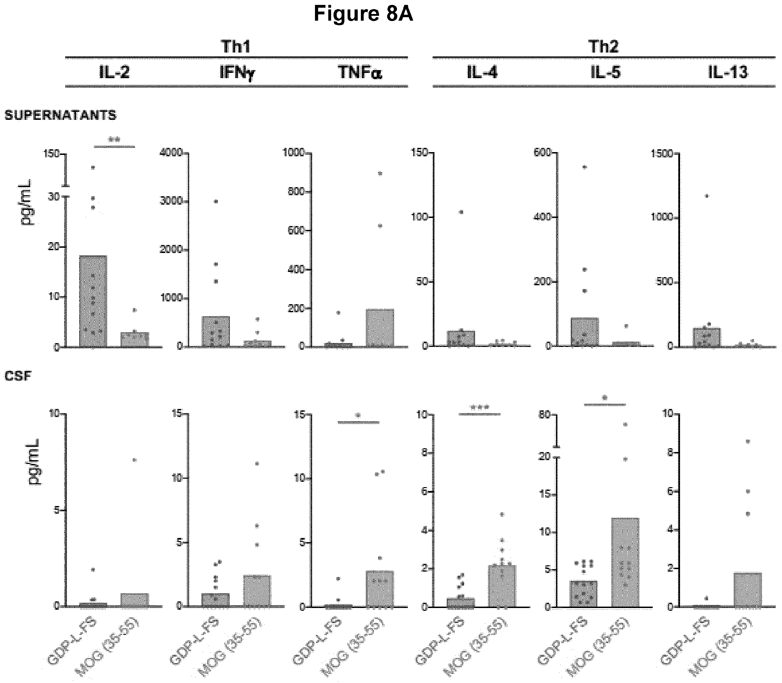

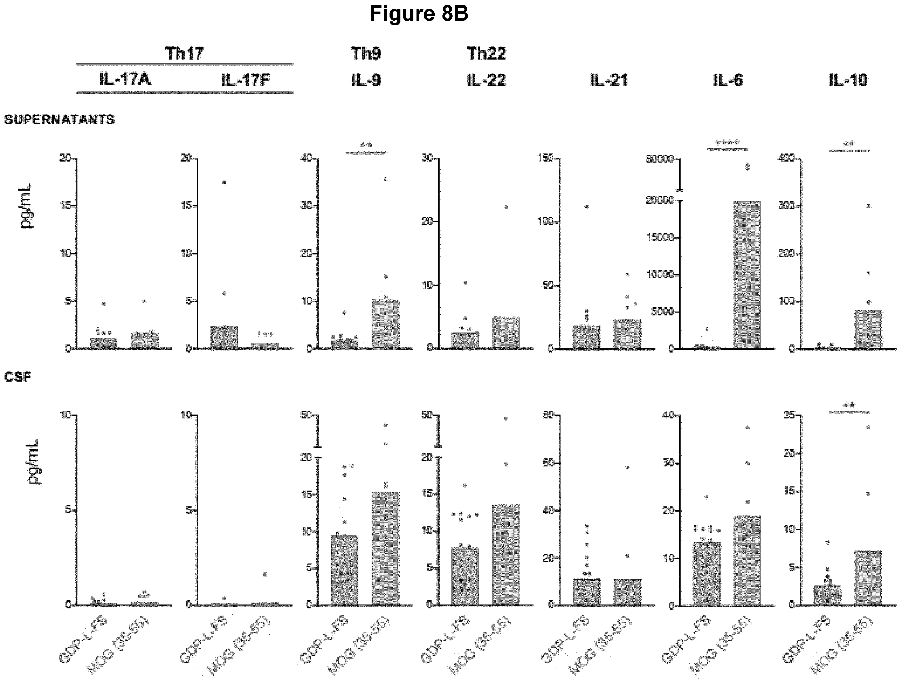

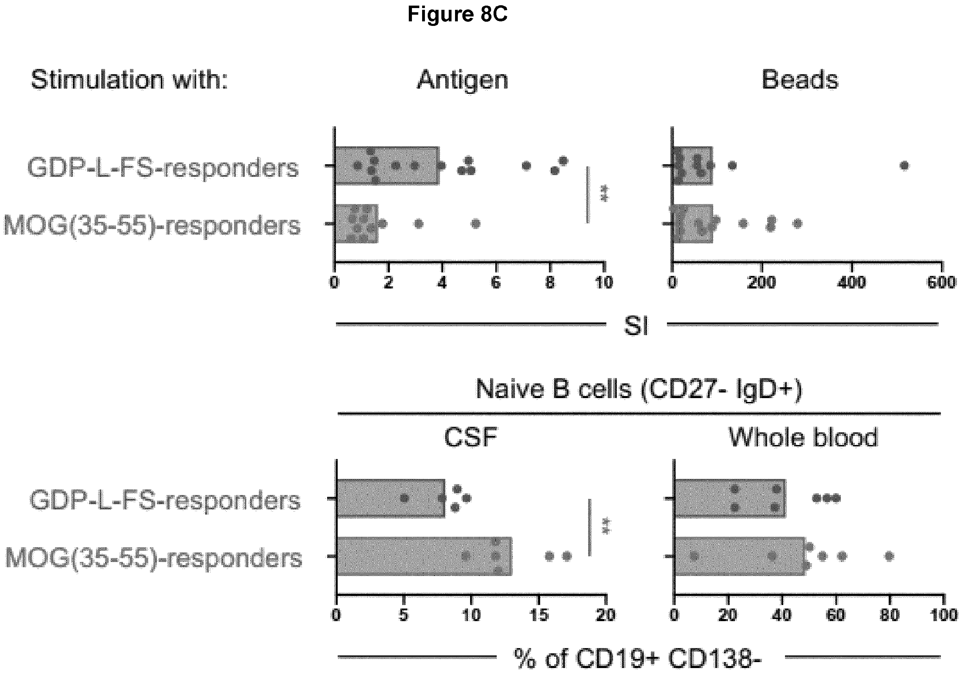

**Figure 8****. Characterization of GDP-L-FS- and MOG (35-55)-specific responses. A+B.** Upper graphs, cytokines released by CSF-infiltrating CD4⁺ T cells from GDP-L-FS- and MOG(35-55)-responders after stimulation with specific peptides (GDP-L-FS and MOG(35-55)) presented by autologous PBMCs. Four wells were pooled for each patient. Lower graphs, cytokines present in the CSF of GDP-L-FS- and MOG(35-55)-responders. Cytokine are expressed as pg/ml. **C.** Upper graph: Proliferative responses of CSF-infiltrating CD4⁺ T cells from GDP-L-FS- and MOG(35-55)-responders after stimulation with the specific peptides presented by autologous PBMCs or anti-CD3, anti-CD28, anti-CD2 stimulating beads. Proliferative responses are expressed as SI. Lower graph: Frequencies of CSF-infiltrating and blood circulating Naïve (lgD+ CD27-) B (CD19+ CD138-) cells in GDP-L-FS- and MOG 35-55-responders. Each dot in the graphs corresponds to a single patient and the bars show the mean. Mann-Whitney test was used to compare GDP-L-FS- and MOG(35-55)-responders. Statistical significance (* p<0.05, ** p<0.01, *** p<0.001 and **** p<0.0001) is shown.

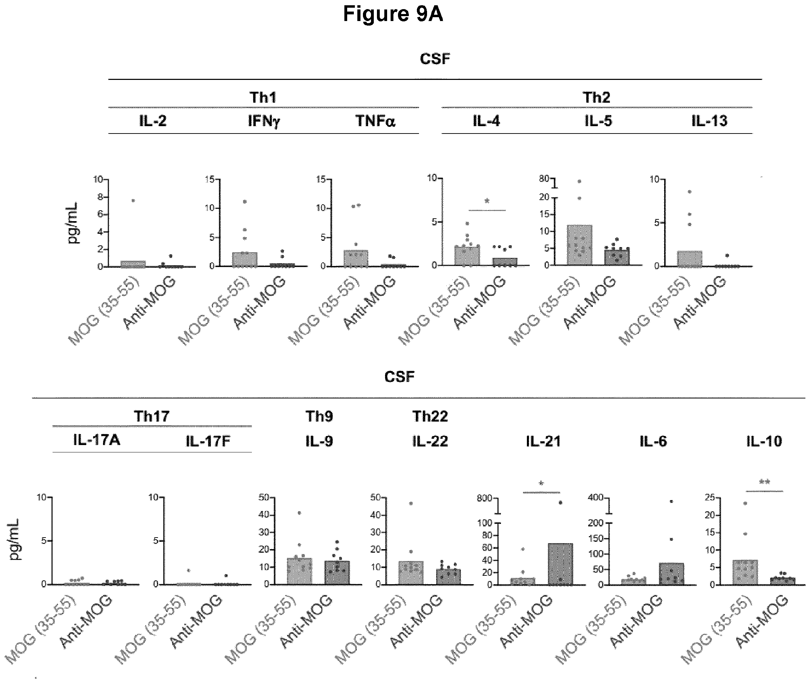

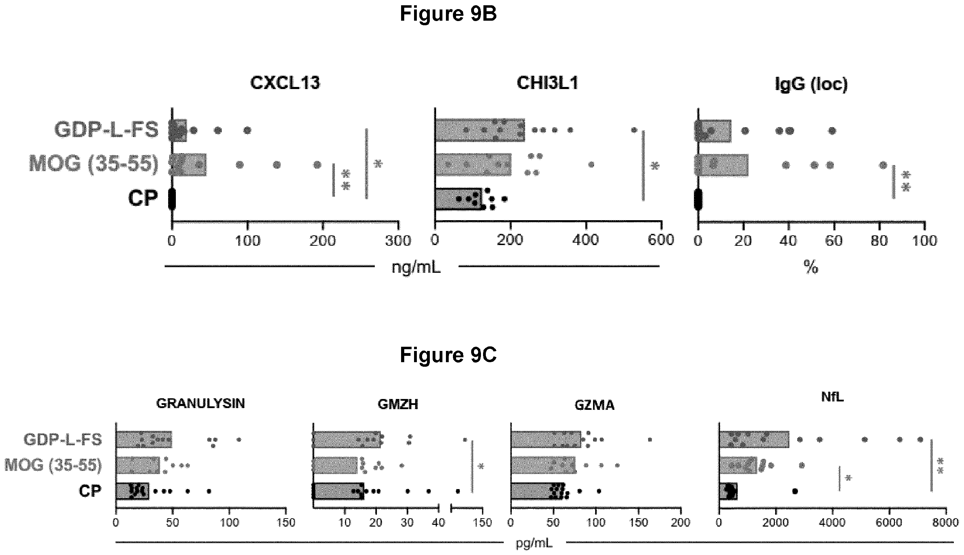

**Figure 9****. Additional characterization of CSF from patients and controls. A.** Cytokines present in the CSF of MOG(35-55)-responders and MOGAD patients. Cytokine release is expressed as pg/ml. **B-C.** Intrathecal abundance in GDP-L-FS- and MOG(35-55)-responders and CP of: **B**, CXCL13, Chitinase-3-like protein 1 (CHI3L1) and intrathecal IgG synthesis IgG(loc); **C**, granulysin, granzyme H (GZMH), granzyme A (GZMA) and Neurofilament Light Chain (NfL). Each dot in the graphs corresponds to a single patient and the bars show the mean. Mann-Whitney test was used to compare MOG(35-55)-responders and anti-MOG patients and Kruskal-Wallis to compare GDP-L-FS-responders, MOG(35-55)-responders and CP. Statistical significance (* p<0.05, ** p<0.01, *** p<0.001 and **** p<0.0001) is shown.

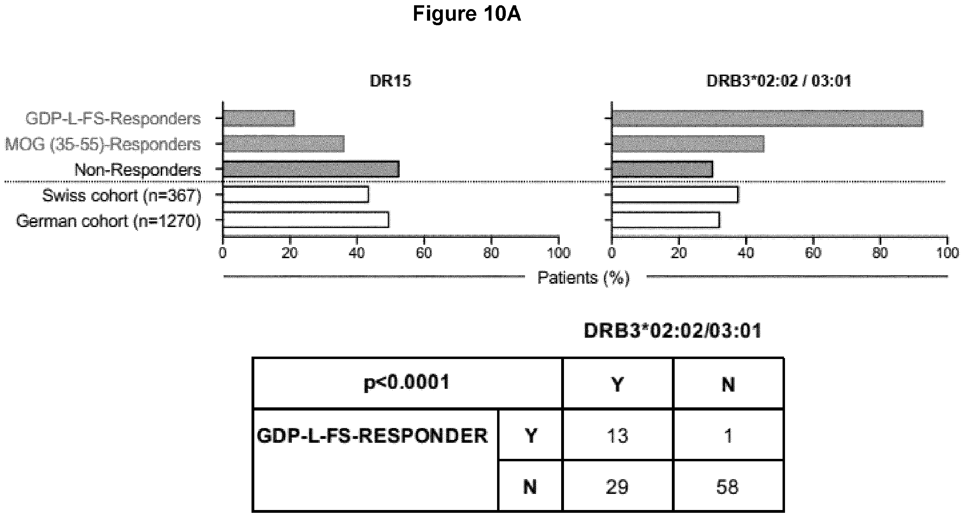

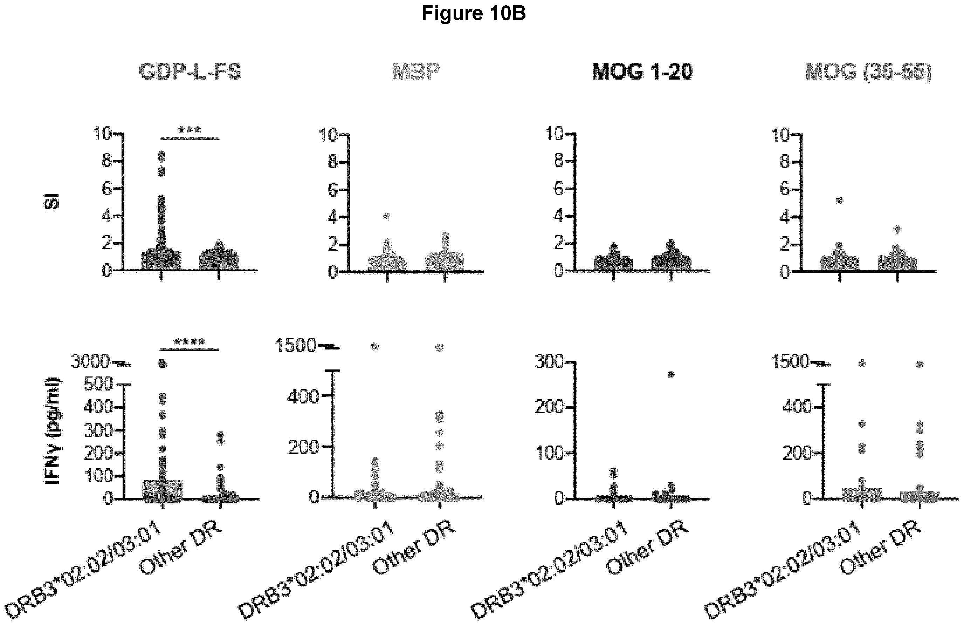

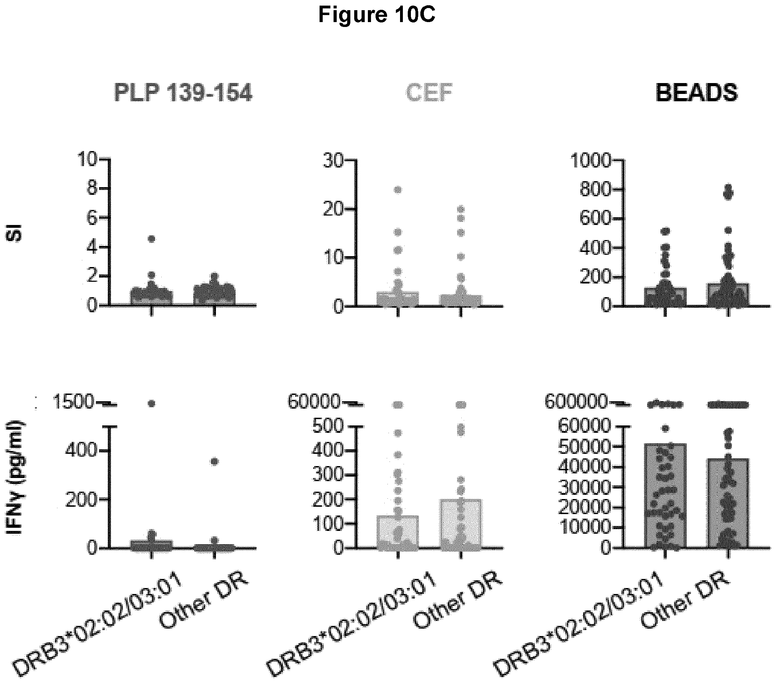

**Figure 10****. HLA class II expression in GDP-L-FS and MOG 35-55-responder patients. A.** Frequency of patients expressing the DR15 (left) and the DRB3*02:02/03:01 (right) genes in GDP-L-FS-, MOG(35-55)- and non- responders as well as in two reference cohorts. Number of patients responding or not to GDP-L-FS and expressing (Y, yes) or not (N, not) the DRB3*02:02/03:01 genes and P values for Fisher's exact tests are shown. **B+C.** CSF-infiltrating CD4⁺ T cell responses (proliferation (SI) and IFN-γ release) to GDP-L-FS, MBP, MOG(1-20), MOG(35-55), PLP and CEF peptides, and anti-CD2, anti-CD28, anti-CD3 stimulatory beads from patients expressing or not the DRB3*02:02/03:01 genes. Each dot in the graphs corresponds to a single well. Mann-Whitney test was used to compare patients expressing DRB3*02:02/03:01 and other DR. Statistical significance (*** p<0.001 and **** p<0.0001) is shown.

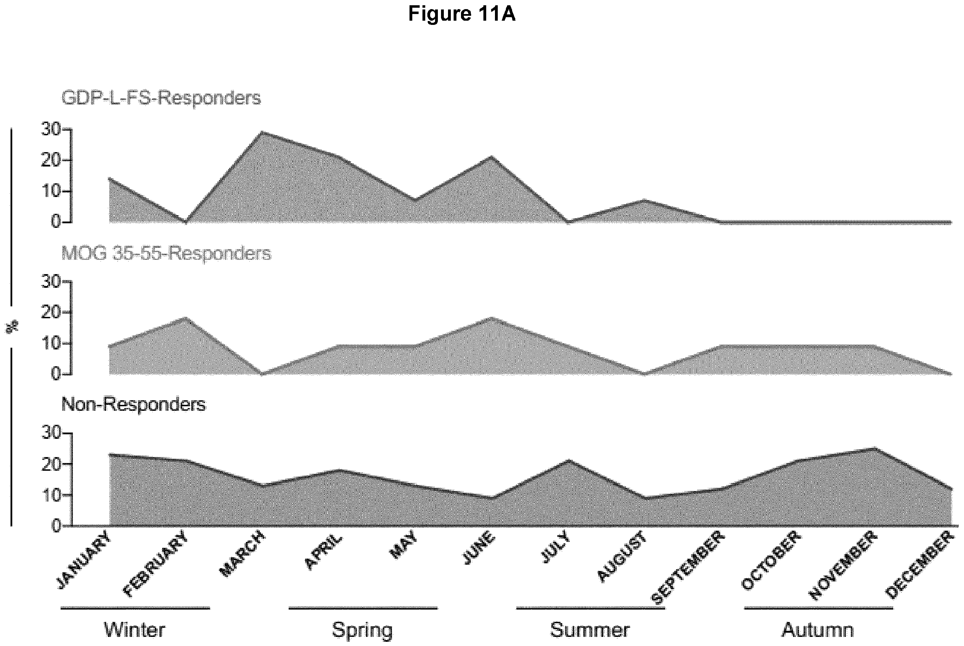

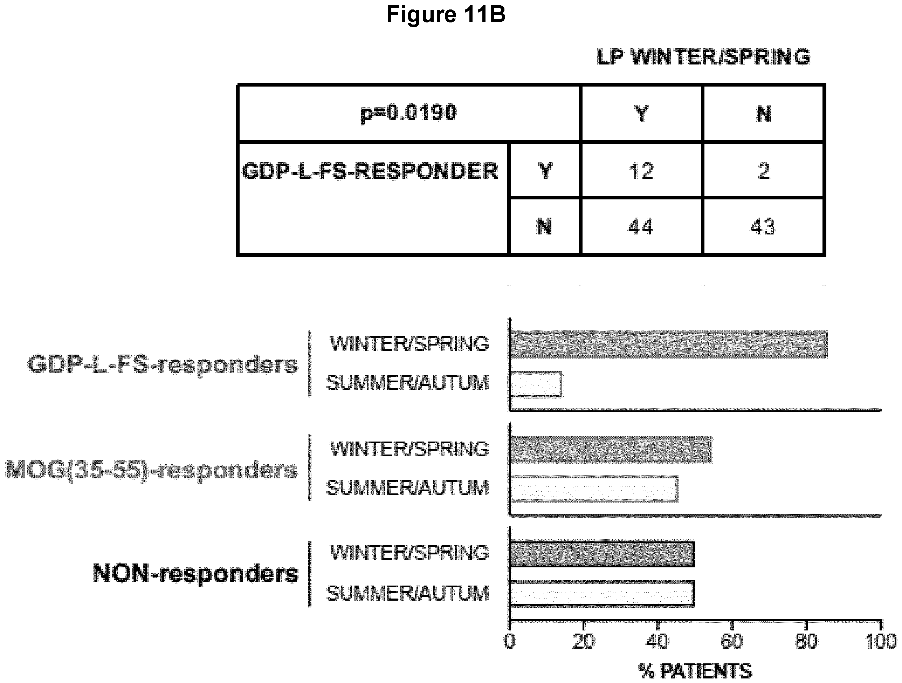

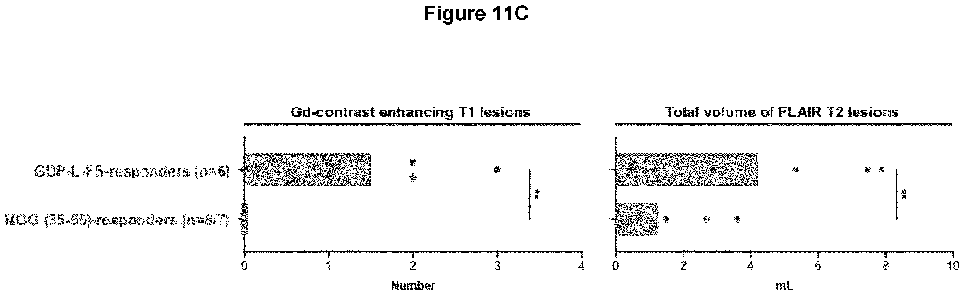

**Figure 11****. Characterization of GDP-L-FS and MOG 35-55-responder patients. A+B.** Monthly distribution of LPs and frequency of samples obtained in winter/spring and summer/autumn in GDP-L-FS-, MOG 35-55- and non-responders. Number of patients responding or not to GDP-L-FS in which LP was obtained in winter/spring (Y, yes) or not (N, not) and P values for Fisher's exact tests are shown. **C.** Number of total contrast enhancing T1 lesions and total volume of flair T2 lesions (expressed in mL) in GDP-L-FS- and MOG(35-55)-responders. Each dot in the graphs corresponds to a single patient and the bars show the mean. T-test was used for normally distributed variables to compare two group of patients. Statistical significance (** p<0.01) is shown.

### DETAILED DESCRIPTION OF THE INVENTION

A method of stratification of an MS patient has been found which allows to specifically tailor therapeutic approaches for an individual patient (personalized treatment). For example, those patients can be selected for a tolerization approach in which body fluid, in particular blood, preferably peripheral blood, or CSF (previously obtained from the MS patient), CD27- Th1 CD4+ cells, preferably CCR7- CD45RA- CD27- Th1 CD4+ cells, have been detected. Preferably, the patient's immune system, in particular T cells and/or antibodies in the body fluid, is reactive against, i. e. responds to the protein GDP-L-FS or a fragment, derivative and/or splice variant thereof.

Stratification preferably means to classify MS patients into different groups depending on the outcome of the method. For example, the patient subgroup that may be identified by the method of the present application is characterized by CD27- Th1 CD4+ cells in the body fluid, in particular peripheral blood or CSF. In one embodiment, the same patient subgroup may further characterized by a responsiveness of T cells and/or antibodies in the body fluid to the protein GDP-L-FS or a fragment, derivative and/or splice variant thereof. In another particular embodiment, this patient subgroup may further be characterized by one or more of the following characteristics:
- inflammation and/or neurodegeneration in the central nervous system, in particular characterized by a Gd-contrast enhancing T1 lesion and/or a FLAIR T2 lesion,
- higher expression of genes associated with Th1 cells or cytotoxicity and/or genes encoding proinflammatory cytokines, such as IL-2 and/or IFN-γ, compared with healthy controls,
- HLA allotype HLA-DRB3*02:02 or DRB3*03:01.

According to one embodiment of the present invention, the CD27- Th1 CD4+ cells detected in the body fluid, in particular peripheral blood or CSF, are further negative for the markers CCR7 and/or CD45RA. In a preferred embodiment, the CD27- Th1 CD4+ cells are negative for both markers CCR7 and CD45RA.

In a particularly preferred embodiment, the patient's T cells respond to at least one of the peptides as defined in SEQ ID Nos: 2 to 6 and SEQ ID NO: 37. In another preferred embodiment, the fragment comprises or consists of any sequence that lies within the sequence as defined by SEQ ID NO: 37. The peptides may also be termed immunodominant.

T cells that respond to the protein GDP-L-FS or a fragment, derivative and/or splice variant thereof, are preferably CD4+ T cells, more preferably CSF-infiltrating CD4+ T cells.

T cell response to a particular stimulus can for example be measured by the proliferation of the T cells and/or by their release of cytokines, in particular IFN-γ. The person skilled in the art is aware of methods measuring proliferation and release/secretion of cytokines, such as IFN-γ. For example, a proliferative response can be measured by a 3H-thymidine assay. Quantification of cytokines can for example be performed by conventional ELISA tests or by bead-based immunoassays that allow quantifying multiple cytokines simultaneously using a flow cytometer.

A patient is preferably classified as a responder when the patient shows a positive proliferative response and/or cytokines, such as IFN-γ, can be detected in response to the stimulus, i. e. preferably a peptide.

The cytosolic enzyme GDP-L-fucose synthase converts GDP-4-keto-6-deoxy-D-mannose into GDP-L-fucose, which is then used by fucosyltransferases to fucosylate all oligosaccharides. In mammals, fucosylated glycans play important roles in many biological processes including blood transfusion reactions, host-microbe interactions, cancer pathogenesis and maintenance of a non-inflammatory environment in the brain. In one embodiment, the GDP-L-fucose synthase shows enzyme activity converting GDP-4-keto-6-deoxy-D-mannose into GDP-L-fucose.

A protein is intended to mean oligopeptides, polypeptides as well as proteins as such. A protein sequence may be defined by a GenBank entry. A protein sequence may also be defined by a UniProtKB/Swiss-Prot entry and/or by a GenPept entry. An entry may be defined by a number, e. g. an accession number. Where applicable, the database entries include the respective accession number (i. e. an entry number) and version number. A protein may also be defined by any other database known to the skilled person. Different isoforms, derivatives and/or splice variants may exist which are also encompassed by the present invention. Thereby, the sequence may vary from the known sequence from, for example, the GenBank or UniProtKB/Swiss-Prot entry.

"A" protein or "the" protein according to the present invention for example refers to a GDP-L-fucose synthase protein, unless otherwise explicitly mentioned.

In a preferred embodiment, the proteins are human proteins and/or the nucleotide sequences and/or gene sequences are human sequences.

A splice variant arises from alternative splicing during gene expression. The splice variant according to the invention is preferably immunodominant.

A fragment is preferably any part of the protein, which is shorter, i.e. has less amino acids, than the parent protein. A fragment may be a peptide. In one embodiment, the fragment comprises 5 to 50, preferably 5 to 20, more preferably 10 to 15 amino acids, even more preferably 15 amino acids. The fragment according to the invention is preferably immunodominant.

A derivative of a sequence is preferably defined as an amino acid sequence which shares a homology or identity over its entire length with a corresponding part of the reference amino acid sequence of at least 75%, more preferably at least 80%, at least 85%, at least 90%, at least 93%, at least 95%, at least 97%, at least 98% or at least 99%. The "corresponding part" in the sense of the present invention preferably refers to the same stretch of amino acids of the same parent sequence. For example, if a derivative with a length of 100 amino acids differs from a stretch of amino acids of SEQ ID NO: 1 (amino acids 1 to 100 of SEQ ID NO: 1) by 20 amino acids, this particular derivative shares an identity of 80 % over its entire length with the corresponding part, i. e. amino acids 1 to 100, of the reference amino acid sequence, i. e. SEQ ID NO: 1. The derivative according to the invention is preferably immunodominant.

A "homology" or "identity" of an amino acid sequence is preferably determined according to the invention over the entire length of the reference amino acid sequence or over the entire length of the corresponding part of the reference amino acid sequence which corresponds to the sequence which homology or identity is defined.

An "identity" is defined as identical amino acids, a "homology" comprises identical amino acids as well as conservative substitutions. The person skilled in the art is aware of conservative substitutions, such as
- aromatic and aromatic F and W/Y
- positively charged and positively charged R and K/H
- negatively charged E and D or
- aliphatic V and L/M/I, or A and S/T.

The nucleotide sequence encoding any of the proteins of the present invention or fragment, derivative or splice variant thereof refers to any coding nucleotide sequence, for example RNA or DNA, in particular mRNA or cDNA. In one embodiment, the nucleotide sequence is a plasmid or any type of vector known to the person skilled in the art. In a preferred embodiment, the nucleotide sequences do not comprise introns and the gene sequences comprise exons and introns.

In a preferred embodiment, the GDP-L-fucose synthase protein
a) has the amino acid sequence as set forth in SEQ ID NO: 1 or
b) has an amino acid sequence which is at least 85 %, preferably at least 90 %, more preferably at least 95 % identical to the amino acid sequence as set forth in SEQ ID NO: 1 or
c) has an amino acid sequence which is at least 70 %, preferably at least 80 %, more preferably at least 90 % homologous to the amino acid sequence as set forth in SEQ ID NO: 1 or
d) has an amino acid sequence which is at least 60 %, preferably at least 70 %, more preferably at least 80 %, even more preferably at least 90 % homologous to the amino acid sequence as set forth in SEQ ID NO: 1 and the protein or fragment or splice variant thereof binds to an autologous HLA allele, is recognized by a T cell and/or is recognized by an antibody which binds to or recognizes the amino acid sequence as set forth in SEQ ID NO: 1 or a fragment thereof or
e) is encoded by a TSTA3 gene, in particular by a gene sequence of nucleotides 143612618 to 143618048 of NC_000008.11, or is encoded by a gene which is at least 80 %, preferably at least 90 %, even more preferably at least 95 % identical to the gene sequence of nucleotides 143612618 to 143618048 of NC_000008.11.

The binding to an autologous HLA allele, recognition by a T cell and/o recognition by an antibody may indicate immunodominance of the protein or fragment or splice variant thereof. Immunodominance can also be tested as disclosed below.

In one embodiment, the fragment comprises 5 to 50, preferably 5 to 20, more preferably 10 to 15, even more preferably 15 amino acids.

In another embodiment, the fragment is
a) at least 85 %, preferably at least 90 %, more preferably at least 95 % identical to a respective corresponding amino acid sequence or
b) at least 70 %, preferably at least 80 %, more preferably at least 90 % homologous to a respective corresponding amino acid sequence or
c) at least 60 %, preferably at least 70 %, more preferably at least 80 %, even more preferably at least 90 % homologous to a respective corresponding amino acid sequence and binds to an autologous HLA allele, is recognized by a T cell and/or is recognized by an antibody which binds to or recognizes the respective amino acid sequence.

The "respective corresponding amino acid sequence" refers to the respective fragment of the corresponding amino acid sequence (i. e. SEQ ID NO: 1) with the same length as the homologous fragment (see also definition of "corresponding part" *supra*). A fragment with these identities and/or homologies may comprise 5 to 50, preferably 5 to 20, more preferably 10 to 15, even more preferably 15 amino acids. The identity and/or homology is determined over the entire length of the respective fragment. In other words, the "corresponding amino acid sequence" refers to the unaltered sequence, i. e. when a fragment of a sequence as set forth in SEQ ID NO: 1 is 85 % identical to a respective corresponding amino acid, the fragment is 85 % identical (over the entire length of the fragment) to the unaltered fragment as "excised", i. e. directly taken or copied from SEQ ID NO: 1.

Therefore, in one embodiment, the protein GDP-L-fucose synthase has an amino acid sequence with a certain homology (at least 60 %, preferably at least 70 %, more preferably at least 80 %, even more preferably at least 90 %) to the respective depicted sequence as set forth in the SEQ ID NOs with the additional requirement that the protein or fragment or splice variant thereof binds to an autologous HLA allele, is recognized by a T cell and/or is recognized by an antibody which binds to or recognizes the amino acid sequence as set forth in the respective SEQ ID NO or a fragment thereof. In another embodiment, the protein or fragment or splice variant thereof binds to an autologous HLA allele and is recognized by a T cell which binds to or recognizes the amino acid sequence as set forth in the respective SEQ ID NO or a fragment thereof.

Assays for measuring and/or predicting binding to an autologous HLA allele, recognition by a T cell or recognition by an antibody are well known to the person skilled in the art. Binding of a peptide to an HLA allele can for example be predicted by the using the well-accepted NetMHCII (http://www.cbs.dtu.dk/services/NetMHCII/) or IEDB (http://www.iedb.org/) in silico peptide binding prediction algorithms. T cell recognition can for example be measured by a T cell proliferation assay, for example by measuring incorporated radioactivity. Binding of a peptide and/or a protein to an antibody can be measured by standard assays known to the person skilled in the art, for example by ELISA. The binding to an autologous HLA allele, recognition by a T cell or recognition by an antibody may indicate immunodominance of a peptide or protein. Immunodominance can also be tested as disclosed below.

In a preferred embodiment the peptides used for the treatment according to the invention comprise fragments of GDP-L-fucose synthase and comprise at least one sequence selected from the group consisting of SEQ ID NOs: 2 to 6 and SEQ ID NO: 37. In especially preferred embodiments the peptides consist of amino acid sequences as depicted in one of the SEQ ID NOs: 2 to 6 and SEQ ID NO: 37. In another preferred embodiment, the fragment comprises or consists of any sequence that lies within the sequence as defined by SEQ ID NO: 37.

The sequences according to SEQ ID NOs: 2 to 6 have previously been identified as immunodominant peptides due to being recognized by disease-relevant T cells, and subsequent validation of recognition by CSF-infiltrating bulk T cells (WO 2020/002674).

The amino acid sequences are recited in Table 2 *infra.* The gene sequence of TSTA3 (encoding GDP-L-FS) can be identified by the NCBI Reference Sequence: NC_000008.11 (REGION: 143612618..143618048).

The following nucleotide sequences represent preferred nucleotide sequences encoding GDP-L-FS (gene name: TSTA3) or fragment, derivative or splice variant thereof of the present invention. Also comprised are coding sequences (CDS), i. e. proteins or peptides, which can also be used in the treatment of MS:

| | |
|---|---|
| RNA: | XM_011517269.1, NM_003313.3, NM_001317783.1, XM_005251051.3 |
| Protein: | XP_011515571.1, NP_003304.1, NP_001304712.1, XP_005251108.2 |

All sequences were retrieved from the respective online databases on June 22, 2018.

The following characteristics indicate that a certain peptide of a protein is immundominant in the context of MS:
a) frequent recognition of this peptide by T cells, i. e. by approximately 10% or more of MS patients, often in the context of a disease-associated HLA allele or haplotype (Sospedra and Martin, 2005), and
b) recognition of this peptide by disease-relevant T cells such as those that respond to peptides at low concentrations (high avidity T cells) (Bielekova et al., 2004) and are therefore considered particularly dangerous, and/or have a proinflammatory phenotype, and/or are isolated from the target organ or compartment (CNS), in the case of MS, brain-, spinal cord- or CSF-infiltrating T cells.

However, high avidity recognition is not a prerequisite, since low-avidity myelin-specific T cells have also been shown to be pathogenic in humanized transgenic mouse models (Quandt et al. 2012).

Thus, it can be tested whether a protein or fragment, derivative or splice variant thereof is immunodominant in the context of MS. Such a test is preferably an *in vitro* test. Particularly suitable is an *in vitro* test that allows measuring the reactivity of T cells and/or antibodies obtained from the blood, CSF or other body fluid of a human subject that had been diagnosed with MS, preferably CSF-infiltrating CD4⁺ T cells, to the tested protein or fragment, derivative or splice variant. The person skilled in the art is aware of methods testing the reactivity of T cells, preferably CD4⁺ T cells, and/or antibodies. For example, the proliferation of CD4⁺ T cells and/or their secretion of IFN-γ or reactivity in a ELISPOT/FLUOROSPOT assay or reactivity against HLA-peptide tetramers can be tested. If the tested protein or fragment, derivative or splice variant thereof induces reactivity in a human subject that had been diagnosed with MS, in case of T cell reactivity in particular a stimulatory index (SI) above 2 and/or an IFN-γ secretion above 20 pg/ml, the tested protein or fragment, derivative or splice variant may be termed immunodominant. It is also possible to select 10 patients who had been diagnosed with MS for such a test. If reactivity is induced in at least 2 patients, the tested protein or fragment, derivative or splice variant may be termed immunodominant. Preferably, the 10 patients have been diagnosed with RRMS according to the established revised McDonald criteria.

It has recently been demonstrated that T cells of MS patients show increased *in vitro* proliferation in the absence of an exogenous antigen (Mohme et al., 2013; Jelcic et al., 2018). These "autoproliferating" T cells are enriched for cells that home to the CNS compartment of MS patients and can thus be considered as a peripheral blood source of brain-/CSF-infiltrating T cells.

In the case that data from testing T cells *in vitro* is not available or in addition to such testings, immune recognition of peptides can also be predicted/inferred from those peptides that will bind well to the HLA-class I or -class II alleles of the individual and for CD8+ and CD4+ T cells respectively. Peptide binding predictions are well known to the skilled person. They can be performed by well-established prediction algorithms (NetMHCII - www.cbs.dtu.dk/services/NetMHCII/; IEDB - www.iedb.org/) and analysis of the HLA-binding motifs (SYFPEITHI - www.syfpeithi.de/).

The protein GDP-L-fucose synthase has previously been identified as immunodominant in MS, thus it has been identified as an autoantigen (WO 2020/002674).

It is not necessary that the binding to the HLA allele is particularly strong. In fact, immunodominance can also occur for peptides that bind poorly to the HLA allele (Muraro et al., 1997, J Clin Invest, 100(2):339-349).

In one aspect of the invention, a GDP-L-FS protein or a fragment, derivative or splice variant thereof, or a nucleotide sequence encoding the GDP-L-FS protein or fragment, derivative or splice variant thereof is used in the treatment of MS in an MS patient, wherein CD27- Th1 CD4+ cells are detected in body fluid, in particular blood, preferably peripheral blood, or CSF, previously obtained from the MS patient.

Treatment for example includes tolerance induction.

In a preferred embodiment, T cells and/or antibodies previously obtained from the body fluid of the MS patient respond to the protein GDP-L-FS or a fragment, derivative and/or splice variant thereof.

Hence, the MS patient selected for treatment is stratified in such a way, that only those patients are treated who show in their body fluid, in particular blood, preferably peripheral blood, or CSF, previously obtained from the MS patient, CD27- Th1 CD4+ cells. In particular, the MS patient is also responsive to a GDP-L-FS peptide.

In another aspect of the invention, at least one GDP-L-FS protein, fragment, derivative, splice variant, nucleotide sequence and/or gene sequence and/or at least one carrier coupled to at least one GDP-L-FS protein, fragment, derivative, splice variant, nucleotide sequence and/or gene sequence is used in a method for inducing antigen-specific tolerance to autoantigens in an MS patient, wherein CD27- Th1 CD4+ cells are detected in body fluid, in particular blood, preferably peripheral blood, or CSF, previously obtained from the MS patient.

In a preferred embodiment, T cells and/or antibodies previously obtained from the body fluid of the MS patient respond to the protein GDP-L-FS or a fragment, derivative and/or splice variant thereof.

Hence, the patients that are being tolerized are selected according to the above criteria.

Tolerance induction is antigen-specific and renders autoreactive T cells non-functional or anergic or induces Treg cells that specifically suppress untoward autoimmunity to said target antigens. The induction of tolerance to target autoantigens is a highly important therapeutic goal in autoimmune diseases. It offers the opportunity to attenuate specifically the pathogenic autoimmune response in an effective way with few side effects. Tolerance induction can also be achieved by applying a whole protein instead of or in addition to an immunodominant peptide being a fragment of the protein (Kennedy et al., 1990).

The immunodominance of the proteins and/or fragments thus allows using the protein and/or a fragment, derivative or splice variant thereof for antigen-specific immunotherapies such as tolerance induction.

According to the present invention, antigen-specific tolerization can be used in all forms of MS: At the time of first manifestation, when differential diagnoses have been excluded, the disease is referred to as CIS provided that the CSF and MRI findings are consistent with the diagnosis. MRI discloses lesions in locations typical for MS, i.e. juxtacortical, periventricular, in the brain stem or spinal cord. If certain criteria are fulfilled that can be summarized as dissemination in space (more than one lesion or clinical symptom/sign) and time (more than one event) then the diagnosis of RRMS can be made. A special scenario is the accidental discovery of MRI lesions compatible with MS without clinical symptoms. This is referred to as RIS and can be considered a pre-stage of CIS and RRMS. More than 80% of patients suffer from one of these, and the majority of patients develops later what is called SPMS. At this time, relapses/exacerbations become less frequent or stop altogether and neurological disability increases steadily either between relapses or without these.

A special form of MS is PPMS, which never shows relapses, but rather begins with steady worsening of neurological symptoms, e.g. of the ability to walk. PPMS affects approximately 10% of MS patients and males and females with equal frequency. Its onset is usually later than CIS or RRMS. With respect to causes and disease mechanisms PPMS is considered similar to the above RIS-CIS-RRMS-SPMS.

Typically, MS is diagnosed according to the revised McDonald criteria. These criteria also allow distinguishing between the different forms and disease activity of MS (Thompson et al., 2018, Lancet Neurol, 17(2):162-173). An MS patient according to the invention is a human being that has been diagnosed with MS.

Preferably, the tolerization approach is applied at an early stage, i.e. RIS, CIS and early RRMS, since it is assumed that the immune processes at this stage are primarily mediated by autoreactive T lymphocytes, while tissue damage, so-called degenerative changes, become gradually more important when the disease advances. However, tolerization is meaningful as long as there is an autoreactive T cell response against the antigens used for tolerization, which could also be during SPMS and PPMS.

In a particularly preferred embodiment, a GDP-L-fucose synthase protein or splice variant thereof, preferably the GDP-L-fucose synthase protein, is used in a tolerization approach at an early stage, i.e. RIS, CIS and early RRMS. The GDP-L-fucose synthase protein has, for example, the sequence as set forth in SEQ ID NO: 1.

The method for inducing tolerance preferably comprises the step of applying to an MS patient in need thereof, i. e. to the human subject, at least one GDP-L-FS protein or fragment (peptide), derivative and/or splice variant thereof, a nucleotide sequence encoding any of the proteins or fragment, derivative or splice variant thereof and/or a gene sequence as described herein or applying at least one carrier comprising at least one protein, fragment, derivative, splice variant, nucleotide sequence and/or gene sequence as described herein.

It is particularly preferred to use the whole protein of GDP-L-fucose synthase (SEQ ID NO: 1) for inducing antigen-specific tolerance. In another preferred embodiment, a fragment (peptide) of the protein is used. It is particularly preferred to use a fragment as set forth in any of SEQ ID NOs: 2 to 6 and SEQ ID NO: 37. In another preferred embodiment, the fragment comprises or consists of any sequence that lies within the sequence as defined by SEQ ID NO: 37.

In a preferred embodiment, a GDP-L-FS protein or peptide or the corresponding nucleotide sequence or gene sequence, preferably at least one of the peptides (or the corresponding nucleotide sequence or gene sequence) GDP-L-FS 51-65 (SEQ ID NO: 2), GDP-L-FS 136-150 (SEQ ID NO: 3), GDP-L-FS 161-175 (SEQ ID NO: 4), GDP-L-FS 246-260 (SEQ ID NO: 5), GDP-L-FS 296-310 (SEQ ID NO: 6) and GDP-L-FS 226-270 (SEQ ID NO: 37), and in addition one or more of the following peptides (or the corresponding nucleotide sequence or gene sequence) may be used for tolerance induction:
MBP 13-32 (SEQ ID NO: 7)
MBP 83-99 (SEQ ID NO: 8)
MBP 111-129 (SEQ ID NO: 9)
MBP 146-170 (SEQ ID NO: 10)
MOG 1-20 (SEQ ID NO: 11)
MOG 35-55 (SEQ ID NO: 12)
PLP 139-154 (SEQ ID NO: 13)

The preferred embodiment that may be used for tolerance induction may also include (alternatively to one or more of the peptides above or in addition) at least one peptide (or the corresponding nucleotide sequence or gene sequence) which sequence lies within the stretch of GDP-L-FS 226-270 (SEQ ID NO: 37). Preferably, this stretch that lies within the stretch of GDP-L-FS 226-270 (SEQ ID NO: 37) encompasses 10 to 20 amino acids, preferably 15 amino acids.

In one embodiment, the nucleotide sequence or gene sequence is applied to the patient via a carrier, e.g. a cell. The antigen may then be expressed by a carrier, e.g. a cell. Transfer of autoantigen-encoding RNA/DNA into a carrier, e.g. a cell, and thus encoding the above autoantigens is also conceivable similar to tumor vaccination approaches that employ antigen-encoding RNAs.

The at least one protein, fragment, derivative, splice variant, nucleotide sequence and/or gene sequence may be applied by nasal, inhaled, oral, subcutaneous (s.c.), intracoelomic (i.c), intramuscular (i.m.), intradermal (i.d.), transdermal (t.d.) or intravenous (i.v.) administration, preferably by routes of administration that are considered tolerogenic, for example by i.v., s.c., i.d., t.d., oral, inhaled, nasal or coupled to a tolerogenic carrier, preferably a red blood cell (RBC). The carrier is preferably applied systemically, in particular intravenously.

In particular, the method may be used for inducing antigen-specific tolerance to autoantigens in early MS or even pre-clinical stages of the disease.

The antigen-specific tolerance protocol provided herein may selectively target both activated and naïve autoreactive T cells specific for multiple potential encephalitogenic epitopes that perpetuate the disease.

The tolerization approach can also be used to prevent MS. This approach may include identifying those individuals (e.g. in a family with an MS patient selected as disclosed herein), who are at a high risk of developing MS. For example, it is possible to tolerize e.g. the children of a mother with MS or the identical twin of a patient with MS, in whom the risk of developing MS would be particularly high.

Diagnosis of MS or one of its forms is made by demonstrating neurological deficits and/or MRI lesions compatible with MS that are disseminated in space and time. The present invention can be used to identify patients particularly likely to profit from antigen-specific tolerance induction, i.e. allow personalizing antigen-specific tolerance approaches. By identifying patients who particularly likely profit from antigen-specific tolerance induction, a patient can be *in vitro* diagnosed with MS, in particular with a subtype of MS. Thus, this *in vitro* testing can be complemented with clinical and imaging findings, i. e. the MS diagnosis according to the state of the art, in particular according to the revised McDonald criteria.

The patient subgroup that may be identified by the present invention potentially has an aggressive form of MS. The aggressive form may be characterized by neurodegenerative and/or neuroinflammatory processes, especially at an early stage of the disease. Hence, identification of the patient subgroup may be possible already at an early stage of the disease. Besides detecting CD27- Th1 CD4+ cells, preferably CCR7- CD45RA- CD27- Th1 CD4+ cells, in the body fluid of the patient and reactivity against the protein GDP-L-FS or a fragment, derivative and/or splice variant thereof, particular characteristics may be:
- inflammation and/or neurodegeneration in the central nervous system, in particular characterized by a Gd-contrast enhancing T1 lesion and/or a FLAIR T2 lesion,
- higher expression of genes associated with Th1 cells or cytotoxicity and/or genes encoding proinflammatory cytokines, such as IL-2 and/or IFN-γ, compared with healthy controls, and/or
- HLA allotype HLA-DRB3*02:02 or DRB3*03:01.

The person skilled in the art knows how to detect inflammation and/or neurodegeneration in a patient's CNS. For example, magnetic resonance imaging (MRI) being a standard imaging technique for the identification of demyelinating lesions can be used in this regard. Most common MRI sequences are T1-weighted and T2-weighted scans which can further be refined by Gd-contrast enhancing or fluid attenuated inversion recovery (FLAIR), respectively.

Higher expression of genes associated with Th1 cells or cytotoxicity and/or genes encoding proinflammatory cytokines, such as IL-2 and/or IFN-γ, compared with healthy controls may for example be measured by standard techniques in the body fluid, in particular blood, preferably peripheral blood, or cerebrospinal fluid (CSF).

Detecting the HLA allotype is particularly important for MS stratification.

The patient subgroup that may be identified by the present invention may also be characterized by elevated levels of biomarkers, preferably neurofilament (NF-L) and/or chitinase (YKL-40), in the CSF of an MS patient (compared with healthy controls), the MS patient preferably being responsive to the protein GDP-L-FS or a fragment, derivative and/or splice variant thereof.

Hence, the biomarker neurofilament (NF-L) and/or chitinase (YKL-40) may be used for the identification of an MS patient subgroup. Therefore, in one embodiment, use of a biomarker, in particular neurofilament (NF-L) and/or chitinase (YKL-40), is disclosed for the identification of an MS patient subgroup, wherein a level of neurofilament (NF-L) and/or chitinase (YKL-40) is detected in the body fluid, preferably CSF, of the MS patient and compared with the level of neurofilament (NF-L) and/or chitinase (YKL-40) in the body fluid, preferably CSF, of a healthy control, wherein the body fluid, preferably CSF, has been previously obtained from the MS patient and the healthy control.

The patient subgroup that may be identified by the present invention may also be characterized by elevated levels (enrichment in frequency and/or absolute numbers) of CD4+ memory (central memory (CM) and effector memory (EM)) Th1 cells in CSF and/or paired blood, in particular peripheral blood, in those patients that respond to the protein GDP-L-FS or a fragment, derivative and/or splice variant thereof with respect to MOG (35-55) responders and/or non-responders. In particular, the CD4+ EM Th1 (CD28+CD27-CCR6-CCR4-CRTh2-) population may be enriched. Hence, CD4+ memory Th1 cells, in particular CD4+ EM Th1 (CD28+CD27-CCR6-CCR4-CRTh2-) cells, may be used as a biomarker for the identification of an MS patient subgroup, wherein a level of CD4+ memory Th1 cells, in particular CD4+ EM Th1 (CD28+CD27-CCR6-CCR4-CRTh2-) cells, is detected in CSF and/or paired blood, in particular peripheral blood, of a GDP-L-FS responder (responsive to the protein GDP-L-FS or a fragment, derivative and/or splice variant thereof) and compared with a MOG (35-55) responder and/or a non-responder, wherein the CSF and the blood has been previously obtained from the MS patient. The identification of this patient subgroup may in particular be possible at an early stage of the disease.

By identifying those patients or patient subgroups with an existing and/or particularly strong proinflammatory (potentially harmful) T cell or antibody response against the respective autoantigen, the patient can thus be *in vitro* diagnosed with MS, in particular with an MS subtype which is especially valuable at an early stage of the disease. In this situation, pretesting the patient with a suitable test to assess whether the patient belongs to the specific subgroup would also allow tailoring the tolerizing treatment (e.g. composition of the peptides/proteins used for tolerization) to the individual patient or patient subgroup with the aim to render the tolerization as specific as possible and also to avoid potential adverse effects. Antigen-specific tolerization, however, can also be performed in patients in whom a T cell response to the tolerizing antigen has not been shown.

In one aspect of the invention, CD27- Th1 CD4+ cells are provided for use in a method of monitoring the response to the method for inducing antigen-specific tolerance as described *supra,* wherein the CD27- Th1 CD4+ cells are detected in body fluid, in particular blood, preferably peripheral blood, or CSF, previously obtained from an MS patient.

In a preferred embodiment, additionally a responsiveness of T cells and/or antibodies previously obtained from the body fluid of the MS patient to the protein GDP-L-FS or a fragment, derivative and/or splice variant thereof as defined *supra* is detected.

Monitoring the response to the method for inducing antigen-specific tolerance preferably means that the success of the tolerance induction can be controlled. In case tolerance induction is successful, the number of CD27- Th1 CD4+ cells in the body fluid (in particular blood, preferably peripheral blood, or CSF), in particular those also being negative for the markers CCR7 and/or CD45RA, decreases in the course of tolerance induction. Hence, in order to monitor the response to the method for inducing antigen-specific tolerance, preferably the number of CD27-Th1 CD4+ cells in the body fluid, in particular those also being negative for the markers CCR7 and/or CD45RA, is measured. The measurement can for example be done by flow cytometry or by using oligonucleotide-labeled antibodies and subsequent sequencing- or PCR-based methods, or by any other method that allows quantifying the cells, for example using antibodies and a suitable detection method.

In one embodiment, the response is monitored 4 to 12 weeks after tolerization, preferably 6 to 10 weeks after tolerization, preferably 8 weeks after tolerization. Tolerization preferably means the date of tolerance induction in the patient, i. e. the date of applying to an MS patient in need thereof, i. e. to the human subject, at least one GDP-L-FS protein or fragment (peptide), derivative and/or splice variant thereof, a nucleotide sequence encoding any of the proteins or fragment, derivative or splice variant thereof and/or a gene sequence as described herein or applying at least one carrier comprising at least one protein, fragment, derivative, splice variant, nucleotide sequence and/or gene sequence as described herein. In case application is done more than once, tolerization preferably means the first day of application.

In one embodiment, the response to tolerance induction can be monitored over a longer time period (e. g. retest every 6 months) in order to test for the maintenance of the tolerizing effect.

In one aspect of the invention, a carrier is provided coupled to at least one GDP-L-FS protein, fragment, derivative, splice variant, nucleotide sequence and/or gene sequence as defined *supra* for use in a method for inducing antigen-specific tolerance to autoantigens in an MS patient, wherein CD27- Th1 CD4+ cells are detected in body fluid, in particular blood, preferably peripheral blood, or CSF, previously obtained from the MS patient.

The person skilled in the art is familiar with possible carriers. For example, the carrier may be any cell, protein, lipid, glycolipid, bead, nanoparticle, virus-like-particle (VLP), or molecule, such as a sugar molecule, or any combination thereof that is suitable for application in humans and to which protein/s and/or fragment/s can be coupled by a coupling process, e. g. by a chemical coupling process, preferably by EDC. The carrier can be derived from one existing in nature or be synthetic. Preferably, the cell, molecule, bead, nanoparticle, or VLP is biodegradable *in vivo* or is at least applicable to living persons and broken down *in vivo* or is eliminated from the body to which the carrier is applied. The term cell also includes cell precursors, e.g. RBC precursors. Preferably, the carrier is a blood cell, even more preferably a red or white blood cell. The white blood cell may be a splenocyte or a PBMC or generally an APC.

In one embodiment, the protein, fragment, derivative and/or splice variant is expressed by the cell, preferably the blood cell. Thereby, the genetic information encoding the protein, fragment, derivative and/or splice variant is introduced into the cell before the protein, fragment, derivative and/or splice variant is expressed by the cell.

Any coupling agent or method for coupling a protein and/or a fragment thereof to a carrier may be used. For example, a synthetic or natural linker may be employed for coupling. One example of such a linker is glycophorin A, present on the surface of red blood cells (RBC). In one embodiment, chemical crosslinking is performed. In a preferred embodiment, the chemical crosslinker EDC catalyzing the formation of peptide bonds between free amino and carboxyl groups is used. Particularly in the presence of EDC, multiple peptides can be coupled to the surface of the carrier thereby allowing for the simultaneous targeting of multiple T cell specificities. Preferably, more than three, more than five, more than 10, more than 15, or even more than 20 different peptides are coupled to the surface of the carrier. In a preferred embodiment, between five and 20, preferably between five and 15 different peptides are used. A peptide is different from another peptide if it does not consist of the same amino acid sequence. The carrier is preferably, but not necessarily a cell. EDC can be used for coupling to any carrier as long as a free amino group is present.

In another embodiment, at least one peptide of the GDP-L-FS protein according to the present invention, in particular at least one peptide as defined by SEQ ID Nos: 2 to 6 and SEQ ID NO: 37 (or any peptide that lies within the sequence as defined by SEQ ID NO: 37), is used with a peptide of the state of the art, in particular with at least one myelin peptide, in particular with at least one or all of the myelin peptides as defined by SEQ ID NOs: 7 to 13.

In one preferred embodiment, the carrier is a blood cell, and the blood cell is chemically coupled by a coupling agent, preferably by EDC, to the at least one protein, fragment, derivative and/or splice variant. A method of manufacturing such a chemically coupled, i. e. antigen-coupled blood cell for example comprises isolating the blood cell from a human subject, adding the at least one protein, fragment, derivative and/or splice variant, i. e. the antigen, and subsequently adding the coupling agent, preferably EDC.

The mechanism of action of cells coupled with peptide by EDC is not fully understood, but involves covalently linking amino- and carboxy groups of peptide and cell surface molecules, subsequent programmed cell death (apoptosis in the case of nucleated cells; eryptosis in the case of RBC) of the peptide-coupled, i. e. antigen-coupled, cells and then tolerogenic presentation of the dying cells in vivo.

The dose of EDC used for the coupling reaction may be titrated to obtain a maximum of safety with best efficacy. In high concentrations, EDC may lead to lysis of cells, in particular of RBC. For optimal stability of the RBC, a final concentration of EDC of less than 15 mg/ml, preferably less than 10 mg/ml, even more preferably less than 5 mg/ml, even more preferably of about 3 mg/ml may be used. The optimal dose may also vary. The person skilled in the art knows how to determine the optimal stability of the RBC and the optimal dose of EDC.

The protein, fragment, derivative and/or splice variant to be coupled may be added in an amount to be readily determined by the person skilled in the art. The person skilled is aware of measures to determine the optimal amount in the interplay with an optimal amount of EDC.

The incubation time can be varied and tailored for each specific coupling reaction (for example, 15 min, 30 min, 45 min, 60 min, 120 min). Coupling efficiency may be better with longer time of incubation. In one embodiment, a maximum is reached after 60 min.

The incubation temperature may also vary. For example, 15-25°C or 2-8°C may be used. In one embodiment, coupling efficiency is higher when the coupling reaction is performed at 15-25°C.

Any excipient that allows the coupling reaction may be used. In one embodiment, the excipient is sterile and endotoxin free. In a preferred embodiment, the excipient is sterile, endotoxin free saline (NaCl 0.9%). Saline is approved for use in humans and provides a maximum of safety.

The person skilled in the art knows how to determine an optimal incubation time and temperature and how to determine possible excipients.

### EXAMPLES

### Materials and Methods

### Patient samples

Paired CSF and blood samples were collected from 105 untreated MS patients, only CSF from 11 control patients (CP) and 10 MOGAD patients negative for anti-AQP4 antibodies, and only peripheral blood mononuclear cells (PBMCs) from four healthy donors (HD) (Table 1). 84 MS patients (80%) had never been treated while 21 (20%) were previously treated but considered untreated at the time of lumbar puncture (Table 1). Patients and controls were recruited from the NIMS-Neuroimmunology and MS Research Section, Department of Neurology, University Hospital Zurich. MS diagnosis was based on the revised McDonald criteria (Polman et al. 2011, Ann Neurol, 69(2):292-302).

**Table 1. Demographic and clinical features of patients and controls.**

| | **MS PATIENTS** | | | | | | | |
|---|---|---|---|---|---|---|---|---|
| | **MAIN COHORT** | | | | **SUB-COHORT** | | | |
| | **All** | **RIS/CIS** | **RRMS** | **PMS*** | **All** | **RIS/CIS** | **RRMS** | **PMS*** |
| **# of Patients** | 105 | 14 | 82 | 9 | 66 | 12 | 50 | 4 |
| **Female/Male Ratio** | 1.9 | 1.8 | 1.9 | 2.0 | 2.1 | 1.4 | 2.3 | 3.0 |
| **Age (y)**** | 35.7 ± 9.8 | 30.6 ± 10.3 | 35.3 ± 9.0 | 46.9 ± 8.0 | 35.6 ± 10.0 | 30.0 ± 8.4 | 35.9 ± 9.6 | 49.5 ± 4.0 |
| | | | | | | | | |

| **MS PATIENTS PREVIOUSLY TREATED** | | | | | | | | |
|---|---|---|---|---|---|---|---|---|
| **Previous treatment** | **Steroids** (Not during the last 4 weeks prior to enrolment) | | **IFN-beta-1a** (Not during the last 3 months prior to enrolment) | | **Glatiramer acetate** (Not during the last 3 months prior to enrolment) | | **Dimethyl fumarate** (Not during the last 3 months prior to enrolment) | |
| **# of Patients** | 14 | | 1 | | 3 | | 3 | |
| | | | | | | | | |

| | **CONTROL PATIENTS** | | | | | | | |
|---|---|---|---|---|---|---|---|---|
| | **UWMA***** | | **Pseudotumors** | | **MOGAD PATIENTS** | | **HEALTHY DONORS** | |
| **# of Patients** | 6 | | 5 | | 10 | | 4 | |
| **Female/Male Ratio** | 1.5 | | | | 4 | | 1.5 | |
| **Aqe (y)**** | 33.4 ± 6.9 | | | | 36.2 ± 16.1 | | 27.4 ± 6.3 | |
| | | | | | | | | |
| * PMS, primary progressive and secondary progressive MS | | | | | | | | |
| ** Mean ± standard deviation is shown | | | | | | | | |
| *** undetectable white matter abnormalities | | | | | | | | |

### Standard Protocol Approvals, Registrations, and Patient Consents

The Cantonal Ethics Committee of Zurich approved the study procedures (EC-No. 2013-0001). Informed consent was obtained from all patients.

### CSF and serum measures

Albumin quotients and CSF-specific oligoclonal bands (OCB) were analyzed as previously reported (Puthenparampil et al., Neurol Neuroimmunol Neuroinflamm, 2021, 8(2):e951). Intrathecal Ig synthesis (Ig(Ioc)) was calculated according to Reiber (Reiber et al., 2009, Mult Scler, 15(12):1466-1480).

### Cell culture and stimulation

PBMCs were freshly isolated using Ficoll (Eurobio, Germany) density gradient centrifugation. CSF-infiltrating CD4⁺ T cells were expanded with PHA in a single round of stimulation using a previously reported protocol aimed to reduce TCR repertoire bias (Planas et al., 2008, Sci Transl Med, 10(462):eaat4301). 6 × 10⁴ PHA-expanded CSF-infiltrating CD4⁺ T cells were seeded in quadruplicate with 2 × 10⁵ irradiated autologous PBMCs as antigen presenting cells and stimulated with peptides (Table 2) at a final concentration of 10 µM and with a T Cell Activation Kit (anti-CD3, anti-CD28, anti-CD2 beads) (Miltenyi Biotec) as positive control.

**Table 2. GDP-L-FS, myelin and CEF Peptides (amino acid sequences)**

| **GDP-L-fucose synthase** | | **SEQ ID NO:** |
|---|---|---|
| GDP-L-fucose synthase whole protein | MGEPQGSMRILVTGGSGLVGKAIQKVVADGAG LPGEDWVFVSSKDADLTDTAQTRALFEKVQPTH VIHLAAMVGGLFRNIKYNLDFWRKNVHMNDNVL HSAFEVGARKVVSCLSTCIFPDKTTYPIDETMIHN GPPHNSNFGYSYAKRMIDVQNRAYFQQYGCTF TAVIPTNVFGPHDNFNIEDGHVLPGLIHKVHLAKS SGSALTVWGTGNPRRQFIYSLDLAQLFIWVLRE YNEVEPIILSVGEEDEVSIKEAAEAVVEAMDFH GEVTFDTTKSDGQFKKTASNSKLRTYLPDFRFT PFKQAVKETCAWFTDNYEQARK | 1 |
| GDP-L-fucose synthase 51-65 | TAQTRALFEKVQPTH | 2 |
| GDP-L-fucose synthase 136-150 | PHNSNFGYSYAKRMI | 3 |
| GDP-L-fucose synthase 161-175 | YGCTFTAVIPTNVFG | 4 |
| GDP-L-fucose synthase 246-260 | EEDEVSIKEAAEAVV | 5 |
| GDP-L-fucose synthase 296-310 | FRFTPFKQAVKETCA | 6 |
| GDP-L-fucose synthase 226-270 | LFIWVLREYNEVEPIILSVGEEDEVSIKEAAEAV VEAMDFHGEVT | 37 |
| | | |

| **Myelin** | | |
|---|---|---|
| MBP 13-32 | KYLATASTMDHARHGFLPRH | 7 |
| MBP 83-99 | ENPVVHFFKNIVTPRT | 8 |
| MBP 111-129 | LSRFSWGAEGQRPGFGYGG | 9 |
| MBP 146-170 | AQGTLSKIFKLGGRDSRSGSPMARR | 10 |
| MOG 1-20 | GQFRVIGPRHPIRALVGDEV | 11 |
| MOG 35-55 | MEVGWYRPPFSRVVHLYRNGK | 12 |
| PLP 139-154 | HCLGKWLGHPDKFVGI | 13 |
| | | |

| **CEFT2 Mix (CEF)** | | |
|---|---|---|
| Influenza A | FVFTLTVPSER | 14 |
| Influenza A | SGPLKAEIAQRLEDV | 15 |
| Influenza A | YDVPDYASLRSLVASS | 16 |
| Influenza B | PYYTGEHAKAIGN | 17 |
| Tetanus | GQIGNDPNRDIL | 18 |
| Influenza A | PKYVKQNTLKLA | 19 |
| Influenza A | PKYVKQNTLKLAT | 20 |
| Influenza A | DRLRRDQKS | 21 |
| EBV | AGLTLSLLVICSYLFISRG | 22 |
| Tetanus | QYIKANSKFIGITEL | 23 |
| Tetanus | QYIKANSKFIGITE | 24 |
| Tetanus | FNNFTVSFWLRVPKVSASHLE | 25 |
| EBV | TSLYNLRRGTALA | 26 |
| Tetanus | KFIIKRYTPNNEIDSF | 27 |
| Tetanus | VSIDKFRIFCKALNPK | 28 |
| EBV | VPGLYSPCRAFFNKEELL | 29 |
| HCMV | DKREMWMACIKELH | 30 |
| EBV | TGHGARTSTEPTTDY | 31 |
| EBV | KELKRQYEKKLRQ | 32 |
| Influenza A | RGYFKMRTGKSSIMRS | 33 |
| EBV | TVFYNIPPMPL | 34 |
| EBV | AEGLRALLARSHVER | 35 |
| EBV | PGPLRESIVCYFMVFLQTHI | 36 |

### Proliferative responses

Proliferation was measured 72 h after stimulation using 3H-thymidine (Hartmann Analytic, Braunschweig, Germany) as previously reported (Planas et al., 2018). The stimulation index of single wells (SI) was calculated as follows: SI = (cpm well with peptide) / (Mean cpm wells without peptide). A well was considered positive if SI ≥2. A patient was considered positive for a peptide if the mean SI of the four wells was ≥2 and at least 3 of the four wells showed a SI ≥ 2.

### Quantification of cytokines

Release of IFN-γ was measured using the ELISA MAX^{™} Deluxe Set Human IFN-γ (Biolegend, San Diego, CA, USA) according to the manufacturer's instructions. A well was considered positive if IFN-γ ≥20 pg/ml and a patient was considered positive for a peptide if the mean IFN-γ of the four wells was ≥ 20 pg/ml and at least 3 of the 4 wells were positive.

Cytokines in supernatants and CSF were measured using the Human T Helper Cytokine Panel LEGENDplex bead-based immunoassay (Biolegend) according to the manufacturer's instructions.

### Immunophenotyping

*Ex vivo* immunophenotyping of intrathecal and paired circulating lymphocytes was performed as previously reported (Puthenparampil et al., 2021; Brodie et al., 2016, Cytometry, 89(7):629-32) in a sub-cohort of 66 MS patients (Table 1, *supra*). SPHEROTM AccuCount Particles (Spherotech, Inc. Lake Forest, IL, USA) were added to count absolute numbers following manufacturer's instructions. A LSR Fortessa cytometer (BD Biosciences, Franklin Lakes, NJ, USA) was used to acquire samples and FACSDiva (BD) and FlowJO (TreeStar Inc., Ashland, OR, USA) softwares for the analysis. Figure 1 summarizes the gating strategy.

### Sorting of cell subsets

PBMCs from four MS patients and four age and gender matched HD were labeled with antibodies against CD4 (APC), CD45RA (BV711), CCR7 (BV421), CD27 (PE) and CD28 (APC-Cy7), all from BioLegend and with live-dead aqua dye (Invitrogen). CD4+ CCR7- CD45RA-CD28+ CD27+ and CD4+ CCR7- CD45RA- CD28+ CD27- lived cells were sorted using 100 µm sorting chips in a Sony SH800SFP cell sorter (4 lasers, Sony). 20,000 sorted cells from each cell population were transferred to an RNase-free tube, resuspended in Qiazol (QIAGEN, Germany) and frozen at -80°C.

### RNA extraction, sequencing and analysis

RNA extraction from frozen cell pellets was performed using the PicoPure RNA Isolation kit (Life Technologies) according to the manufacturer's instruction. RNA-sequencing (RNAseq) was performed using Illumina Sequencing 200M at the Functional Genomics Center Zurich as previously reported (Jelcic et al., 2018, Multiple Sclerosis Cell, 175(1):85-100). The RNAseq data analysis consisted of: (i) cleaning of raw reads using Trimmomatic (Version 0.36) (Bolger et al., 2014, Bioinformatics, 30(15):2114-2120); (ii) pseudo alignment of sequences to the Human reference genome (build GRCh38.p13, gene annotation from GENCODE Release 32) and gene expression quantification using Kallisto (Version 0.44) (Bray et al., 2016, Nat Biotechnol, 34(5):525-527); (iii) read alignment using STAR (v2.7.3) (Bray et al., 2016) and (iv) differentially expressed genes detection using a count based negative binomial model implemented in the software package EdgeR (R version: 3.6.1, EdgeR version: 3.28.0) (Robinson et al., 2010, Bioinformatics, 26(1):139-140). A generalized linear model adapted for over-dispersed data was used to assess differential expression. Genes showing p-value < 0.001 for altered expression were considered differentially expressed.

### ELISAs

The following ELISA kits: NF-L (Human Diagnostics, Umea, Sweden); CXCL13/BLC/BCA-1, granzyme A and granulysin (R&D System, MN, USA); CHI3L1 (MicroVue, Athens, OH, USA); perforin, granzyme B and granzyme H (Invitrogen-Thermo Fisher Scientific, MA, USA), were used according to manufacturer's instructions.

### HLA typing

Patients were typed for HLA-class I (A* and B*) and HLA class II (DRB1*, DRB3*, DRB4*, DRB5*, DQA1* and DQB1*) as previously reported (Puthenparampil et al., 2021).

### MOG-antibody detection assay

Serum and CSF samples were analyzed for MOG IgG antibodies as described before (Reindl et al., 2020, Neurol Neuroimmunol Neuroinflamm, 7(2)). For screening, serum samples were diluted 1:20 and 1:40 and CSF samples 1:2. Positive samples were end-point titrated and MOG-IgG positivity was confirmed using an anti-human IgG(Fc) specific secondary antibody as recently described (Reindl et al., 2020).

### MRI

Eight patients were scanned with a 3T Philips Ingenia and 22 patients with 3T Siemens Skyra. The MRI protocol included a 3D pre- and post-Gadolinium contrast-enhanced gradient echo pulse sequence (MPRAGE) as well as a 3D fluid-attenuated inversion recovery (FLAIR) sequence.

From FLAIR, the number and the total volume in ml of all hyperintense lesions were determined by an automatic algorithm based on convolutional neural networks (Kruger et al., 2020, Neuroimage Clin, 28:102445). All results were manually corrected by two experienced technical raters. Differences in the corrections were resolved by consensus in a second reading phase. Similarly, the number of contrast-enhancing lesions was determined.

Whole brain, gray matter, and thalamic volumes in ml were determined on the pre-contrast MPRAGE image using the automatic processing pipeline Biometrica MS^{®} analysis platform (version 2.1, jung diagnostics GmbH, Hamburg, Germany) (Schippling et al., 2017, J Neurol, 264(3):520-528).

### Statistics

GraphPad Prism 8.0 (GraphPad Software, La Jolla, California, USA) was used to perform the statistical analysis. Unpaired T-test was used to compare two groups of normally distributed variables and U-test (Mann-Whitney) for not-normally distributed variables. For the comparison of more than two groups of patients Kruskal-Wallis test was used for not-normally distributed variables. Spearman r was used to test the linear correlation between not-normally distributed variables. The significance level was set at p < 0.05. Associations between patient specificity, seasonal distribution of LP and HLA were performed using Fisher's Exact Test with 5% significance.

### Results

### Identification of GDP-L-FS- and myelin-responders

Proliferation and IFN-γ release of CSF-infiltrating CD4⁺ T cells from 105 MS patients against five immunodominant GDP-L-FS (Planas et al., 2018) and seven myelin peptides (Bielekova et al., 2004) are shown in Figure 2A+B. GDP-L-FS peptides induced the highest frequency of positive wells using proliferation as readout and MOG(35-55) peptide using IFN-γ release (Table 3).

**Table 3. Percentage of positive wells and patients for each peptide using proliferation and IFN-γ release.**

| | **Positive wells %** | | **Positive patients %** | |
|---|---|---|---|---|
| | **Proliferation** | **IFNγ release** | **Proliferation** | **IFNγ release** |
| **GDP-L-FS (51-65)** | 5.48 | 7.16 | 3.80 | 5.71 |
| **GDP-L-FS (136-150)** | 7.14 | 8.35 | 5.71 | 7.62 |
| **GDP-L-FS (161-175)** | 7.38 | 6.92 | 6.66 | 5.71 |
| **GDP-L-FS (246-260)** | 6.90 | 8.85 | 4.76 | 8.57 |
| **GDP-L-FS (296-310)** | 6.43 | 12.65 | 4.76 | 11.42 |
| **MBP (13-32)** | 0.72 | 4.34 | 0.00 | 1.90 |
| **MBP (83-99)** | 1.92 | 6.28 | 0.96 | 5.71 |
| **MBP (111-129)** | 1.20 | 6.75 | 0.96 | 4.76 |
| **MBP (146-170)** | 0.72 | 3.86 | 0.00 | 2.85 |
| **MOG (1-20)** | 1.20 | 4.58 | 0.00 | 1.90 |
| **MOG (35-55)** | 3.61 | 13.49 | 1.92 | 12.38 |
| **PLP (139-154)** | 1.92 | 3.86 | 0.96 | 3.80 |
| | | | | |
| **CEF** | 23.39 | 38.90 | 22.80 | 35.2 |

Responses to a viral/bacterial peptide pool (CEF) (Fig. 2A+B and Table 3) were also analyzed. IFN-γ secretion identified more positive wells than proliferation for myelin and CEF peptides, but comparable results for GDP-L-FS peptides (Fig. 2C). Accordingly, the strongest correlation between proliferation and IFN-γ has been found for GDP-L-FS peptides (Fig. 2D).

The frequency of patients with positive responses to the different peptides is summarized in Table 3. In 14 patients (13.34%), the main reactivity in proliferation and/or IFN-γ release was against several GDP-L-FS peptides and were classified as GDP-L-FS-responders (Fig. 3A+B). Among these, 6 patients (42.8%) showed also positive responses to myelin peptides. The association between GDP-L-FS reactivity and reactivity to myelin peptides was significant (p=<0.0001, Fisher's exact test). Four patients (3.8%) responded only to MBP peptides and were classified as MBP-responders, while eleven patients (10.4%), responding only against MOG(35-55), were classified as MOG(35-55)-responders. 76 patients (72.4%) did not react to any autoantigen and were classified as non-responders (Fig. 3C+D). The response to CEF peptides was comparable in the different groups of patients with the exception of MOG(35-55)-responders, in whom it was lower (Fig. 3A+B). Due to the low number of MBP-responders (<5), this group was not analyzed further.

### Distinct CSF-infiltrating and circulating T cells in GDP-L-FS-responders

Immunophenotyping of CSF-infiltrating and circulating lymphocytes was performed in a sub-cohort of MS patients (Table 1, *supra*) consisting of: GDP-L-FS- (n=7), MOG(35-55)- (n=7) and non-responders (n=52) patients (Table 4).

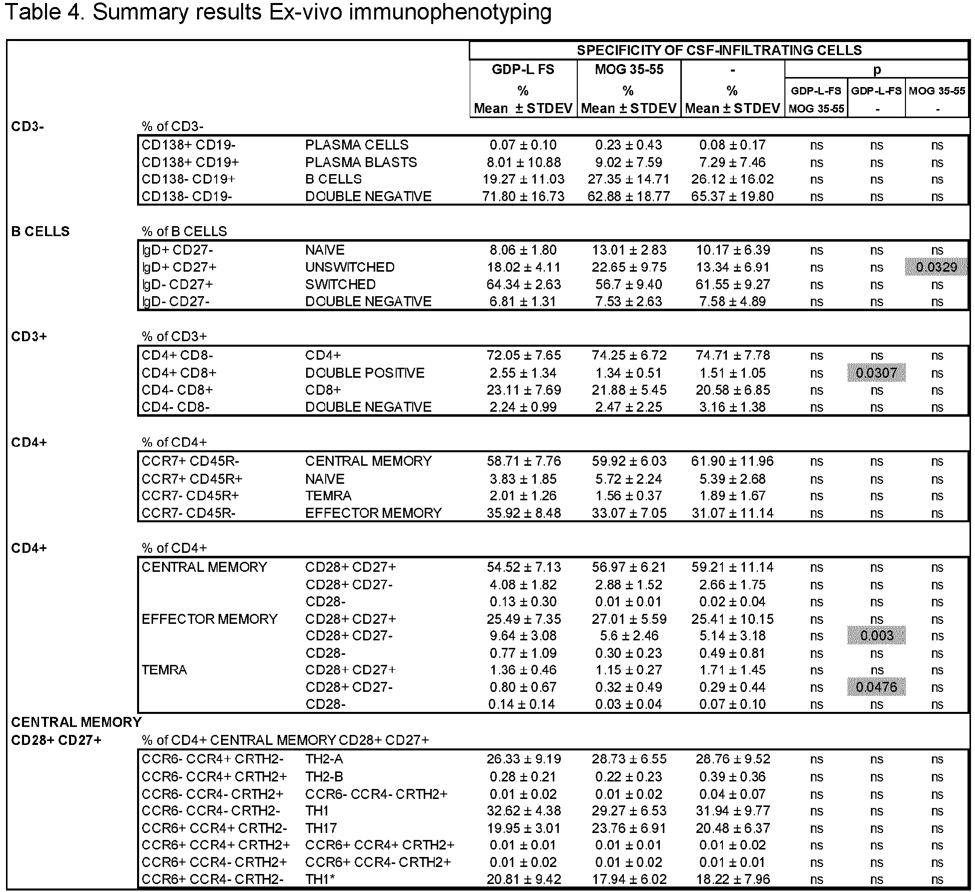

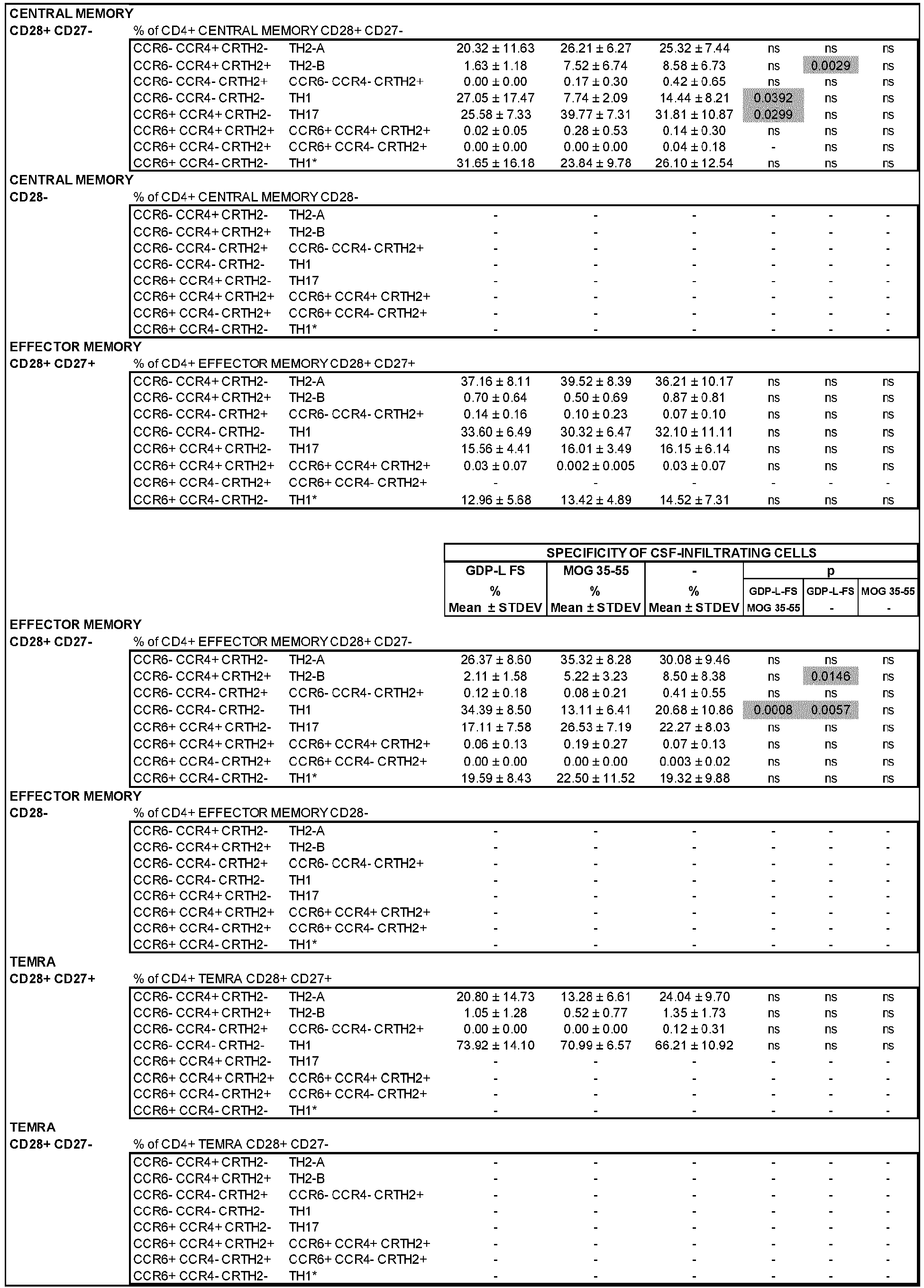

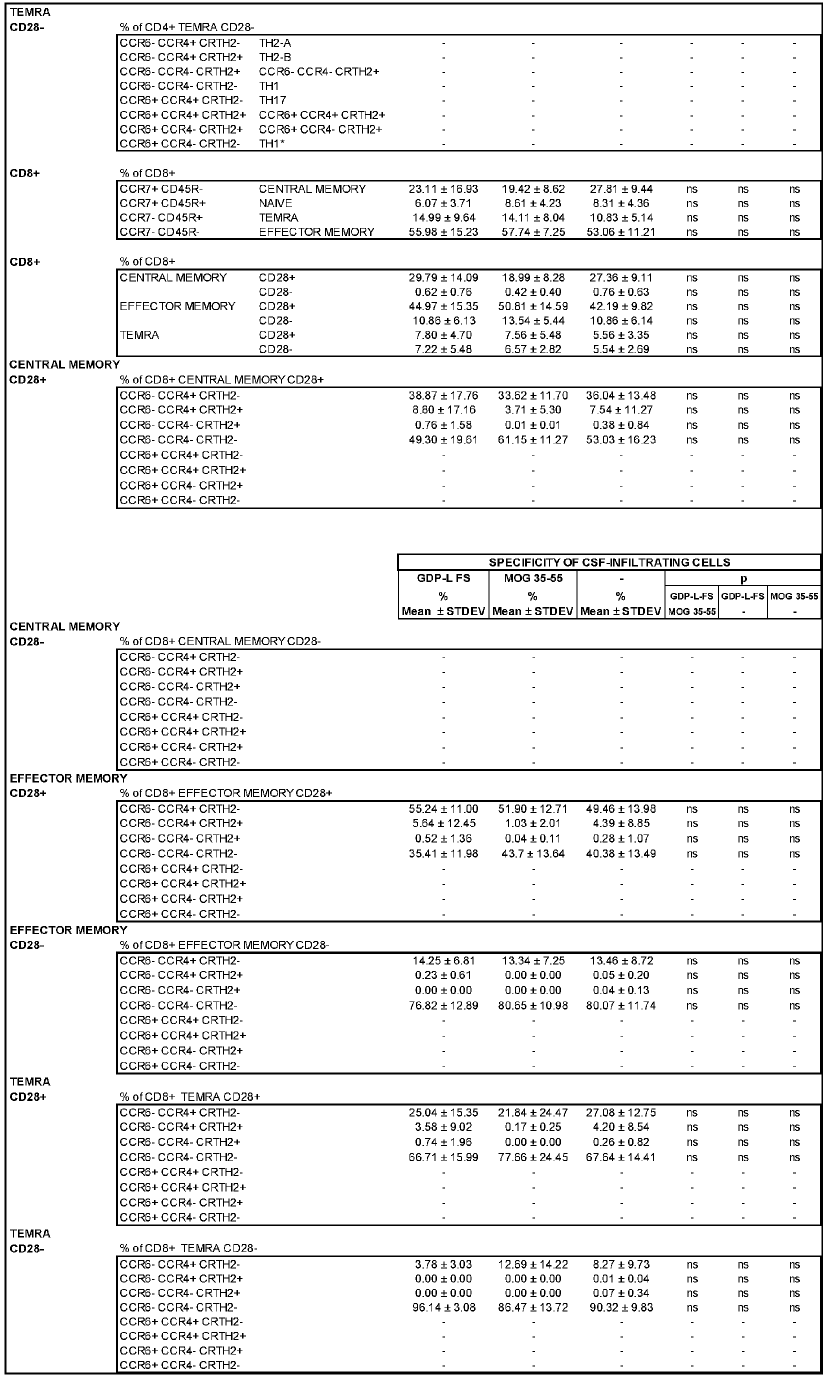

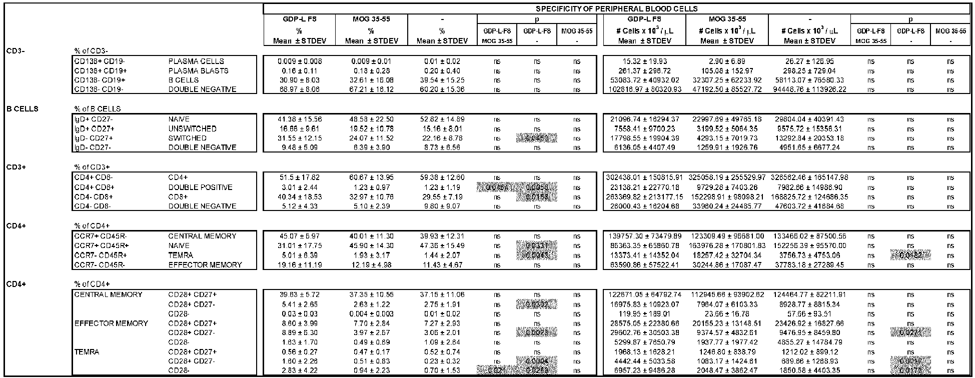

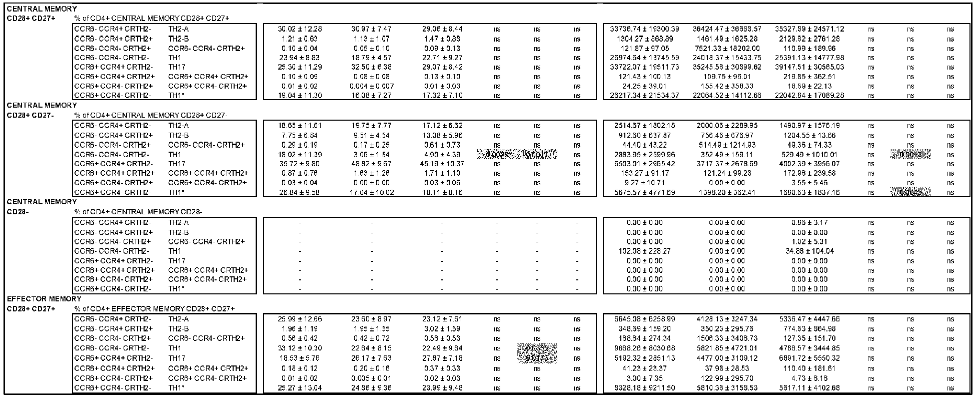

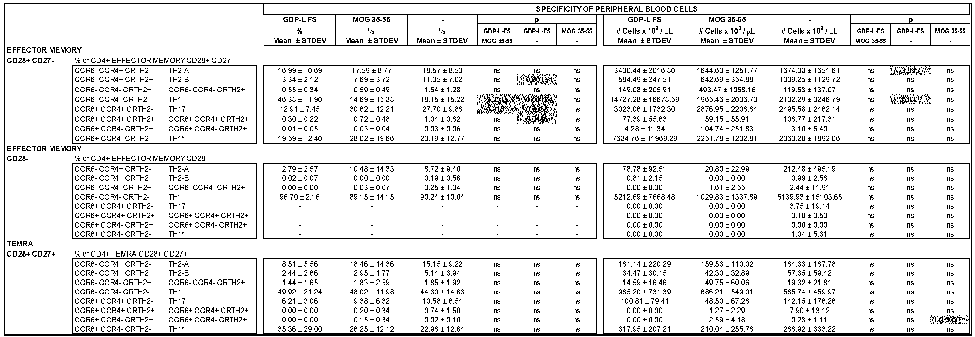

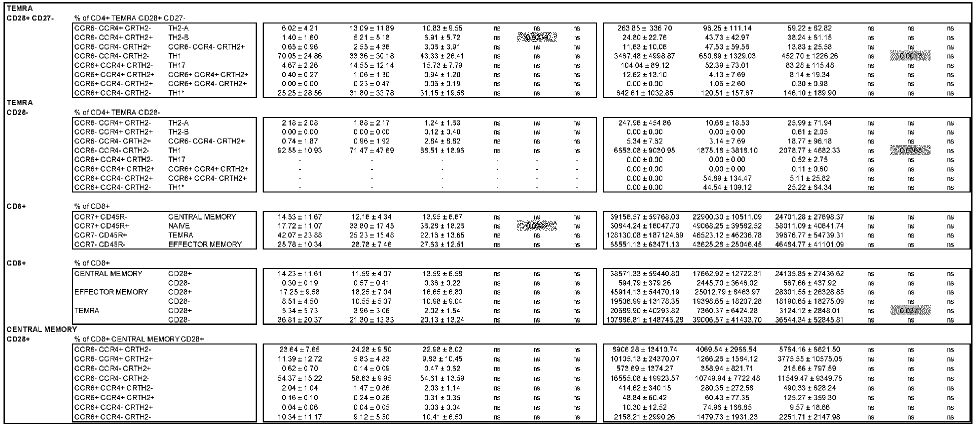

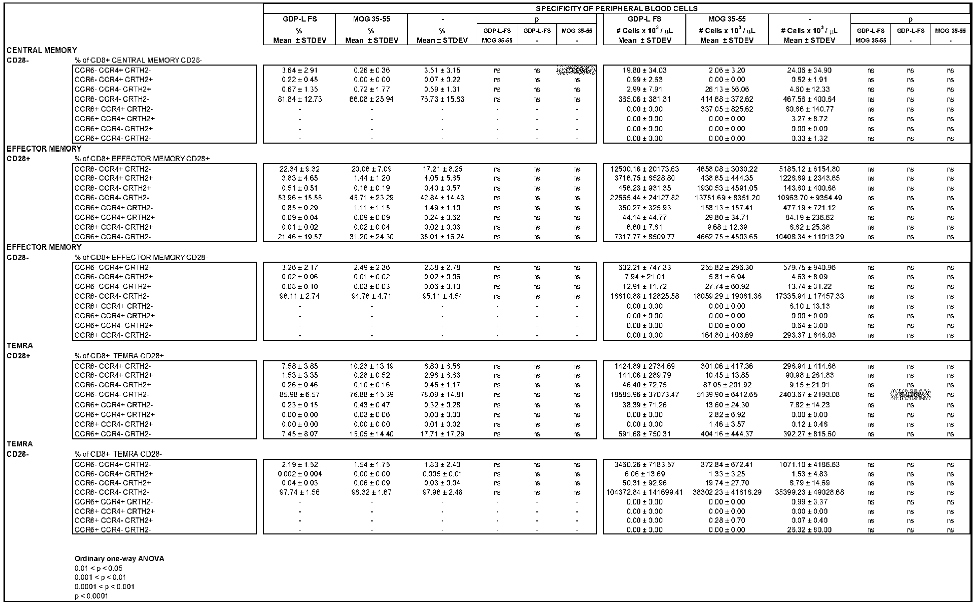

Effector memory (EM, CCR7⁻ CD45RA⁻) CD4⁺ T cells expressing CD28 but not CD27 (EM CD27-), were significantly more abundant in the CSF of GDP-L-FS-responders (Fig. 4A-B and Fig. 5A). Among these EM CD27- cells, only cells with a Th1 (CCR6⁻ CCR4⁻ CRTh2⁻) functional phenotype (EM CD27- Th1), showed significantly higher frequencies (Fig. 4B and Fig. 5B). Analysis of paired blood samples demonstrated that these cells were also significantly more abundant in frequency and absolute counts in the blood (Fig. 4A&C and Fig. 5C-F). CSF-infiltrating and circulating CD4⁺ T cells with a TEMRA (CCR7⁻ CD45RA⁺) differentiation state and the co-stimulatory molecules CD28⁺ CD27⁻ and CD28- were also more abundant in GDP-L-FS-responders (Fig. 5A&C-F).

### Transcriptional signatures of EM CD27- CD4+ T cells in GDP-L-FS-responders

EM CD27- and CD27+ CD4⁺ T cells were sorted from the peripheral blood of four GDP-L-FS-responders (Fig. 6A) and the transcriptome analyzed by RNAseq. 265 differentially expressed genes (fold change 1.5, p< 0.001) were identified, 119 upregulated and 146 downregulated, in EM CD27- versus EM CD27+ cells (Fig. 7A). The upregulated genes included: (i) transcripts linked to cytotoxic CD8+ (Hidalgo et al., 2008, Am J Transplant, 8(3):627-636) and CD4+ (Patil et al., 2018, Sci Immunol, 3(19)) T cells such as *ADGRG1* (*GPR56*), *ADGRG5* (*GPR114*), *CCL4, CCL5, CST7, CTSW, CX3CR1, ENC1, FCRL6, FGFBP2, GNLY, GZMB, GZMH, MYO6, PRF1, PRSS23, S1PR5, SLAMF7, SPON2, TGFBR3, TRGC2* and *ZEB2;* (ii) transcription factors (TFs) important in the development of cytotoxic CD4⁺ T cells such as Eomes (Pearce et al., 2003, Sci, 302(5647):1041-1043) and T-bet (encoded by *TBX21*) (Eshima et al., 2012, Immunol Lett, 144(1-2):7-15); and (iii) other genes associated with Th1 cells such as *IFNG* and *GZMA.* In contrast, cytokines, chemokine receptors and TFs associated with other Th subsets (*CCR4, CCR6, IL4R, IL6R, GATA3* and *FoxP3*) were downregulated in EM CD27- cells.

The same sorted EM subpopulations from four HD (Fig. 6A) were also analyzed. The 265 genes that differentiate EM CD27- and CD27+ cells in GDP-L-FS-responders did not discriminate EM subpopulations in HD (Fig. 7A). Differential gene expression analysis of EM CD27- cells from GDP-L-FS-responders and HD also identified 145 differentially expressed genes (fold change 1.5, p<0.001), with 66 upregulated in the GDP-L-FS-responders that include 19 genes linked to cytotoxicity and Th1 T cells and 79 downregulated including genes associated with other T cell subsets (Fig. 6B and Fig. 7A).

The transcription of genes characteristically associated with Th1 cells and cytotoxicity (TBX21, GZMA, EOMES, GNLY, GZMH and SLAMF7) was overall increased and transcription of genes associated with other Th subsets (GATA3, CCR4 and CCR6) decreased in EM CD27- cells from GDP-L-FS-responders compared to the same cells from HD and to EM CD27+ cells (Fig. 7B).

The genes identified in Figures 6 and 7 have the following identifiers (GENCODE database, Release 32 (GRCh38.p13)):

| **Gene** | **Identifier** |
|---|---|
| CD27 | ENSG00000139193 |
| CD28 | ENSG00000178562 |
| ADGRG1 | ENSG00000205336 |
| ADGRG5 | ENSG00000159618 |
| B3GAT1 | ENSG00000109956 |
| CCL4 | ENSG00000275302 |
| CCL5 | ENSG00000271503 |
| CST7 | ENSG00000077984 |
| CTSW | ENSG00000172543 |
| CX3CR1 | ENSG00000168329 |
| ENC1 | ENSG00000171617 |
| FCRL6 | ENSG00000181036 |
| FGFBP2 | ENSG00000137441 |
| GNLY | ENSG00000115523 |
| GZMB | ENSG00000100453 |
| GZMH | ENSG00000100450 |
| MYO6 | ENSG00000196586 |
| PRF1 | ENSG00000180644 |
| PRSS23 | ENSG00000150687 |
| S1PR5 | ENSG00000180739 |
| SLAMF7 | ENSG00000026751 |
| SPON2 | ENSG00000159674 |
| TGFBR3 | ENSG00000069702 |
| TRGC2 | ENSG00000227191 |
| ZEB2 | ENSG00000169554 |
| GZMA | ENSG00000145649 |
| EOMES | ENSG00000163508 |
| IFNG | ENSG00000111537 |
| TBX21 | ENSG00000152217 |
| CCR4 | ENSG00000183813 |
| CCR6 | ENSG00000112486 |
| GATA3 | ENSG00000107485 |
| IL4R | ENSG00000077238 |
| FOXP3 | ENSG00000049768 |
| IL6R | ENSG00000160712 |
| IL12RB2 | ENSG00000081985 |
| IL17RB | ENSG00000056736 |

### Characterization of GDP-L-FS- and MOG(35-55)-specific responses

The functional phenotype of GDP-L-FS- and MOG(35-55)-specific CD4⁺ T cells was analyzed in the supernatant of positive wells stimulated with the cognate antigen (Fig. 8A+B). GDP-L-FS-specific cells released significantly higher amounts of IL-2 than MOG(35-55)-specific cells. This higher IL-2 is most likely involved in the significantly stronger ability of GDP-L-FS-specific cells to proliferate in response to specific peptides, while the proliferation to unspecific stimulating beads was comparable in GDP-L-FS- and MOG(35-55)-specific cells (Fig. 8C upper graph). IFN-γ was the main cytokine released by GDP-L-FS-specific cells indicating a Th1 functional phenotype. MOG(35-55)-specific cells, in addition to IFN-γ, released cytokines associated with other Th subsets (IL-9, IL-6 and IL-10) suggesting a polyfunctional phenotype (Fig. 8A+B). IL-10 also was significantly higher in the CSF of MOG(35-55)-responder as well as Th2 associated cytokines (Fig. 8A+B). Since some of the cytokines released by MOG(35-55)-specific cells have been found elevated in CSF from MOGAD patients (Kaneko et al., 2018, J Neurol Neurosurg Psychiatry, 89(9):927-936), and since both groups of patients share the target antigen, it was verified that none of the MS patients had positive anti-MOG IgG titers in serum or CSF (Table 5) and the cytokine profile in the CSF from MOGAD patients was analyzed (Table 1 (*supra*) and Fig. 9A).

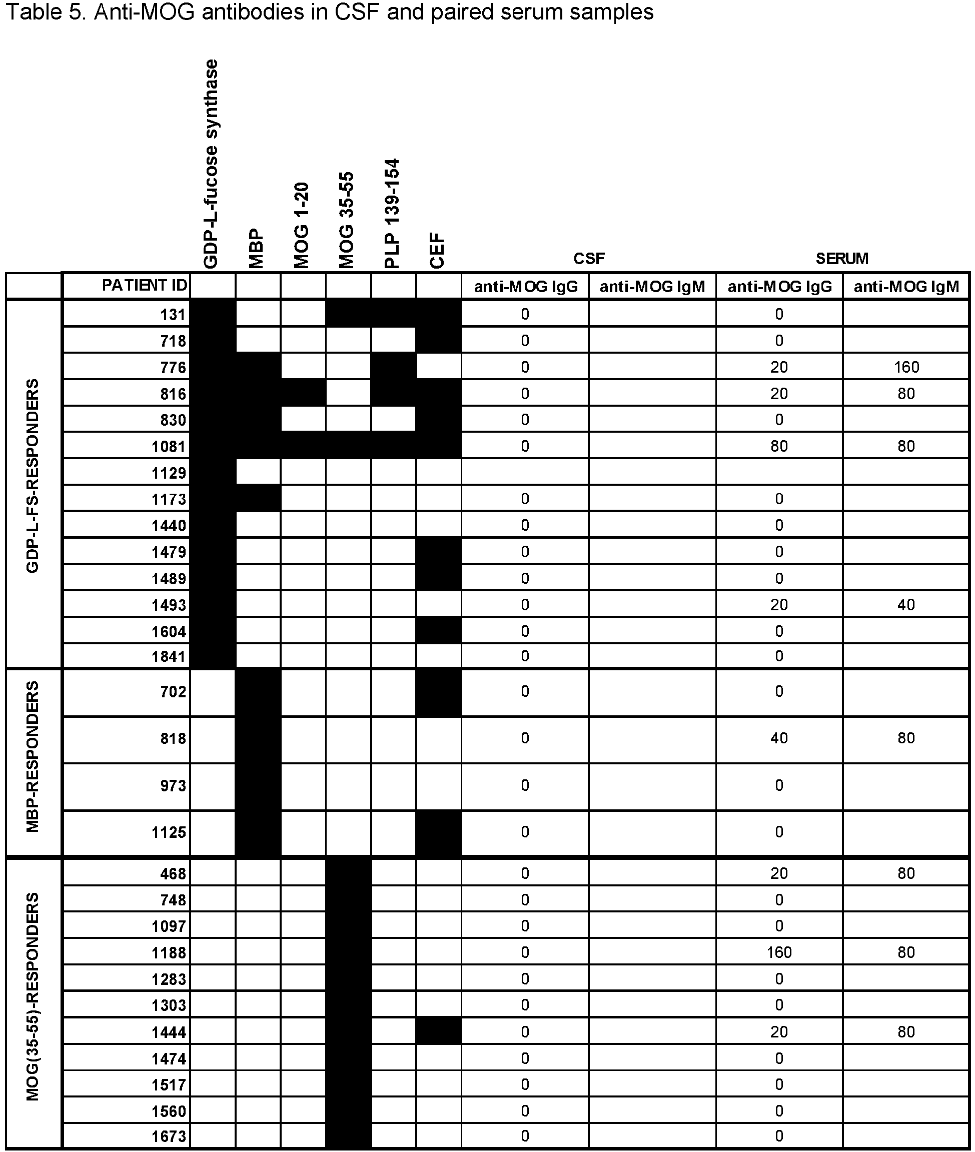

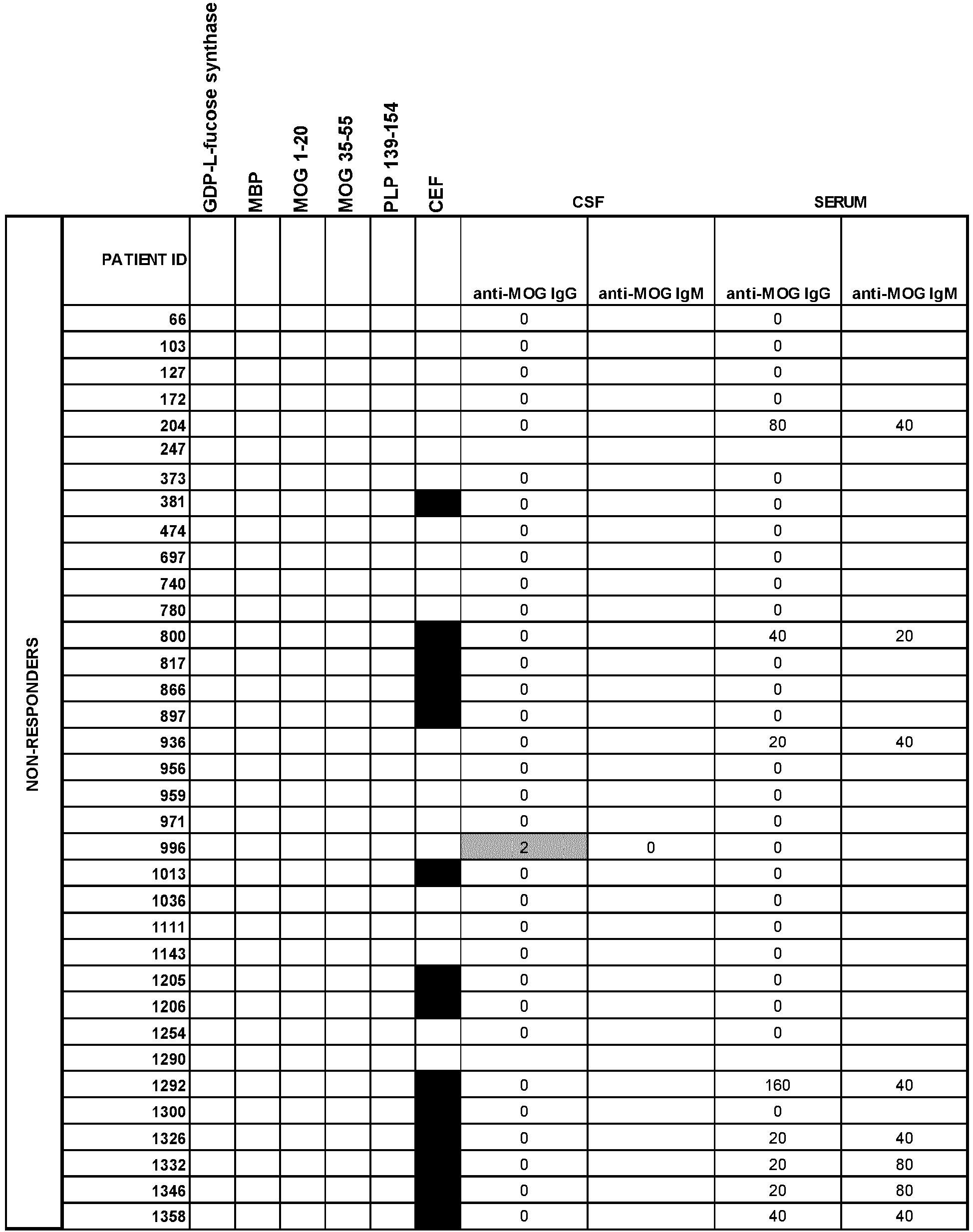

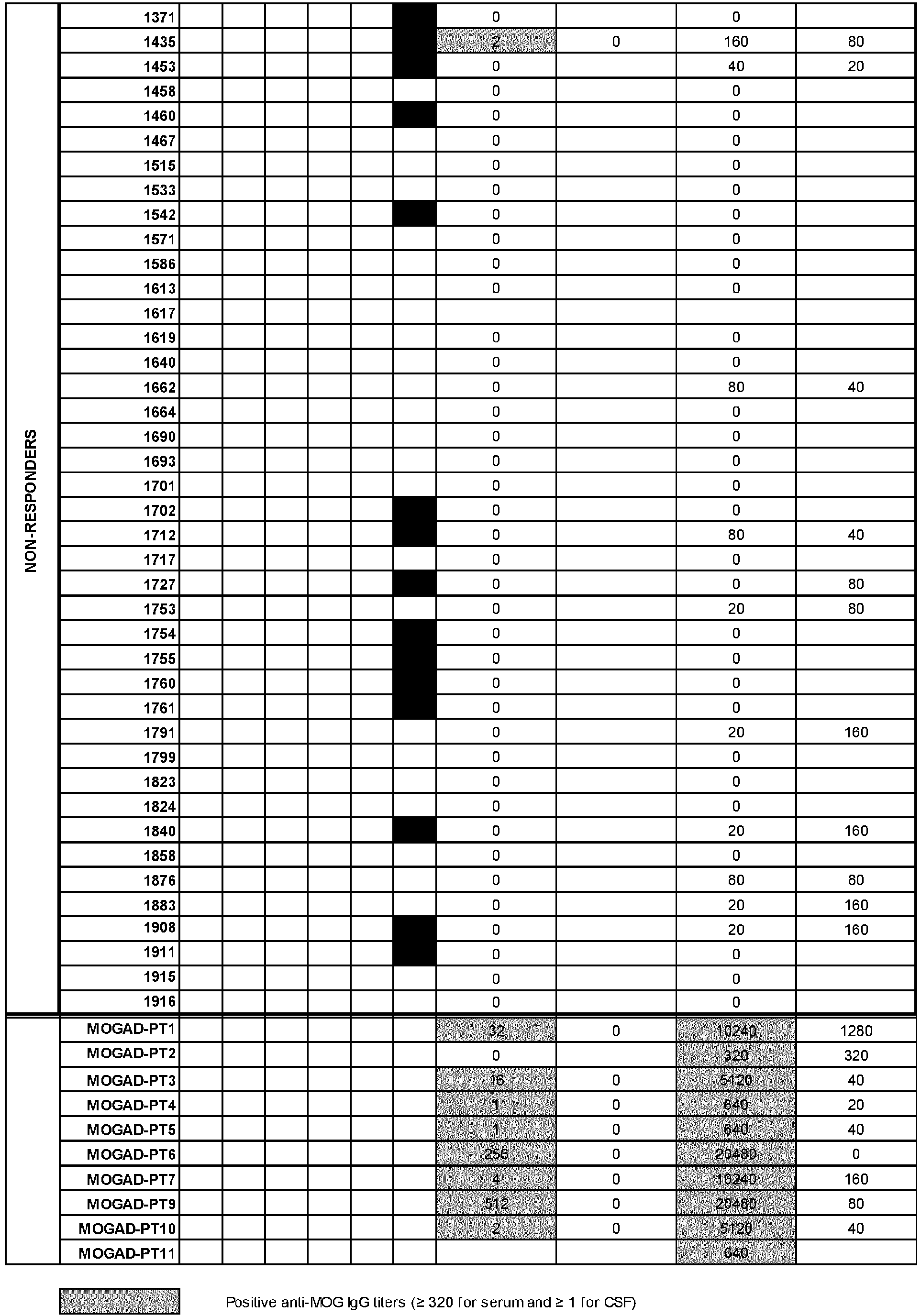

Although cytokines such as IL-9, IL-22 and IL-6 were high in both groups, the Th2-associated cytokines IL-4 and IL-10 were significantly higher in MOG(35-55)-responders (Fig. 9A).

Supporting a role of humoral immunity in MOG(35-55)-responders, significantly higher frequencies of CSF-infiltrating- but not circulating naïve B cells were found in these patients (Fig. 8C lower graph). Intrathecal amounts of CXCL13 (Ansel et al., 2000, Nature, 406(6793):309-314) was higher in MOG(35-55)-responders and CHI3L1, a general marker of inflammation (Bonneh-Barkay et al., 2012, Brain Pathol, 22(4):530-546), in GDP-L-FS-responders when compared with control patients (CP, Table 1 (*supra*)) (Fig. 9B). Both groups of patients showed significantly higher intrathecal IgG synthesis than CP (Fig. 9B).

Further CSF analysis of cytotoxic and neurodegenerative markers revealed undetectable amounts of perforin and granzyme B, no significant differences for granulysin and higher levels of granzyme H, granzyme A and neurofilament light chain (NfL) (Bergman et al., 2016, Neurol Neuroimmunol Neuroinflamm, 3(5):e271) in GDP-L-FS- responders (Fig. 9C).

### Association of GDP-L-FS reactivity and HLA-DRB3*02:02/03:01

All patients were HLA typed (Table 6).

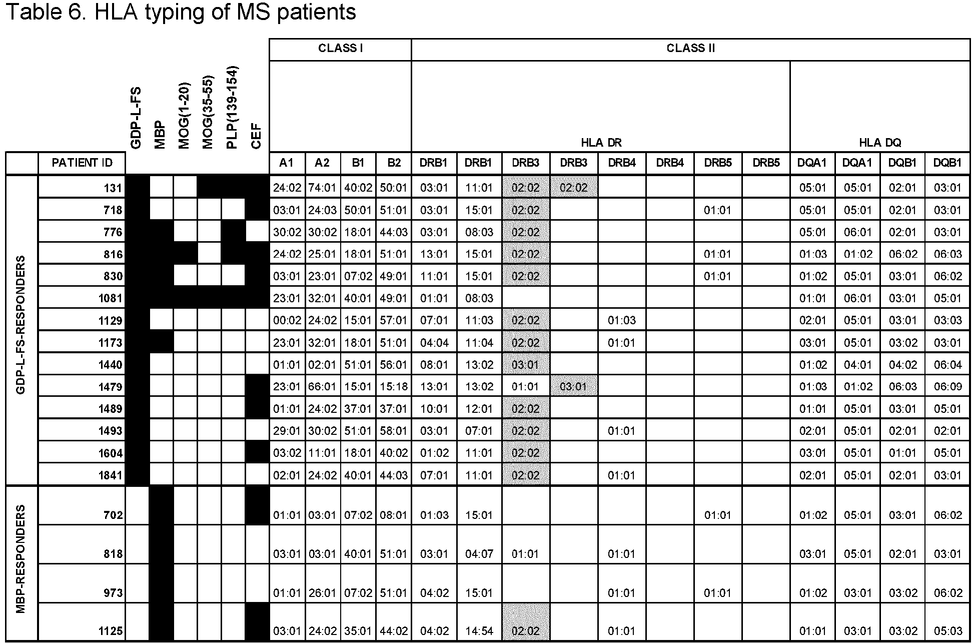

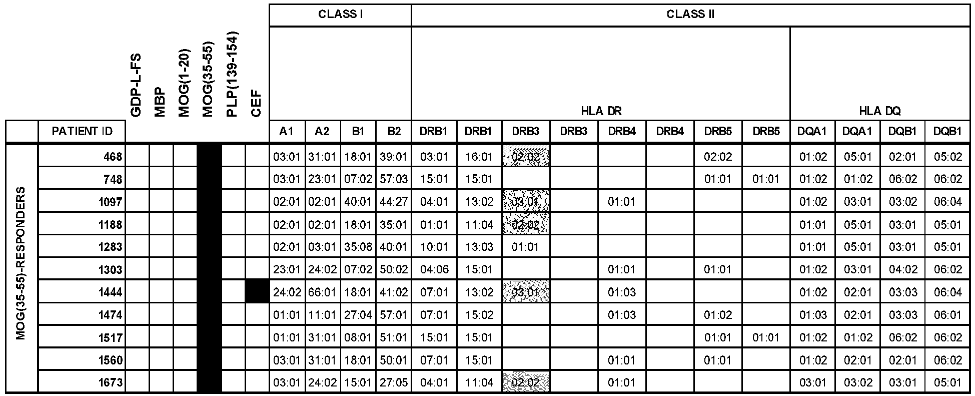

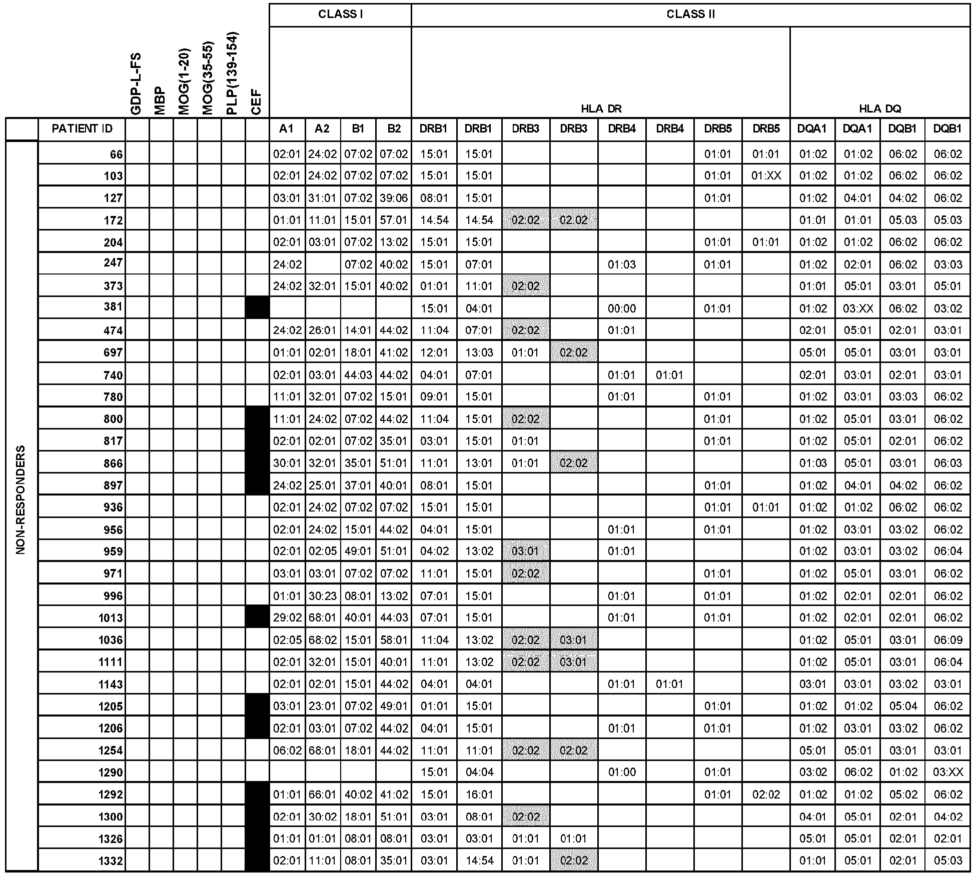

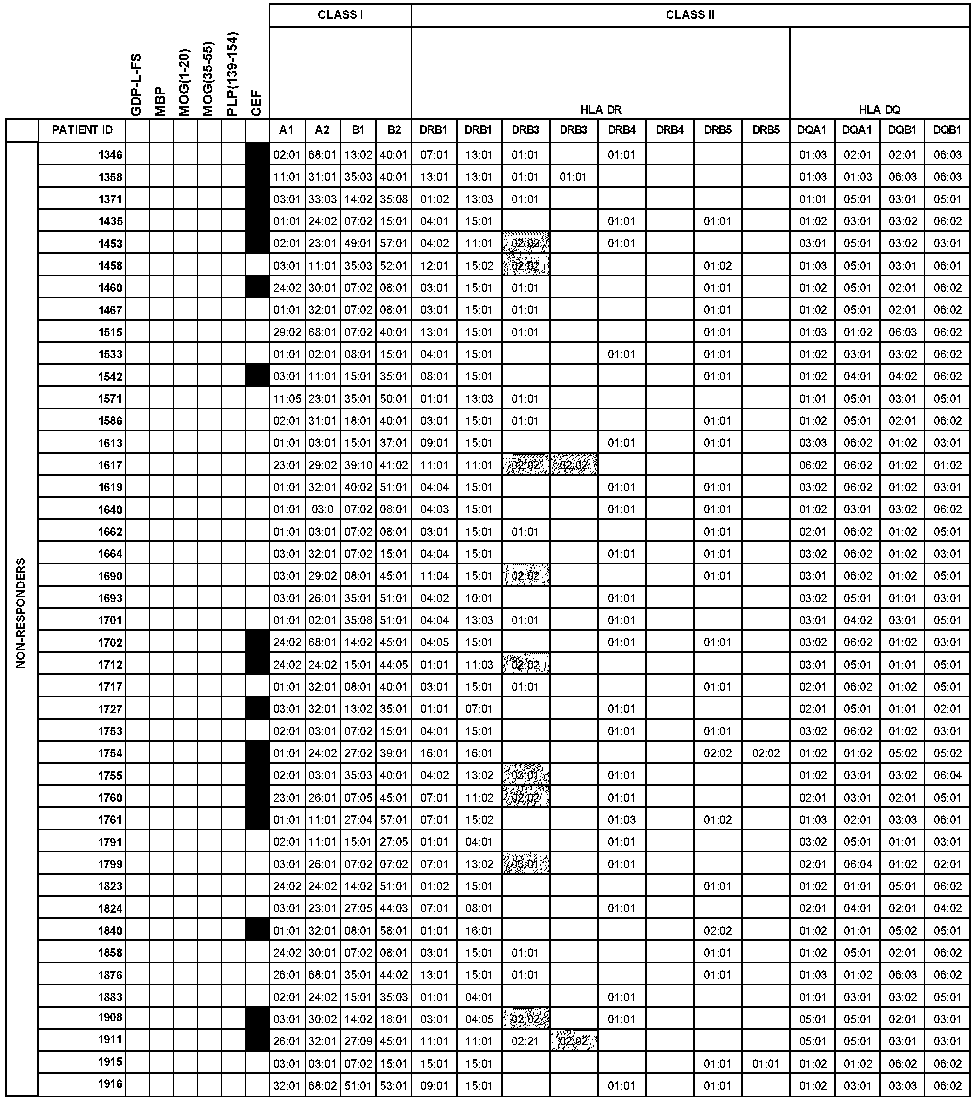

Figure 10A summarizes frequencies of GDP-L-FS-, MOG(35-55)- and non-responders expressing the MS-associated DR15 haplotype and DRB3*02:02/03:01 alleles. Based on two previously typed MS cohorts (German cohort (n=1270) and Swiss cohort (n=367)), it was expected that 40-50% of MS patients express the DR15 haplotype and 30-40% the DRB3*02:02/03:01 alleles. The frequency of GDP-L-FS-responders expressing the DR15 haplotype (21.4%) was lower than expected, while those expressing the DRB3*02:02/03:01 alleles (92.8%) was much higher. The association between GDP-L-FS specificity and DRB3*02:02/03:01 alleles was significant (p=<0.0001, Fisher's exact test, Fig. 10A).

Figure 10B+C show CSF-infiltrating CD4⁺ T cell responses to GDP-L-FS, myelin and CEF peptides as well as stimulating beads in patients expressing or not DRB3*02:02/03:01 alleles. Only the response to GDP-L-FS peptides, both by proliferation and IFN-γ release, was significantly higher in DRB3*02:02/03:01 patients than in patients expressing other HLA class II alleles.

### Demographic and clinical features of patients with different specificity

Demographic and clinical characteristics of GDP-L-FS-, MOG(35-55)- and non-responder patients did not show significant differences (Table 7).

**Table 7. Features of MS patients with different specificity**

| **Demographic and clinical features** | | | | |
|---|---|---|---|---|
| | **All** | **GDP-L-FS** | **MOG 35-55** | **NON** |
| ***Number of patients*** | 105 | 14 | 11 | 76 |
| ***Femal*/*Male ratio*** | 1.9 | 1.8 | 0.8 | 2.6 |
| ***Age (years)*** | 34.7 ± 9.1 | 39.1 ± 11.2 | 37.8 ± 11.8 | 34.7 ± 9.2 |
| ***Age at onset (years)*** | 33.2 ± 9.3 | 37.1 ± 10.3 | 35.7 ± 12.3 | 33.1 ± 8.7 |

| ***Clinical course*** | | | | |
|---|---|---|---|---|
| ***RIS*/*CIS (%)*** | 13.3 | 14.3 | 9.1 | 15.8 |
| ***RRMS (%)*** | 78.1 | 78.6 | 81.8 | 76.3 |
| ***PMS* (%)*** | 8.6 | 7.1 | 9.1 | 7.9 |
| ***Disease duration (months)*** | 28.6 ± 51.9 | 24.3 ± 47.1 | 24.6 ± 31.4 | 30.1 ± 55.9 |
| ***CSF-specific OCB (%)*** | 94.2 | 100.0 | 100.0 | 97.4 |

| **Markers of altered BBB permeability, clinical activity and brain MRI findings** | | | | |
|---|---|---|---|---|
| | **GDP-L-FS** | **MOG 35-55** | **P******* | |
| ***CSF cell count (cells*/*uL)*** | 7.07 ± 8.87 | 8.00 ± 9.80 | ns | |
| ***BBB damage** (% patients)*** | 35.70 | 36.36 | ns | |
| ***Time since last relapse (days)*** | 57.09 ± 76.94 | 132.20 ± 220.04 | ns | |
| ***Clinically active (% patients)*** | 76.92 | 81.81 | ns | |
| ***Brain MRI semi-automatic analysis****** | | | | |
| ***Volume absolute (mL and z-values******)*** | | | | |
| ***Whole brain (mL)*** | 1020.58 ± 209.41 | 1124.69 ± 100.99 | ns | |
| ***z-values*** | | | | |
| ***Thalamus (mL)*** | 10.57 ± 1.56 | 11.86 ± 1.03 | ns | |
| ***z-values*** | | | | |
| ***Gray Matter (mL)*** | 630.05 ± 125.44 | 670.40 ± 48.46 | ns | |
| ***z-values*** | | | | |
| ***Gd contrast enhancing T1 lesions*** | 1.50 ± 1.04 | 0.00 ± 0.00 | **0.001465** | |
| ***FLAIR T2 lesions (*#*)*** | 25.83 ± 25.74 | 17.85 ± 22.01 | ns | |
| ***FLAIR T2 lesions total volume*** | 4.19 ± 3.17 | 1.25 ± 1.04 | **0.048184** | |

| | | | | |
|---|---|---|---|---|
| **PMS, SPMS and PPMS.* ** *BBB damage (Q_{ALB}-Q_{NORM} >0). ***Brain MRI semi-automatic analysis was performed in 6 GDP-L-FS and 8 MOG 35-55 responder patients from which brain MRIs at the time of LP were available. ****z-values, brain volumes taking into account age and the total intracranial volume. *****p. for the comparison of two groups of patients we used unpaired T-test for normally distributed variables and U-test (Mann-Whitney) for not-normally distributed variables.* | | | | |

Further characterization revealed a different seasonal distribution regarding the time point when the LP had been performed (Fig. 11A+B), with a significant association between GDP-L-FS specificity and LP during winter/spring months (=<0.0001, Fisher's exact test).

Markers of compromised BBB permeability, disease activity and several MRI parameters were also examined (Table 7, *supra*). While most of these markers were comparable between GDP-L-FS- and MOG(35-55)-responders, the semi-automated and blinded analysis of retrospectively collected brain MRI scans from six GDP-L-FS- and eight MOG(35-55)-responders revealed statistically significant differences. The total number of contrast-enhancing T1 lesions as well as the total volume of FLAIR T2 lesions (Fig. 11C) were significantly higher in GDP-L-FS-compared to MOG(35-55)-responders.

### Summary of main results

This study represents the most comprehensive analysis of T cell specificity performed in MS to date since it: (i) analyzes CSF-infiltrating CD4⁺ T cells instead of PBMCs, (ii) evaluates the reactivity not only against seven long-known immunodominant/encephalitogenic myelin peptides (Bielekova et al., 2004) but also to five recently discovered immunodominant GDP-L-FS peptides, (iii) uses as a readout IFN-γ release in addition to proliferation based on the relevant role of Th1 cells in MS (Bielekova et al., 2000, Nat Med, 6(10):1167-1175; Planas et al., 2018; Jelcic et al., 2018; Bielekova et al., 2004), and (iv) is performed in a large cohort of 105 MS patients. Proliferation confirmed a stronger reactivity against GDP-L-FS peptides while IFN-γ release identified MOG(35-55) as the strongest stimulatory peptide. 14 GDP-L-FS-, four MBP- and eleven MOG(35-55)-responders were identified.

GDP-L-FS-responders frequently also recognized myelin peptides. Interestingly, ex-vivo immunophenotyping demonstrated that EM CD27- Th1 CD4+ were significantly more abundant in the CSF and also peripheral blood of GDP-L-FS-responders and that these cells in the blood expressed Th1- and cytotoxicity-associated genes (Patil et al., 2018).

Cytokine analysis showed a role of Th1 responses in GDP-L-FS-responders and Th2 in MOG(35-55)-responders.

GDP-L-FS-specific responses were associated with DRB3*02:02/03:01 alleles, and were significantly more frequent in CSF samples obtained during winter and spring.

Finally, GDP-L-FS- and MOG(35-55)-responders also differed regarding MRI findings. It is remarkable that GDP-L-FS-responders showed significantly higher numbers of contrast-enhancing T1 lesions and higher volumes of FLAIR T2 lesions indicating higher inflammation and more extensive demyelination accordingly with more damaging immune responses.

In summary, the results reveal, for the first time in a T cell-mediated autoimmune disease, an association between T cell specificity and features of disease heterogeneity. The results might have important implications for personalized treatment approaches aiming at antigen-specific tolerance.

### EMBODIMENTS

1. Method for stratification of a multiple sclerosis (MS) patient comprising the following steps:
   - obtaining body fluid, in particular blood, preferably peripheral blood, or cerebrospinal fluid (CSF), from an MS patient, and
   - detecting CD27- Th1 CD4+ cells in the body fluid.
2. The method according to embodiment 1, wherein the method further comprises:
   - detecting responsiveness of T cells and/or antibodies in the body fluid to the protein GDP-L-fucose synthase (GDP-L-FS) or a fragment, derivative and/or splice variant thereof.
   In other words, a method according to embodiment 1 is provided as embodiment 2, wherein the method further comprises:
   - detecting responsiveness of T cells and/or antibodies in the body fluid to GDP-L-fucose synthase (GDP-L-FS) protein or a fragment, derivative and/or splice variant thereof.
   A method for stratification of a multiple sclerosis (MS) patient is particularly preferred which comprises
   - obtaining peripheral blood or cerebrospinal fluid (CSF) from an MS patient, and
   - detecting CD27- Th1 CD4+ cells in the peripheral blood or CSF, and
   - detecting responsiveness of T cells and/or antibodies in the peripheral blood or CSF to the protein GDP-L-fucose synthase (GDP-L-FS) or a fragment, derivative and/or splice variant thereof.
   GDP-L-FS protein has been identified as an autoantigen. Hence, a method according to embodiment 1 is provided as embodiment 2, wherein the method further comprises:
   - detecting responsiveness of T cells and/or antibodies in the body fluid to an autoantigen, wherein the autoantigen is the protein GDP-L-fucose synthase (GDP-L-FS) or a fragment, derivative and/or splice variant thereof.
   In other words, a method according to embodiment 1 is provided as embodiment 2, wherein the method further comprises:
   - detecting responsiveness of T cells and/or antibodies in the body fluid to an autoantigen, wherein the autoantigen is GDP-L-fucose synthase (GDP-L-FS) protein or a fragment, derivative and/or splice variant thereof.
3. The method according to embodiment 2, wherein the protein GDP-L-FS
   a) has an amino acid sequence as set forth in SEQ ID NO: 1 or
   b) has an amino acid sequence which is at least 85 %, preferably at least 90 %, more preferably at least 95 % identical to the amino acid sequence as set forth in SEQ ID NO: 1 or
   c) has an amino acid sequence which is at least 70 %, preferably at least 80 %, more preferably at least 90 % homologous to the amino acid sequence as set forth in SEQ ID NO: 1 or
   d) has an amino acid sequence which is at least 60 %, preferably at least 70 %, more preferably at least 80 %, even more preferably at least 90 % homologous to the amino acid sequence as set forth in SEQ ID NO: 1 and the protein or fragment or splice variant thereof binds to an autologous HLA allele, is recognized by a T cell and/or is recognized by an antibody which binds to or recognizes the amino acid sequence as set forth in SEQ ID NO: 1 or a fragment thereof or
   e) is encoded by a TSTA3 gene, in particular by a gene sequence of nucleotides 143612618 to 143618048 of NC_000008.11, or is encoded by a gene which is at least 80 %, preferably at least 90 %, even more preferably at least 95 % identical to the gene sequence of nucleotides 143612618 to 143618048 of NC_000008.11.
4. The method according to embodiment 2 or 3, wherein the fragment comprises 5 to 50, preferably 5 to 20, more preferably 10 to 15 amino acids, even more preferably 15 amino acids.
5. The method according to any one of embodiments 2 to 4, wherein the fragment is
   a) at least 85 %, preferably at least 90 %, more preferably at least 95 % identical to a respective corresponding amino acid sequence or
   b) at least 70 %, preferably at least 80 %, more preferably at least 90 % homologous to a respective corresponding amino acid sequence or
   c) at least 60 %, preferably at least 70 %, more preferably at least 80 %, even more preferably at least 90 % homologous to a respective corresponding amino acid sequence and binds to an autologous HLA allele, is recognized by a T cell and/or is recognized by an antibody which binds to or recognizes the respective amino acid sequence.
6. The method according to any of the preceding embodiments, wherein the fragment comprises a sequence selected from the group comprising SEQ ID NOs: 2 to 6 and SEQ ID NO: 37, preferably consists of a sequence selected from the group comprising SEQ ID NOs: 2 to 6 and SEQ ID NO: 37.
7. A GDP-L-FS protein or a fragment, derivative or splice variant thereof, or a nucleotide sequence encoding the GDP-L-FS protein or fragment, derivative or splice variant thereof as defined in any one of embodiments 3 to 6, for use in the treatment of MS in an MS patient, wherein CD27- Th1 CD4+ cells are detected in body fluid, in particular blood, preferably peripheral blood, or CSF, previously obtained from the MS patient.
8. The GDP-L-FS protein or a fragment, derivative or splice variant thereof, or the nucleotide sequence encoding the GDP-L-FS protein or fragment, derivative or splice variant thereof for use according to embodiment 7, wherein T cells and/or antibodies previously obtained from the body fluid of the MS patient respond to the protein GDP-L-FS or a fragment, derivative and/or splice variant thereof.
   In a particularly preferred embodiment, a GDP-L-FS protein or a fragment, derivative or splice variant thereof, or a nucleotide sequence encoding the GDP-L-FS protein or fragment, derivative or splice variant thereof as defined in any one of embodiments 3 to 6, for use in the treatment of MS in an MS patient is provided, wherein CD27- Th1 CD4+ cells have been detected in peripheral blood or CSF previously obtained from the MS patient, and wherein T cells and/or antibodies previously obtained from the peripheral blood or CSF of the MS patient respond to the protein GDP-L-FS or a fragment, derivative and/or splice variant thereof.
9. At least one GDP-L-FS protein, fragment, derivative, splice variant, nucleotide sequence and/or gene sequence as defined in any of embodiments 3 to 6, and/or at least one carrier coupled to at least one GDP-L-FS protein, fragment, derivative, splice variant, nucleotide sequence and/or gene sequence as defined in any of embodiments 3 to 6 for use in a method for inducing antigen-specific tolerance to autoantigens in an MS patient, wherein CD27- Th1 CD4+ cells are detected in body fluid, in particular blood, preferably peripheral blood, or CSF, previously obtained from the MS patient.
10. The at least one GDP-L-FS protein, fragment, derivative, splice variant, nucleotide sequence and/or gene sequence and/or the at least one carrier coupled to at least one GDP-L-FS protein, fragment, derivative, splice variant, nucleotide sequence and/or gene sequence for use according to embodiment 9, wherein T cells and/or antibodies previously obtained from the body fluid of the MS patient respond to the protein GDP-L-FS or a fragment, derivative and/or splice variant thereof.
   In a particularly preferred embodiment, at least one GDP-L-FS protein, fragment, derivative, splice variant, nucleotide sequence and/or gene sequence as defined in any of embodiments 3 to 6, and/or at least one carrier coupled to at least one GDP-L-FS protein, fragment, derivative, splice variant, nucleotide sequence and/or gene sequence as defined in any of embodiments 3 to 6 for use in a method for inducing antigen-specific tolerance to autoantigens in an MS patient is provided, wherein CD27- Th1 CD4+ cells have been detected in peripheral blood or CSF previously obtained from the MS patient, and wherein T cells and/or antibodies previously obtained from the peripheral blood or CSF of the MS patient respond to the protein GDP-L-FS or a fragment, derivative and/or splice variant thereof.
11. The at least one GDP-L-FS protein, fragment, derivative, splice variant, nucleotide sequence and/or gene sequence and/or the at least one carrier coupled to at least one GDP-L-FS protein, fragment, derivative, splice variant, nucleotide sequence and/or gene sequence for use according to embodiment 9 or 10, wherein the at least one GDP-L-FS protein, fragment, derivative, splice variant, nucleotide sequence and/or gene sequence and/or the at least one carrier coupled to at least one GDP-L-FS protein, fragment, derivative, splice variant, nucleotide sequence and/or gene sequence is applied by nasal, inhaled, oral, subcutaneous (s.c.), intracoelomic (i.c), intramuscular (i.m.), intradermal (i.d.), transdermal (t.d.) or intravenous (i.v.) administration, preferably by i.v., s.c., i.d., t.d., oral, inhaled or nasal administration.
12. CD27- Th1 CD4+ cells for use in a method of monitoring the response to the method for inducing antigen-specific tolerance according to any of embodiments 9 to 11, wherein the CD27- Th1 CD4+ cells are detected in body fluid, in particular blood, preferably peripheral blood, or CSF, previously obtained from an MS patient.
13. The CD27- Th1 CD4+ cells for use according to embodiment 12, wherein additionally a responsiveness of T cells and/or antibodies previously obtained from the body fluid of the MS patient to the protein GDP-L-FS or a fragment, derivative and/or splice variant thereof as defined in any of embodiments 3 to 6 is detected.
14. The CD27- Th1 CD4+ cells for use according to embodiment 12 or 13, wherein the number of CD27- Th1 CD4+ cells in the body fluid, in particular those also being negative for the markers CCR7 and/or CD45RA, decreases in the course of tolerance induction.
15. The method according to any one of embodiments 1 to 6, the GDP-L-FS protein or a fragment, derivative or splice variant thereof, or the nucleotide sequence encoding the GDP-L-FS protein or fragment, derivative or splice variant thereof for use according to embodiment 7 or 8, the at least one GDP-L-FS protein, fragment, derivative, splice variant, nucleotide sequence and/or gene sequence and/or the at least one carrier coupled to at least one GDP-L-FS protein, fragment, derivative, splice variant, nucleotide sequence and/or gene sequence for use according to any one of embodiments 9 to 11, or the CD27- Th1 CD4+ cells for use according to any one of embodiments 12 to 14, wherein the CD27- Th1 CD4+ cells are further negative for the markers CCR7 and/or CD45RA.
16. The method according to any one of embodiments 1 to 6 or 15, the GDP-L-FS protein or a fragment, derivative or splice variant thereof, or the nucleotide sequence encoding the GDP-L-FS protein or fragment, derivative or splice variant thereof for use according to embodiment 7 or 8 or 15, the at least one GDP-L-FS protein, fragment, derivative, splice variant, nucleotide sequence and/or gene sequence and/or the at least one carrier coupled to at least one GDP-L-FS protein, fragment, derivative, splice variant, nucleotide sequence and/or gene sequence for use according to any one of embodiments 9 to 11 or 15, or the CD27- Th1 CD4+ cells for use according to any one of embodiments 12 to 14, wherein the MS patient has one or more of the following characteristics:
   - inflammation and/or neurodegeneration in the central nervous system, in particular characterized by a Gd-contrast enhancing T1 lesion and/or a FLAIR T2 lesion,
   - higher expression of genes associated with Th1 cells or cytotoxicity and/or genes encoding proinflammatory cytokines, such as IL-2 and/or IFN-γ, compared with healthy controls,
   - HLA allotype HLA-DRB3*02:02 or DRB3*03:01.
17. A method for stratifying an MS patient, the method comprising: detecting in a sample obtained from the patient CD27- Th1 CD4+ cells, to thereby stratify the patient.
18. The method according to embodiment 17, wherein the sample comprises body fluid.
19. The method according to embodiment 17 or 18, wherein the body fluid comprises blood, e.g., peripheral blood, or cerebrospinal fluid (CSF).
20. The method according to any one of embodiments 17 to 19, wherein the method comprises detecting responsiveness of T cells and/or antibodies in the body fluid to the protein GDP-L-fucose synthase (GDP-L-FS) or a fragment, derivative and/or splice variant thereof.
21. A method for treating an MS patient, the method comprising: detecting in a sample obtained from the patient CD27- Th1 CD4+ cells, and administering to the patient an MS therapy, to thereby treat the patient.
22. The method according to embodiment 21, wherein the sample comprises body fluid.
23. The method according to embodiment 21 or 22, wherein the body fluid comprises blood, e.g., peripheral blood, or cerebrospinal fluid (CSF).
24. The method according to any one of embodiments 21 to 23, wherein T cells and/or antibodies previously obtained from the sample of the patient respond to the protein GDP-L-FS or a fragment, derivative and/or splice variant thereof.
25. The method according to any one of embodiments 21 to 24, wherein the MS therapy comprises immunodominant peptides.
26. The method according to any one of embodiments 21 to 25, wherein the MS therapy comprises treating the patient with antigen-specific immunotherapies, such as tolerance induction.
27. The method according to any one of embodiments 21 to 26, wherein treating the patient comprises administering to the patient immunodominant peptides selected from MBP, PLP and MOG, e.g., disclosed in EP 2 205 273 B1.
28. The method according to any one of embodiments 21 to 27, wherein treating the patient comprises administering to the patient immunodominant proteins or peptides selected from GDP-L-FS or a fragment, derivative or splice variant thereof, and a protein from the RASGRP family or a fragment, derivative or splice variant thereof, e. g., disclosed in WO 2020/002674.
29. The method according to any one of embodiments 25 to 28, wherein the immunodominant peptides are coupled, e.g., chemically, to white or red blood cells.
30. Use of the GDP-L-FS protein or a fragment, derivative or splice variant thereof, or a nucleotide sequence encoding the GDP-L-FS protein or fragment, derivative or splice variant thereof as defined in any one of embodiments 3 to 6, for the manufacture of a medicament for the treatment of MS in an MS patient, wherein CD27- Th1 CD4+ cells are detected in body fluid, in particular blood, preferably peripheral blood, or CSF, previously obtained from the MS patient.
31. The use according to embodiment 30, wherein T cells and/or antibodies previously obtained from the body fluid of the MS patient respond to the protein GDP-L-FS or a fragment, derivative and/or splice variant thereof.
   In a particularly preferred embodiment, the GDP-L-FS protein or a fragment, derivative or splice variant thereof, or a nucleotide sequence encoding the GDP-L-FS protein or fragment, derivative or splice variant thereof as defined in any one of embodiments 3 to 6, is used for the manufacture of a medicament for the treatment of MS in an MS patient, wherein CD27- Th1 CD4+ cells have been detected in peripheral blood or CSF previously obtained from the MS patient, and wherein T cells and/or antibodies previously obtained from the peripheral blood or CSF of the MS patient respond to the protein GDP-L-FS or a fragment, derivative and/or splice variant thereof.

## Claims

1. Method for stratification of a multiple sclerosis (MS) patient comprising the following steps:
- obtaining body fluid, in particular blood, preferably peripheral blood, or cerebrospinal fluid (CSF), from an MS patient, and
- detecting CD27- Th1 CD4+ cells in the body fluid.

2. The method according to claim 1, wherein the method further comprises:
- detecting responsiveness of T cells and/or antibodies in the body fluid to the protein GDP-L-fucose synthase (GDP-L-FS) or a fragment, derivative and/or splice variant thereof.

3. The method according to claim 2, wherein the protein GDP-L-FS
a) has an amino acid sequence as set forth in SEQ ID NO: 1 or
b) has an amino acid sequence which is at least 85 %, preferably at least 90 %, more preferably at least 95 % identical to the amino acid sequence as set forth in SEQ ID NO: 1 or
c) has an amino acid sequence which is at least 70 %, preferably at least 80 %, more preferably at least 90 % homologous to the amino acid sequence as set forth in SEQ ID NO: 1 or
d) has an amino acid sequence which is at least 60 %, preferably at least 70 %, more preferably at least 80 %, even more preferably at least 90 % homologous to the amino acid sequence as set forth in SEQ ID NO: 1 and the protein or fragment or splice variant thereof binds to an autologous HLA allele, is recognized by a T cell and/or is recognized by an antibody which binds to or recognizes the amino acid sequence as set forth in SEQ ID NO: 1 or a fragment thereof or
e) is encoded by a TSTA3 gene, in particular by a gene sequence of nucleotides 143612618 to 143618048 of NC_000008.11, or is encoded by a gene which is at least 80 %, preferably at least 90 %, even more preferably at least 95 % identical to the gene sequence of nucleotides 143612618 to 143618048 of NC_000008.11.

4. The method according to claim 2 or 3, wherein the fragment comprises 5 to 50, preferably 5 to 20, more preferably 10 to 15 amino acids, even more preferably 15 amino acids.

5. The method according to any one of claims 2 to 4, wherein the fragment is
a) at least 85 %, preferably at least 90 %, more preferably at least 95 % identical to a respective corresponding amino acid sequence or
b) at least 70 %, preferably at least 80 %, more preferably at least 90 % homologous to a respective corresponding amino acid sequence or
c) at least 60 %, preferably at least 70 %, more preferably at least 80 %, even more preferably at least 90 % homologous to a respective corresponding amino acid sequence and binds to an autologous HLA allele, is recognized by a T cell and/or is recognized by an antibody which binds to or recognizes the respective amino acid sequence.

6. The method according to any of the preceding claims, wherein the fragment comprises a sequence selected from the group comprising SEQ ID NOs: 2 to 6 and SEQ ID NO: 37, preferably consists of a sequence selected from the group comprising SEQ ID NOs: 2 to 6 and SEQ ID NO: 37.

7. A GDP-L-FS protein or a fragment, derivative or splice variant thereof, or a nucleotide sequence encoding the GDP-L-FS protein or fragment, derivative or splice variant thereof as defined in any one of claims 3 to 6, for use in the treatment of MS in an MS patient, wherein CD27- Th1 CD4+ cells are in body fluid, in particular blood, preferably peripheral blood, or CSF, previously obtained from the MS patient.

8. The GDP-L-FS protein or a fragment, derivative or splice variant thereof, or the nucleotide sequence encoding the GDP-L-FS protein or fragment, derivative or splice variant thereof for use according to claim 7, wherein T cells and/or antibodies previously obtained from the body fluid of the MS patient respond to the protein GDP-L-FS or a fragment, derivative and/or splice variant thereof.

9. At least one GDP-L-FS protein, fragment, derivative, splice variant, nucleotide sequence and/or gene sequence as defined in any of claims 3 to 6, and/or at least one carrier coupled to at least one GDP-L-FS protein, fragment, derivative, splice variant, nucleotide sequence and/or gene sequence as defined in any of claims 3 to 6 for use in a method for inducing antigen-specific tolerance to autoantigens in an MS patient, wherein CD27-Th1 CD4+ cells are detected in body fluid, in particular blood, preferably peripheral blood, or CSF, previously obtained from the MS patient.

10. The at least one GDP-L-FS protein, fragment, derivative, splice variant, nucleotide sequence and/or gene sequence and/or the at least one carrier coupled to at least one GDP-L-FS protein, fragment, derivative, splice variant, nucleotide sequence and/or gene sequence for use according to claim 9, wherein T cells and/or antibodies previously obtained from the body fluid of the MS patient respond to the protein GDP-L-FS or a fragment, derivative and/or splice variant thereof.

11. The at least one GDP-L-FS protein, fragment, derivative, splice variant, nucleotide sequence and/or gene sequence and/or the at least one carrier coupled to at least one GDP-L-FS protein, fragment, derivative, splice variant, nucleotide sequence and/or gene sequence for use according to claim 9 or 10, wherein the at least one GDP-L-FS protein, fragment, derivative, splice variant, nucleotide sequence and/or gene sequence and/or the at least one carrier coupled to at least one GDP-L-FS protein, fragment, derivative, splice variant, nucleotide sequence and/or gene sequence is applied by nasal, inhaled, oral, subcutaneous (s.c.), intracoelomic (i.c), intramuscular (i.m.), intradermal (i.d.), transdermal (t.d.) or intravenous (i.v.) administration, preferably by i.v., s.c., i.d., t.d., oral, inhaled or nasal administration.

12. CD27- Th1 CD4+ cells for use in a method of monitoring the response to the method for inducing antigen-specific tolerance according to any of claims 9 to 11, wherein the CD27- Th1 CD4+ cells are detected in body fluid, in particular blood, preferably peripheral blood, or CSF, previously obtained from an MS patient.

13. The CD27- Th1 CD4+ cells for use according to claim 12, wherein additionally a responsiveness of T cells and/or antibodies previously obtained from the body fluid of the MS patient to the protein GDP-L-FS or a fragment, derivative and/or splice variant thereof as defined in any of claims 3 to 6 is detected.

14. The method according to any one of claims 1 to 6, the GDP-L-FS protein or a fragment, derivative or splice variant thereof, or the nucleotide sequence encoding the GDP-L-FS protein or fragment, derivative or splice variant thereof for use according to claim 7 or 8, the at least one GDP-L-FS protein, fragment, derivative, splice variant, nucleotide sequence and/or gene sequence and/or the at least one carrier coupled to at least one GDP-L-FS protein, fragment, derivative, splice variant, nucleotide sequence and/or gene sequence for use according to any one of claims 9 to 11, or the CD27- Th1 CD4+ cells for use according to claim 12 or 13, wherein the CD27- Th1 CD4+ cells are further negative for the markers CCR7 and/or CD45RA.

15. The method according to any one of claims 1 to 6 or 14, the GDP-L-FS protein or a fragment, derivative or splice variant thereof, or the nucleotide sequence encoding the GDP-L-FS protein or fragment, derivative or splice variant thereof for use according to claim 7 or 8 or 14, the at least one GDP-L-FS protein, fragment, derivative, splice variant, nucleotide sequence and/or gene sequence and/or the at least one carrier coupled to at least one GDP-L-FS protein, fragment, derivative, splice variant, nucleotide sequence and/or gene sequence for use according to any one of claims 9 to 11 or 14, or the CD27- Th1 CD4+ cells for use according to any one of claims 12 to 14, wherein the MS patient has one or more of the following characteristics:
- inflammation and/or neurodegeneration in the central nervous system, in particular **characterized by** a Gd-contrast enhancing T1 lesion and/or a FLAIR T2 lesion,
- higher expression of genes associated with Th1 cells or cytotoxicity and/or genes encoding proinflammatory cytokines, such as IL-2 and/or IFN-γ, compared with healthy controls,
- HLA allotype HLA-DRB3*02:02 or DRB3*03:01.
